(19) Europäisches Patentamt
European Patent Office
Office européen des brevets

(11) EP 0 330 050 B1

(12) # EUROPÄISCHE PATENTSCHRIFT

(45) Veröffentlichungstag und Bekanntmachung des Hinweises auf die Patenterteilung:
**23.08.2000 Patentblatt 2000/34**

(51) Int. Cl.[7]: **C09B 15/00**, C07D 219/00, G01N 33/58, G01N 33/543

(21) Anmeldenummer: **89102487.9**

(22) Anmeldetag: **14.02.1989**

(54) **Spezielle chemilumineszierende Acridinderivate und ihre Verwendung in Lumineszenzimmunoassays**

Special chemiluminescent acridine derivatives and their use in luminescence immunoassays

Dérivés spéciaux d'acridine chimiluminescents et leur utilisation dans des tests d'immunoluminescence

(84) Benannte Vertragsstaaten:
**AT BE CH DE ES FR GB GR IT LI LU NL SE**

(30) Priorität: **20.02.1988 DE 3805318**

(43) Veröffentlichungstag der Anmeldung:
**30.08.1989 Patentblatt 1989/35**

(73) Patentinhaber:
**Dade Behring Marburg GmbH**
**35001 Marburg (DE)**

(72) Erfinder:
- **Kinkel, Tonio, Dr.**
  **D-6230 Frankfurt am Main 80 (DE)**
- **Molz, Peter, Dr.**
  **D-6500 Mainz (DE)**
- **Schmidt, Erwin, Dr.**
  **D-6233 Kelkheim (Taunus) (DE)**
- **Schnorr, Gerd, Dr.**
  **D-6368 Bad Vilbel (DE)**
- **Skrzipczyk, Heinz Jürgen, Dr.**
  **D-6232 Bad Soden am Taunus (DE)**

(56) Entgegenhaltungen:
**EP-A- 0 082 636**
**EP-A- 0 216 553**
**EP-A- 0 257 541**
**EP-A- 0 273 115**

- **CLINICAL CHEMISTRY, Band 29, Nr. 8, August 1983, Seiten 1474-1479, Winston, US; I. WEEKS et al.: "Acridinium esters as high-specific-activity labels in immunoassay"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Der Gegenstand der vorliegenden Erfindung sind chemilumineszierende Acridinderivate, Verfahren zu ihrer Herstellung und ihre Verwendung in Lumineszenzimmunoassays.

Lumineszierende Verbindungen finden bereits vielseitige Anwendung. Sie werden als Indikatoren in Bioassays, Enzymimmunoassays und Lumineszenzimmunoassays eingesetzt (vgl. W.P. Collins "Alternative Immunoassays", Verlag John Wiley & Sons Ltd., Chichester, 1985), werden aber auch in Nukleinsäure Hybridisierungsassays verwendet (vgl. J.A. Matthews et al. "Analytical Biochemistry", 151, 205-209, 1985). Außerdem werden chemilumineszierende Verbindungen bei der "flow injection analysis", in post-column-Detektoren in der Flüssigkeitschromatographie, in der Strömungsforschung und in künstlichen Lichtquellen eingesetzt.

Bei den Chemilumineszenzimmunoassays haben insbesondere zwei Strukturtypen von chemilumineszierenden Markierungssubstanzen größere Bedeutung erlangt. Es handelt sich hierbei einerseits um die Luminol- bzw. Isoluminolderivate, die bei H.R. Schroeder et al., "Methods in Enzymology", Academic Press Inc., New York, Vol. LVII, 1978, 424 ff sowie in den britischen Patenten 2 008 247 und 2 041 920, den deutschen Patentschriften 26 18 419 und 26 18 511, sowie in der europäischen Patentanmeldung 135 071 beschrieben sind. Einen Überblick über die praktisch Anwendung der Isoluminolverbindungen als Lumineszenzindikatoren findet man bei W.G. Wood, J. Clin.Chem. Clin. Biochem. 22, 1984 905-918.

Andererseits haben auch Acridiniumesterverbindungen als chemilumineszierende Markierungssubstanzen Anwendung gefunden. Derartig Acridiniumester sind aus dem US-Patent 3 352 791, den britischen patenten 1 316 363 und 1 461 877 sowie aus der europäischen Patentanmeldung 82 636 bekannt. Die Anwendung der Acridiniumester als Markierungssubstanzen in Immunoassays ist bei Weeks et al., Clin. Chem. 29/8 (1983), 1474-1479, beschrieben. Auch die Verwendung von Phenanthridiniumestern als Markierungssubstanz in Lumineszenzimmunoassays ist aus der europäischen Patentanmeldung 170 415 bereits bekannt.

Die Chemilumineszenz von Acridiniumestern kann durch Zugabe von alkalischer $H_2O_2$-Lösung gestartet werden. Für den Mechanismus der Chemilumineszenz hat F. McCapra, Acc.Chem. Res. 9, 201, 1976, eine überzeugende Erklärung gegeben. Dabei ist offensichtlich die Art der Abgangsgruppe sowohl für die Lichtquantenausbeute als auch für die hydrolytische Stabilität entscheidend.

Die bisher schon bekannten Acridiniumester haben gegenüber den Luminol- und Isoluminolverbindungen den Vorteil einer höheren Lichtquantenausbeute, die auch durch an den Indikator gebundene Proteine nicht beeinträchtigt wird (vgl. Weeks et al., Clin. Chem. 29/8 (1983), 1474-1479).

Obwohl die aus der europäischen Patentanmeldung 82 636 bekannten Acridiniumphenylester bei Anregung der Chemilumineszenz durch milde Oxidationsmittel sich durch eine hohe Nachweisempfindlichkeit auszeichnen, weisen sie für die praktische Anwendung störende Nachteile auf. Vor allem ist die Phenylesterbindung in wäßrigen Systemen auch schon bei Raumtemperatur sehr labil. Hinzu kommt, daß unter den dort angegebenen Oxidationsbedingungen die Acridiniumphenylester eine Lichtemission zeigen, die erst nach etwa 10 Sekunden weitgehend, d.h. zu über 95 %, abgeklungen ist. Im Vergleich dazu haben andere nicht isotopische Assayverfahren weitaus kürzere Meßzeiten und ermöglichen dadurch einen höheren Probendurchsatz.

Es wurde bereits die Verwendung von chemilumizierenden Acridiniumderivaten vorgeschlagen, die bei hoher Lichtquantenausbeute eine schnellere Reaktionskinetik aufweisen und damit kurze Meßzeiten für einen Lumineszenzimmunoassay ermöglichen (vgl. deutsche Patentanmeldung P 36 28 573.0) Es handelt sich dabei um Acridiniumderivate der Formel

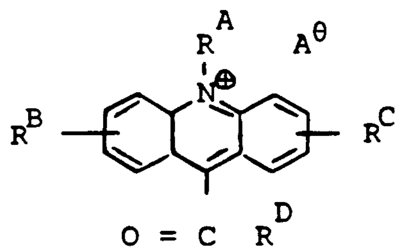

in der $R^A$ Wasserstoff, ein Alkyl-, Alkenyl- oder Alkinylrest mit 1 bis 10 Kohlenstoffatomen, eine Benzyl- oder Arylgruppe ist, $R^B$ und $R^C$ Wasserstoff, eine Alkylgruppe mit 1 bis 4 Kohlenstoffatomen, eine substituierte oder unsubstituierte Aminogruppe, eine Carboxy-, Alkoxy-, Cyano-, Nitrogruppe oder Halogen sind, $R^D$ einen Rest darstellt, in dem eine Sulfonamidgruppe über den Stickstoff direkt an die Carbonylgruppe gebunden ist oder ein Thioalkyl- oder Thioarylrest der Formel

$$- S - Y - R^E$$

ist, wobei Y eine verzweigte oder unverzweigte aliphatische oder eine aromatische Gruppe ist, die auch Heteroatome enthalten kann, und $R^E$ eine reaktive Gruppe ist, die unter schonenden Bedingungen selektiv mit Amino-, Carboxy- Thiol- oder anderen funktionellen Gruppen in Substanzen von biologischem Interesse eine Bindung eingehen kann, und $A^\theta$ ein die Chemilumineszenz nicht beeinträchtigendes Anion ist.

Es hat sich nun herausgestellt, daß spezielle Acridiniumderivate sich auf Grund ihrer hervorragenden Stabilität und ihrer unerwartet hohen Nachweisempfindlichkeit besonders für den Einsatz als chemilumineszierende Verbindungen eignen. Erfindungsgegenstand sind demzufolge chemilumineszierende Acridiniumderivate der Formel I

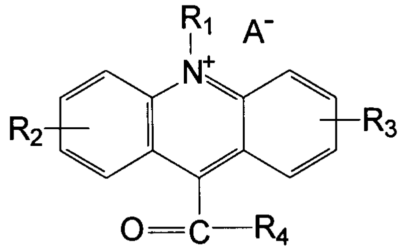

(I)

in der $R_1$ Wasserstoff
ein Alkyl-, Alkenyl-- oder Alkinylrest mit bis zu 10 Kohlenstoffatomen eine Benzyl- oder Arylgruppe ist;
$R_2$ und $R_3$ Wasserstoff,eine Alkylgruppe mit 1 bis 4 Kohlenstoffatomen, eine substituierte oder unsubstituierte Aminogruppe, eine Carboxy-, Alkoxy-, Cyano- oder Nitrogruppe oder Halogen sind;
$R_4$ einen Rest der Formel II oder III darstellt

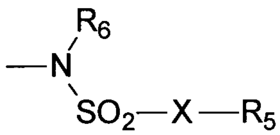

(II)

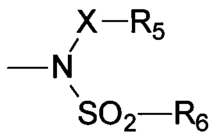

(III)

worin $R_5$ eine reaktive Gruppe ist, die unter schonenden Bedingungen selektiv mit Amino-, Carboxy-, Thiol- oder anderen funktionellen Gruppen in Substanzen von biologischem Interesse eine Bindung eingehen kann;
$R_6$ eine Alkylgruppe mit 1 bis 4 Kohlenstoffatomen oder eine Phenylgruppe ist, die mit bis zu 3 Alkyl- oder Alkoxygruppen mit jeweils 1 bis 4 Kohlenstoffatomen, mit einer $-(O-CH_2-CH_2-)_n-OR$ Gruppe substituiert sein kann, wobei

n die Bedeutung 0 bis 8 hat und
R eine Morpholinoethyl- oder eine N,N-Dimethylaminoethyl- oder eine Alkygruppe mit 1 bis 4 Kohlenstoffatomen ist, oder
mit einer Ethylendioxygruppe substituiert sein kann; und
X eine Arylengruppe ist, die
direkt oder über eine Alkylen- oder eine Oxyalkylengruppe an das Stickstoff-oder Schwefelatom gebunden ist,
und
über eine Alkylen- oder Oxyalkylengruppe an den Rest $R^5$ gebunden ist, und

die auch mit
Alkyl-, Alkenyl-, Hydroxy-, Amino-, Alkoxy- oder Aryloxygruppen
und/oder
Heteroatomen einfach oder mehrfach substitiuert sein kann oder
den Rest einer aromatischen natürlichen Aminosäure bedeutet oder
eine Phenylengruppe ist - wenn $R_6$ eine Phenylgruppe ist - die einfach oder mehrfach mit Alkygruppen mit 1 bis 6 Kohlenstoffatomen substituiert ist und

$A^-$ ein die Chemolumineszenz nicht beeinträchtigendes Anion ist.

Unter den Substanzen von biologischem Interesse sind vor allem Antigene zu verstehen. Unter diesem Begriff fallen z. B. Hormone, Steroide, Arzneimittel, Arzneimittelmetabolite, Toxine, Alkaloide und auch Antikörper.

Als Aminosäuren eignen sich Phenylalanin, Histidin und Tryptophan:

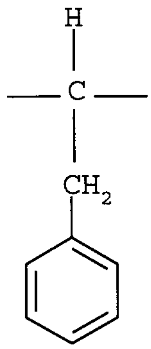 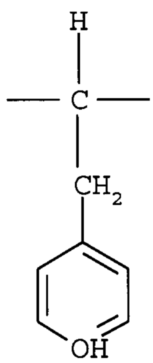 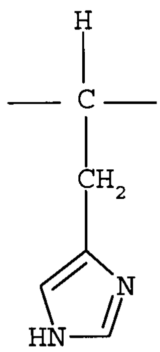 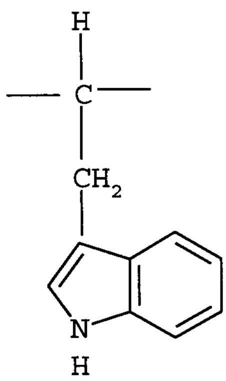

Phenylalanin(Phe) Tyrosin(Tyr) Histidin(His) Tryptophan(Try)

Das die Chemilumineszenz nicht beeinträchtigende Anion kann z.B. ein Tetrafluoroborat-, Perchlorat-, Halogenid-, Alkylsulfat-, Halosulfonat-, Alkylsulfonat oder Arylsulfonat-Anion sein. Auch jedes andere Anion kann eingesetzt werden, sofern es die Chemilumineszenz nicht löscht oder abschwächt.

Die Heteroalkylgruppen oder heterocyclischen Gruppen enthalten vorzugsweise Heteroatome, die zu einer Steigerung der Wasserlöslichkeit der erfindungsgemäßen Verbindungen beitragen können, wie z.B. Stickstoff, Sauerstoff, Schwefel, Phosphor, bzw. deren Kombinationen. Besonders geeignete Heterocyclen sind z.B. Morpholin, Piperazin, Piperidin, Tetrahydrofuran, Dioxane usw.

Besondere Bedeutung haben Acridiniumderivate nach Anspruch 1, die dadurch gekennzeichnet sind, daß X eine Gruppe der Formel IV ist,

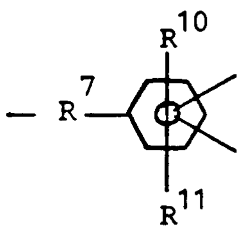

(IV)

in der $R^7$ ein Substituent der Formel $-(CH_2)_n-$ oder $((CH_2)_m-O)_n-$ ist mit n= 0 bis 4 und m= 1 bis 6, $R^8$ ein Substituent in ortho-, meta- oder para-stellung zu $R^7$ der Formel $-(CH_2)_p-$, eine Polyalkylenoxid-Gruppe der Formel $-(O-(CH_2)_m-)_p$ oder $-((CH_2)_m-O-)_p$ bevorzugt mit p= 1-6 und m= 1-6, oder ein verzweigter oder unverzweigter Kohlenwasserstoffrest mit 1 - 4 C-Atomen, ist und die Substituenten $R^9$ - $R^{11}$ Wasserstoff oder geradkettige oder verzweigte Kohlenwasserstoffreste mit bis zu 30 C-Atomen sind, wobei eine oder mehrere $-CH_2$-Einheiten

auch durch O, S, SO, $SO_2$, NH oder N-Alkyl ersetzt sein können und zwei dieser Substituenten ringförmig verknüpft sein können.

Von besonderer Bedeutung für die Einsatzmöglichkeiten der erfindungsgemäßen Acridiniumderivate ist der Substituent $R^5$. Durch die geeignete Auswahl dieser Gruppe erhält das Acridiniumderivat eine so hohe Reaktivität, daß es schon unter milden Bedingungen selektiv mit einer funktionellen Gruppe der nachzuweisenden biologischen Substanz eine Bindung eingehen kann. Geeignete reaktive Gruppen sind aus der folgenden Zusammenstellung zu ersehen:

a) 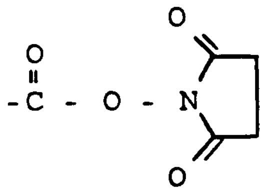

b) 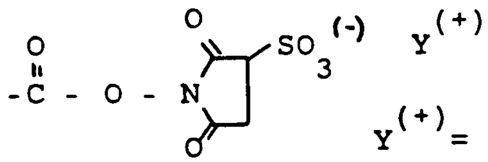

$Y^{(+)} = H^{(+)}$, $Alkali^{(+)}$

c) 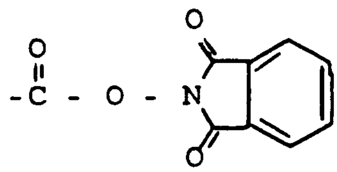

d) $-SO_2-CH=CH_2$

e) 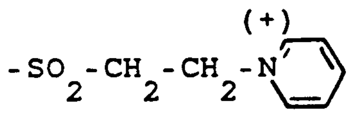 Halogenid$^{(-)}$

f) $-N=C=S$

g) 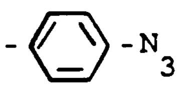

h) 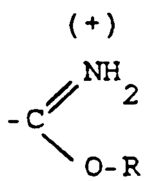 Halogenid$^{(-)}$

i) 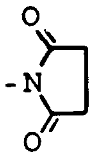

k) 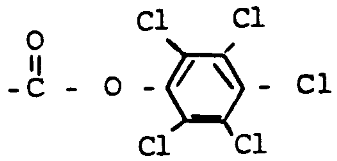

l) 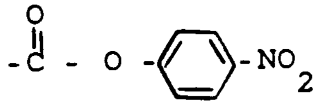

m) 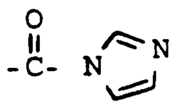

n) 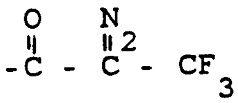

o) 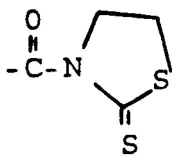

In vielen Fällen haben sich erfindungsgemäße Acridiniumderivate als geeignet erwiesen, bei denen $R^5$ eine Gruppe der Formel V

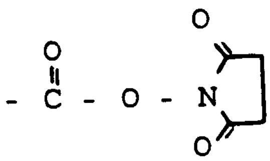 (V)

ist.

Weiterhin haben sich Acridiniumverbindungen der Formel VI als besonders geeignet erwiesen. Die Formel VI lautet

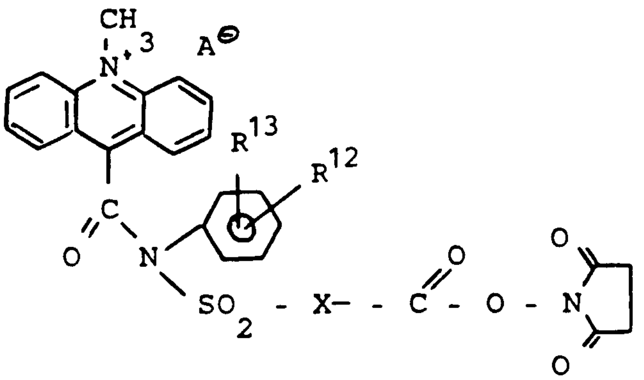

(VI)

in der X eine Gruppe der Formel VII

$$-\bigcirc-(CH_2)_n- \quad (VII)$$

mit n= 2 oder 4
und $R^{12}$ und $R^{13}$ unabhängig voneinander Wasserstoff, eine Alkylgruppe, eine Alkoxygruppe mit 1 - 4 C Atomen, eine $(-O-CH_2-CH_2)_n-OR$-Gruppe, wobei n die Bedeutung 0 - 8 hat und R eine Morpholinoethyl- oder eine Alkylgruppe mit 1 - 4 C-Atomen oder eine N,N-Dimethylaminoethyl-Gruppe ist,
oder zusammen eine Ethylendioxy-Gruppe sind
und $A^{\theta}$ die in Anspruch 1 genannten Bedeutungen hat. Diese Verbindungen stellen leicht wasserlösliche Produkte dar.

Unter den zuletzt genannten Verbindungen der Formel VII sind wiederum diejenigen ganz besonders bevorzugt, in denen X eine p-Ethylenphenyl-Gruppe, $R^{12}$= H und $R^{13}$ eine p-Methoxy-Gruppe oder $R^{12}$ eine ortho-methoxy- und $R^{13}$ eine para-Methoxy-Gruppe oder $R^{12}$ und $R^{13}$ zusammen eine 3,4-Ethylendioxy-Gruppe sind, wie z.B. die Verbindungen der Formel

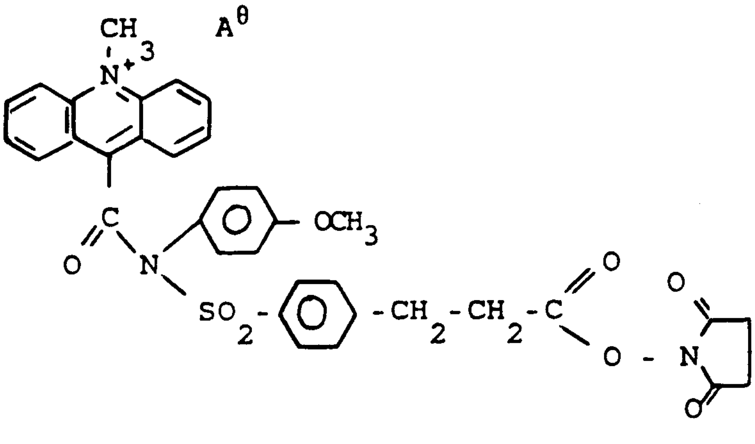

Weitere besonders geeignete Acridiniumderivate haben die Formel VIII

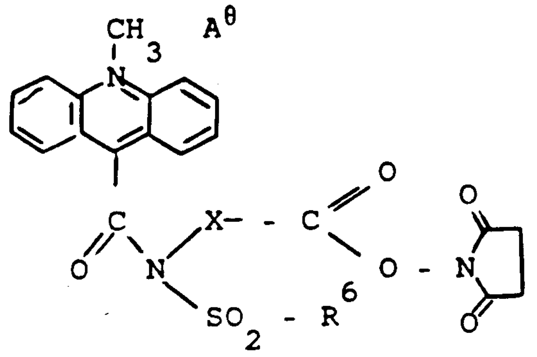

(VIII)

in der $R^6$ eine Alkylgruppe mit 1-4 C-Atomen oder eine Phenylgruppe ist, die mit bis zu drei Alkyl- oder Alkoxygruppen mit jeweils 1-4 C-Atomen, mit einer $-(-O-CH_2-CH_2)_n-OR$-Gruppe, wobei n die Bedeutung 0-8 hat und R eine Morpholinoethyl- oder eine N,N-Dimethylaminoethyl-Gruppe oder Alkylgruppe mit 1 - 4 C-Atomen ist oder mit einer Ethylendioxy Gruppe substituiert sein kann und X die in Anspruch 1 oder 2 genannten Bedeutungen hat oder in der $R^6$ eine Phenylgruppe ist, die mit bis zu drei Alkylgruppen mit jeweils 1-4 C-Atomen substituiert sein kann und X- eine ortho-, meta- oder para-phenylengruppe ist. Auch diese Verbindungen stellen leicht wasserlösliche Produkte dar.

Es ist überraschend, daß am Amidstickstoff Sulfonyl-substituierte Acridinium-9-carbonsäureamide eine ausgezeichnete Chemilumineszenz aufweisen, denn es ist bekannt, daß Acridinium-9-carbonsäureamide im Gegensatz zu den Acridinium-9-carbonsäureestern keinerlei Chemilumineszenz zeigen (vgl. F. McCapra in W. Carruthers und J.K. Sutherland: Progress in Organic Chem., Vol. 8, 231-277, 1973, Butterworth, London).

Ein bedeutender Vorteil der erfindungsgemäßen Acridiniumverbindungen gegenüber den aus der Europäischen Patentanmeldung 82 636 bekannten Acridiniumphenylestern liegt in der wesentliche schnelleren Reaktionskinetik der Lichtemission (vgl. P 36 28 573.0).

Ein weiterer Vorteil ist durch die Stabilität der mittels der erfindungsgemäßen Verbindungen hergestellten Tracer gegeben. Figur 1 zeigt das Ergebnis eines Stabilisitätstests, bei dem nach Lagerung bei erhöhter Temperatur (50°C) die Intensität des jeweiligen Chemilumineszenzsignals gemessen wurde. Kurve 1 bezieht sich auf aus der Verbindung a) N-(4-methoxyphenyl)-N-[4(2-succinimidyloxycarbonylethyl)benzolsulfonyl]-10-methylacridinium-9-carbonsäureamidfluorosulfonat (6),

die Kurve b) auf aus der Verbindung N-(4-Methoxyphenyl)-N-[4-(4-succinimidyloxycarbonylbutyl)benzolsulfonyl]-10-methyl-acridinium-9-carbonsäureamid-fluorosulfonat (11) und die Kurve c) auf aus der Verbindung 4-(2-Succinimidyloxycarbonylethyl)-phenyl-10-methylacridinium-9-carboxylatmethosulfat (europäische Patentanmeldung 82 636, S. 10) dargestellte Tracer.

Es ist deutlich zu erkennen, daß die erfindungsgemäßen Tracer aus den Verbindungen a) und b) stabiler sind als die entsprechende Verbindung aus c).

Ein ähnliches Ergebnis erhält man bei einem entsprechenden Test bei 4°C. Figur 2 zeigt die Signalintensität nach Lagerung bei 4°C. Wiederum erweist sich der aus der Verbindung a) hergestellte Tracer als entscheidend stabiler als der aus der Verbindung c) hergestellte.

Zur Herstellung der erfindungsgemäßen Acridinium-Sulfonamidderivate kann vom Acridin-9-carbonsäurechlorid (IX) ausgegangen werden. Um dieses herzustellen wird z.B Acridin nach dem von Lehmstedt und Hundertmark in Ber. 63, 1229 (1930) angegebenen Verfahren in Ethanol/Eisessig mit Kaliumcyanid zum 9-Cyanacridin umgesetzt. Hieraus wird vorzugsweise nach Umkristallisation durch Umsetzung mit Schwefelsäure und Natriumnitrit entsprechend dem von Lehmstedt und Wirth in Ber. 61, 2044 (1928) beschriebenen Verfahren die Acridin-9-carbonsäure gewonnen. Durch Umsetzung der Acridin-9-carbonsäure z.B. mit Thionylchlorid wird die Verbindung der Formel IX

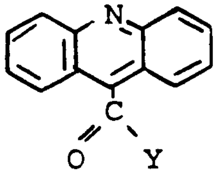

(IX)

gewonnen, in der Y die Bedeutung von Chlor hat. Anstelle eines Halogens kann in die Verbindung IX für Y auch eine

Oxycarbonylalkyl-, Oxycarbonylaryl- oder Imidazolidgruppe eingeführt werden.

Das Säurechlorid (IX) läßt sich dann mit einer geschützten Sulfonamidcarbonsäure der Formel X

$$R^{6} - SO_{2} - \overset{\overset{H}{|}}{N} - X - \overset{\overset{O}{\|}}{C} - OZ \qquad (X)$$

oder der Formel XI

$$R^{6} - \overset{\overset{H}{|}}{N} - SO_{2} - X - COOZ \qquad (XI)$$

umsetzen, in denen X und $R^6$ die vorstehend genannten Bedeutungen haben und Z ein die Carboxygruppe schützender Rest ist, der anschließend abgespalten wird. Beispielsweise kann für diese Reaktion das N-(4-Benzoxycarbonylphenyl)-N-4-toluolsulfonsäureamid eingesetzt werden. Vorteilhaft ist die Verwendung der t-Butylester, deren Schutzgruppe unter besonders schonenden Bedingungen eingeführt und wieder abgespalten werden kann. Die nach Abspaltung der Schutzgruppe entstehende Säure wird dann mit einer geeigneten Verbindung beispielsweise mit N-Hydroxysuccinimid, in den Rest $R^5$ überführt. Hieraus wird die chemilumineszierende Acridinverbindung durch Alkylierung am Stickstoff in 10-stellung gewonnen.

Die erhaltenen Acridiniumverbindungen lassen sich dann mit einer Substanz von biologischem Interesse, z.B. einem Antigen, einem Antikörper, einem Hormon, einem Arzneimittel, einem Arzneimittelmetaboliten, einem Toxin oder einem Alkaloid zu einer lumineszierenden Verbindung umsetzen. Dabei wird das Acridiniumderivat entweder direkt oder über ein Brückenmolekül wie z.B. Aminosäuren, Oligo- oder Polyaminosäuren, Peptide oder synthetische Polymere an die biologisch interessante Substanz unter Ausbildung eines stabilen, immunologisch aktiven Konjugates gebunden. Dieses Konjugat wird auch als Tracer bezeichnet und wird in den nachfolgend beschriebenen Lumineszenzimmunoassays eingesetzt. Für den erfindungsgemäßen Lumineszenzimmunoassay zur Bestimmung einer antigenen Substanz in einer Flüssigkeitsprobe nach einem kompetitiven oder einem Sandwich-Verfahren wird mindestens eine immunologisch aktive Komponente benötigt, die auf einer festen Phase immobilisiert ist und außerdem der lumineszierende Tracer.

Nach Beendigung der Immunreaktion und eventuell benötigten Waschschritten wird die Lichtemission durch sukzessive oder gleichzeitige Zugabe von einem oder mehreren Reagenzien ausgelöst, wobei mindestens ein Reagenz ein Oxidationsmittel in gebundener oder ungebundener Form enthält. Der Lumineszenzimmunoassay kann nun in verschiedener Weise durchgeführt werden.

Eine Möglichkeit besteht darin, daß man den mit dem Antigen spezifisch reagierenden immobilisierten Antikörper mit einer Probe der zu untersuchenden Flüssigkeit und einem Konjugat aus dem Antigen und einem chemilumineszierenden Acridiniumderivat (Antigen-Tracer) inkubiert, die Probe und den nicht gebundenen Tracer abtrennt, den gebundenen Tracer mit den notwendigen Reagenzien zusammenbringt, um eine Lichtemission hervorzurufen, und dann aus der gemessenen Stärke der Lichtemission die Menge des vorhandenen Antigens bestimmt.

Eine andere Möglichkeit für die Durchführung des Lumineszenzimmunoassays besteht darin, daß man einen mit dem Antigen spezifisch reagierenden immobilisierten Antikörper mit einer Probe der zu untersuchenden Flüssigkeit mit einem Konjugat aus einem zweiten, spezifisch reagierenden Antikörper und einem chemilumineszierenden Acridinium-Derivat inkubiert, die Probe und das nicht gebundene, markierte Konjugat abtrennt, das gebundene, markierte Konjugat mit den notwendigen Reagenzien zusammenbringt, um eine Lichtemission hervorzurufen, und aus der gemessenen Stärke der Lichtemission die Menge des vorhandenen Antigens bestimmt.

Die vorstehend genannten Lumineszenzimmunoassays können auch in der Weise durchgeführt werden, daß man vor der Zugabe des markierten Konjugats die zu untersuchende Flüssigkeit vom immobilisierten Antikörper abtrennt.

In anderen erfindungsgemäß durchführbaren Lumineszenzimmunoassays ist nicht der Antikörper, sondern das Antigen immobilisiert. So kann ein mit dem Antikörper spezifisch reagierendes immobilisiertes Antigen mit einer Probe der zu untersuchenden Flüssigkeit und einer Lösung eines Konjugats aus dem Antikörper und einem chemilumineszierenden Acridiniumderivats inkubiert werden, dann die Probe und das nicht gebundene markierte Konjugat abgetrennt und dann das gebundene, markierte Konjugat mit den notwendigen Reagenzien zusammengebracht werden. Dann tritt eine Lichtemission auf, aus deren Stärke die Menge des vorhandenen Antigens bestimmt werden kann.

Eine weitere Variante besteht darin, daß man ein mit dem Antikörper spezifisch reagierendes, immobilisiertes Antigen mit einer Lösung eines Konjugats aus dem Antikörper und einem chemilumineszierenden Acridiniumderivat inkubiert, das nicht umgesetzte, markierte Konjugat abtrennt, eine Probe der zu untersuchenden Flüssigkeit zufügt, die Probe anschließend wieder abtrennt, das gebundene, markierte Konjugat mit den notwendigen Reagenzien zusammenbringt, um eine Lichtemission hervorzurufen und aus dieser dann die Menge des vorhandenen Antigens bestimmt.

Schließlich ist der Lumineszenzimmunoassay auch in der Weise durchführbar, daß man ein mit dem Antikörper spezifisch reagierendes, immobilisiertes Antigen mit einer Lösung eines Konjugats aus dem Antikörper und einem chemilumineszierenden Acridiniumderivat inkubiert, eine Probe der zu untersuchenden Flüssigkeit zufügt, die Probe und das nicht gebundene Konjugat abtrennt, das gebundene, markierte Konjugat mit den notwendigen Reagenzien zusammenbringt und dann aus der gemessenen Lichtemission die Menge des vorhandenen Antigens bestimmt.

Die Herstellung der erfindungsgemäßen Acridiniumverbindungen wird durch die Beispiele 1 bis 7 näher erläutert.

**Beispiel 1:**

N-(4-Methoxyphenyl)-N-[4-(2-benzyloxycarbonylethyl) benzolsulfonyl]acridin-9-carbonsäureamid (3)

17 g 4′-[N-(4-methoxyphenyl)sulfamido]-3-phenylpropion säurebenzylester (1) in 400 ml Dichlormethan werden mit

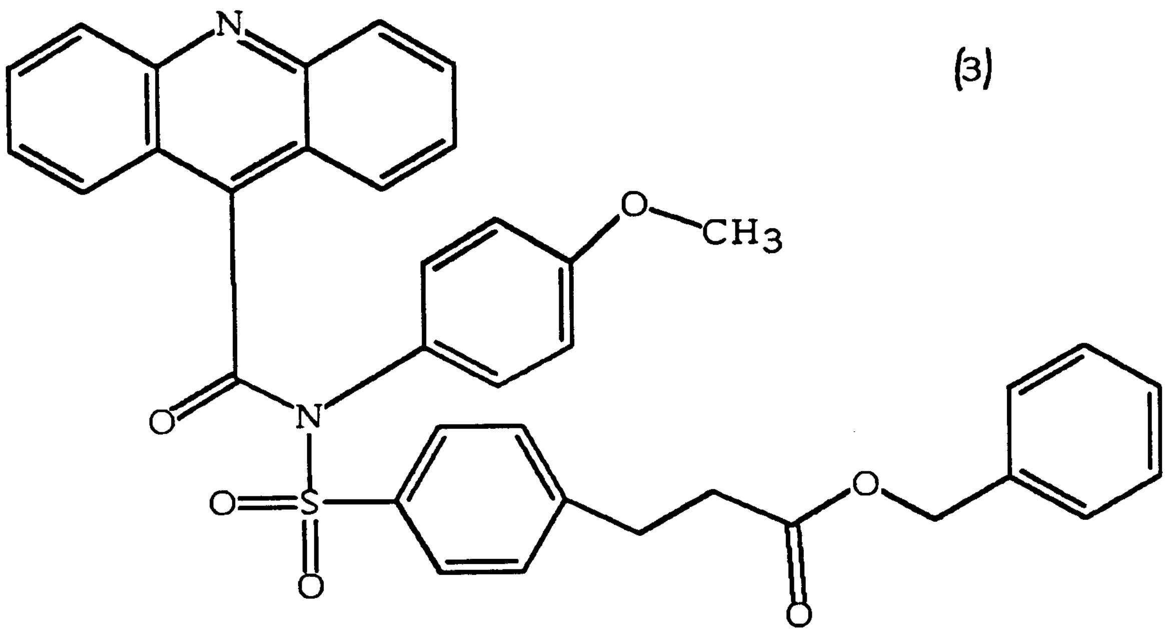

(3)

460 mg 4-(N,N-Dimethylamino)pyridin und 22,1 ml Triethylamin versetzt, nach 10 min gibt man 11.12 g Acridin-9-carbonsäurechlorid-hydrochlorid (2) zu und erhitzt 6 Stunden zum Rückfluß. Die abgekühlte Lösung wird kurz mit 2 N NaOH verrührt, die abgetrennte organische Phase mit $H_2O$ gewaschen, über Magnesiumsulfat getrocknet und eingeengt. Der Rückstand wird durch Säulenchromatographie gereinigt.

Ausbeute: 60 % Schmelzpunkt: 130 - 132°C
NMR (DMSO, 100 MHz): δ= 2,7-3,0 ppm (d,br,2H), δ= 3,0-3,3 ppm (d br,2H), δ= 3,5 ppm (s,br,3H), δ= 5,1 ppm (s,2H), δ= 6,5 ppm (d,br,2H), δ= 7,1 ppm (d,br, 2H), δ= 7,35 ppm (s,5H), δ= 7,5-8,3 ppm (m,12H)

N-(4-Methoxyphenyl)-N-[4-(2-carboxyethyl)benzolsulfonyl]acridin-9-carbonsäureamid-hydrobromid (4)

6,3 g (3) werden in 30 ml 33 % HBr/Eisessig 2 Stunden auf 60°C erhitzt, nach Abkühlen fügt man 60 ml Diisopropylether hinzu, saugt den Niederschlag ab und trocknet im Vakuum

Ausbeute: 90 % Schmelzpunkt: Zersetzung 237°C

NMR (DMSO, 100 MHz): δ= 2,7 ppm (d,br.2H), δ= 3,1 ppm (d,br,2H), δ= 3,5 ppm (s,br,3H), δ= 6,5 ppm (d,br,2H), δ= 7,0-8,4 ppm (m,15H)

N-(4-Methoxyphenyl)-N-[4-(2-succinimidyloxycarbonylethyl)benzolsulfonyl]acridin-9-carbonsäureamid (5)

3,1 g (4) in 50 ml Tetrahydrofuran werden mit 1,41 ml Triethylamin versetzt, auf -20°C abgekühlt und mit 0,474 ml Chlorameisensäureethylester versetzt. Man rührt 20 min nach, gibt 575 mg N-Hydroxysuccinimid zu, rührt 3 Stunden bei -20°C und läßt über Nacht unter Rühren auf Raumtemperatur kommen. Vom Niederschlag wird abgesaugt, das Filtrat eingeengt, der Rückstand in Dichlormethan oder Ethylacetat aufgenommen, die resultierende Lösung mit Wasser, $NaHCO_3$-Lösung und Wasser gewaschen und über $MgSO_4$ getrocknet. Die

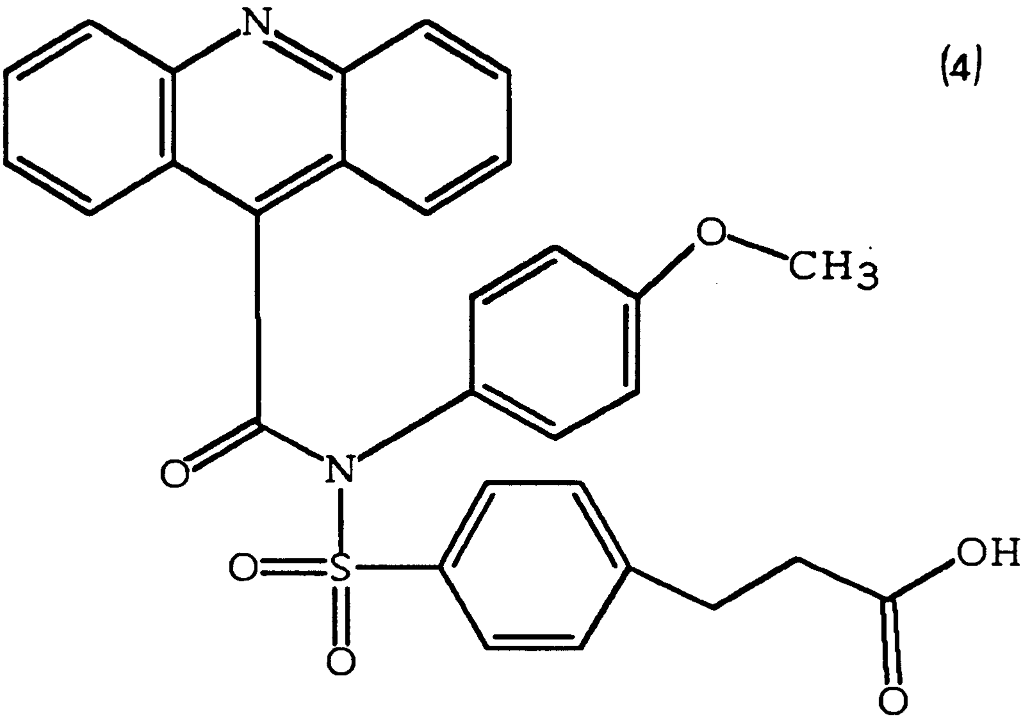

(4)

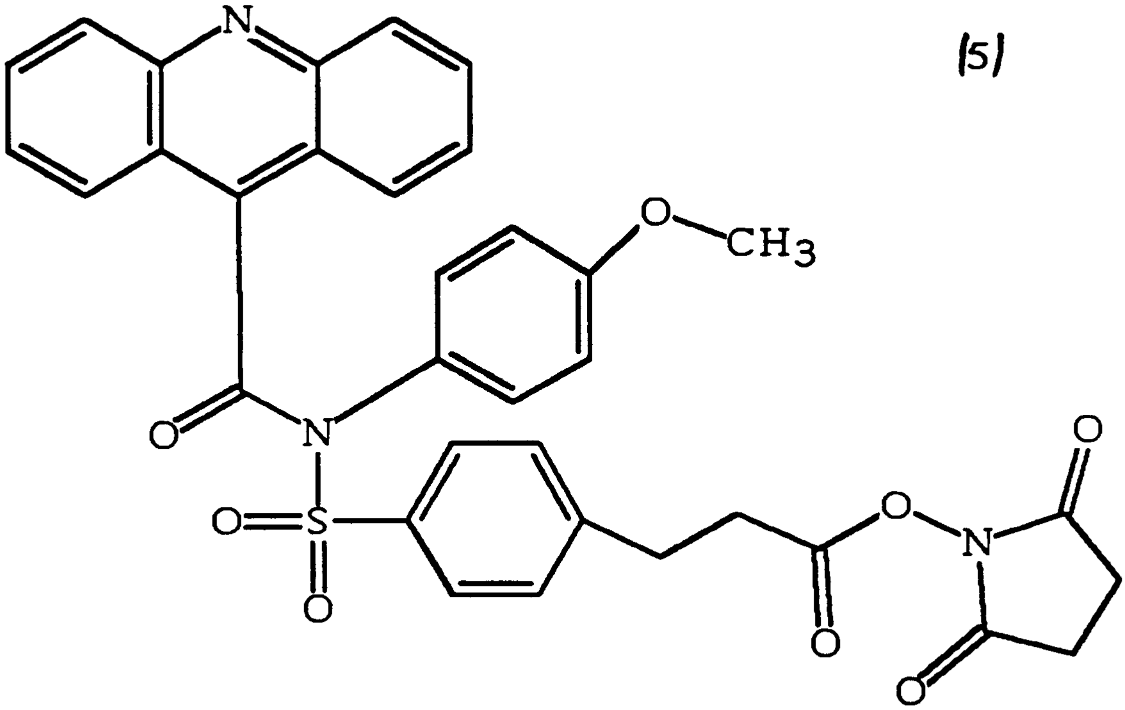

(5)

organische Phase wird eingeengt und der Rückstand aus Toluol umkristallisiert.

Ausbeute: 50 %
NMR (DMSO, 100 MHz) δ= 2.8 ppm (s,4H), δ= 3,2 ppm (s,br,4H), δ= 3,5 ppm (s,Br,3H), δ= 6,5 ppm (d,br,2H), δ= 7,2 ppm (d,br,2H), δ= 7,6-8,4 ppm (m,12H)
IR: 3400 $cm^{-1}$ (br), 3060, 2930, 1815(w), 1780(w), 1740(s), 1690(m), 1600(w), 1510(m), 1370(m), 1250(m). 1203(m), 1175(m).

N-(4-Methoxyphenyl)-N-[4(2-succinimidyloxycarbonylethyl)benzolsulfonyl]-10-methylacridinium-9-carbonsäureamid-fluorosulfonat (6)

1,27 g (5) in 60 ml Dichlormethan werden bei -20°C mit 0,4 ml Methylfluorosulfonat versetzt. Man läßt 2 Stunden bei -20°C rühren und über Nacht auf Raumtemperatur auftauen. Auf Zusatz von Toluol fällt ein gelber Feststoff aus, der abgesaugt und im Vakuum getrocknet wird.

Ausbeute: 80 %
NMR (DMSO, 100 MHz): δ= 2.9 ppm (s.4H), δ= 3.2 ppm (s,br,4H), δ= 3,5 ppm (s,br,3H), δ= 4,8 ppm (s,br,3H), δ= 6,5 ppm (br, 2H), δ= 7,2 ppm (br,2H), δ= 7,6-9,0 ppm (m,12H)
IR= 3400 $cm^{-1}$ (br), 3160, 2970, 1810(w), 1785(w), 1740(s), 1695(m), 1600(w), 1555(w), 1510(m), 1370(m), 1290(m), 1250(m), 1210(m), 1170(m)
Massenspektrum: m/Z: 652 $M^+$ (Kation)

**Beispiel 2:**

Die Darstellung von N-(4-methoxyphenyl)-N-[4-(4-succinimidyl¨ oxycarbonylbutyl)benzolsulfonyl]-10-methylacridinium-9-carbonsäureamid-fluorosulfonat (11) ausgehend von 4′-N-(4-Methoxyphenyl)sulfamido]-5-phenylvaleriansäurebenzylester (7) und Acridin-9-carbonsäurechlorid-hydrochlorid (2) erfolgt analog zur Synthese von (6). Die Ausbeuten der einzelnen Syntheseschritte sowie die spektroskopische Charakterisierung sind nachfolgend angegeben.

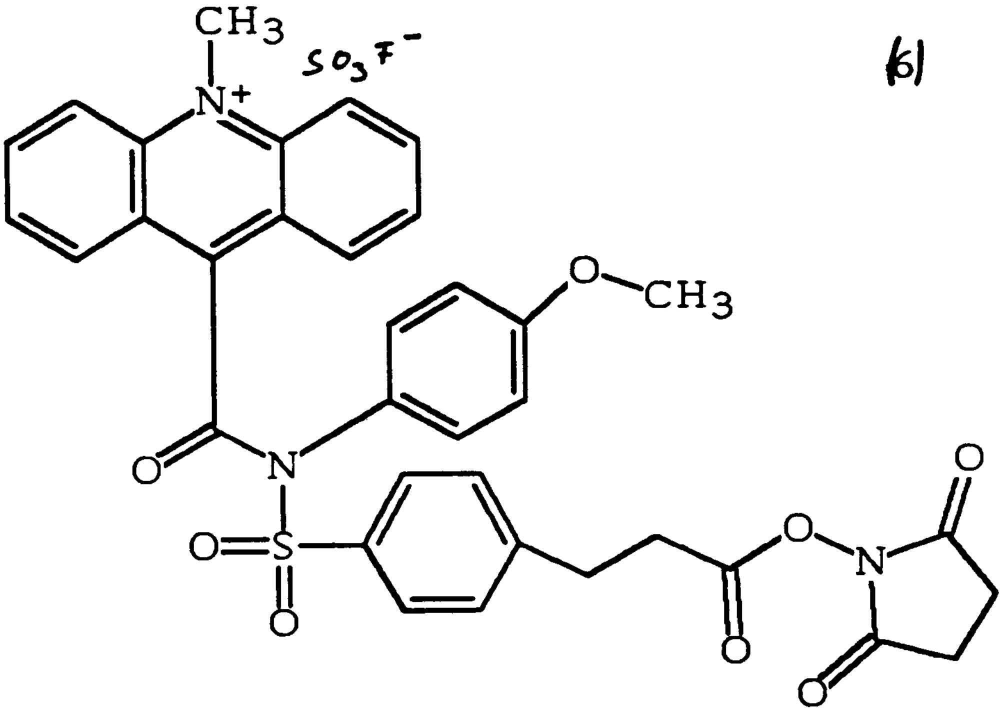

(6)

N-(4-Methoxyphenyl)-N-[4-(4-benzyloxycarbonylbutyl)benzolsulfonyl]acridin-9-carbonsäureamid (8)

Ausbeute: 40 % zähes Öl, erstarrt teilweise
NMR ($CDCl_3$, 100 MHz): δ= 2,85 (m,4H), δ= 2,45 ppm (t,br,2H), δ= 2,8 ppm (t,br,2H), δ= 3,5 ppm (s,3H), δ= 5,15 ppm (s,2H), δ= 6,3 ppm (d,2H), δ= 6,9 ppm (d,2H), δ= 7,3-8,3 ppm (m,17H).

N-(4-methoxyphenyl)-N-[4-(4-carboxybutyl)benzolsulfonyl]acridin-9-carbonsäureamid-hydrobromid (9)

Ausbeute: 95 % Schmelzpunkt: Zersetzung 153-5°C
NMR (DMSO, 100 MHz): δ= 1,7 ppm (s,br,4H), δ= 2,3 ppm (t,br,2H), δ= 2,8 ppm (s,br,2H), δ= 3,5 ppm (s,br,3H), δ= 6,5 ppm (br,2H), δ= 7,05 ppm (br,2H), δ= 7,5-8,5 ppm (m,12H).

N-(4-Methoxyphenyl)-N-[4-(4-succinimidyloxycarbonylbutyl)benzolsulfonyl]acridin-9-carbonsäureamid (10)

Ausbeute: 25 % Schmelzpunkt: Zersetzung 75 - 80°C
NMR (DMSO, 100 MHz): δ= 1,8 ppm (br,4H), δ= 2,3 ppm (s,2H), δ= 2,85 ppm (s,br,6H), δ= 3,5 ppm (s,br,3H), δ= 6,5 ppm (d,br,2H), δ= 7,05 ppm (d,br,2H), δ= 7,5-8,3 ppm (m,12H)

N-(4-Methoxyphenyl)-N-[4-(4-succinimidyloxycarbonylbutyl)benzolsulfonyl]-10-methylacridinium-9-carbonsäureamid-fluorosulfonat (11)

Ausbeute: 90 %
NMR (DMSO, 100 MHz): δ= 1,8 ppm (br,4H), δ= 2,3 ppm (s,br,2H), δ= 2,8 ppm (s,br,6H), δ= 3,5 ppm (s,br,3H), δ= 4,8 ppm (br,3H), δ= 6,5 ppm (br,2H), δ= 7,05 ppm (br,2H), δ= 7,5-9,0 ppm (m,12H)
IR: 3340 $cm^{-1}$ (br), 3060(w), 2930(m), 2870(w), 1810(w), 1785(w), 1740(s), 1695(m), 1600(w), 1550(w), 1510(m), 1460(w), 1370(m), 1290(s), 1250(s), 1205(s), 1170(s)
Massenspektrum: m/z= 680 $M^+$ (Kation)

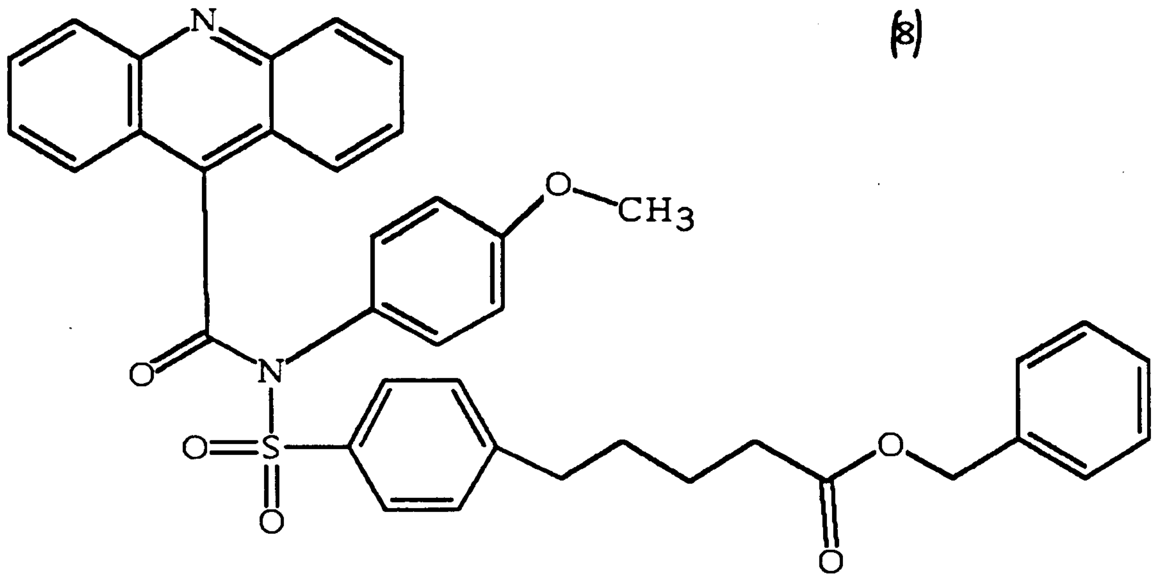

(8)

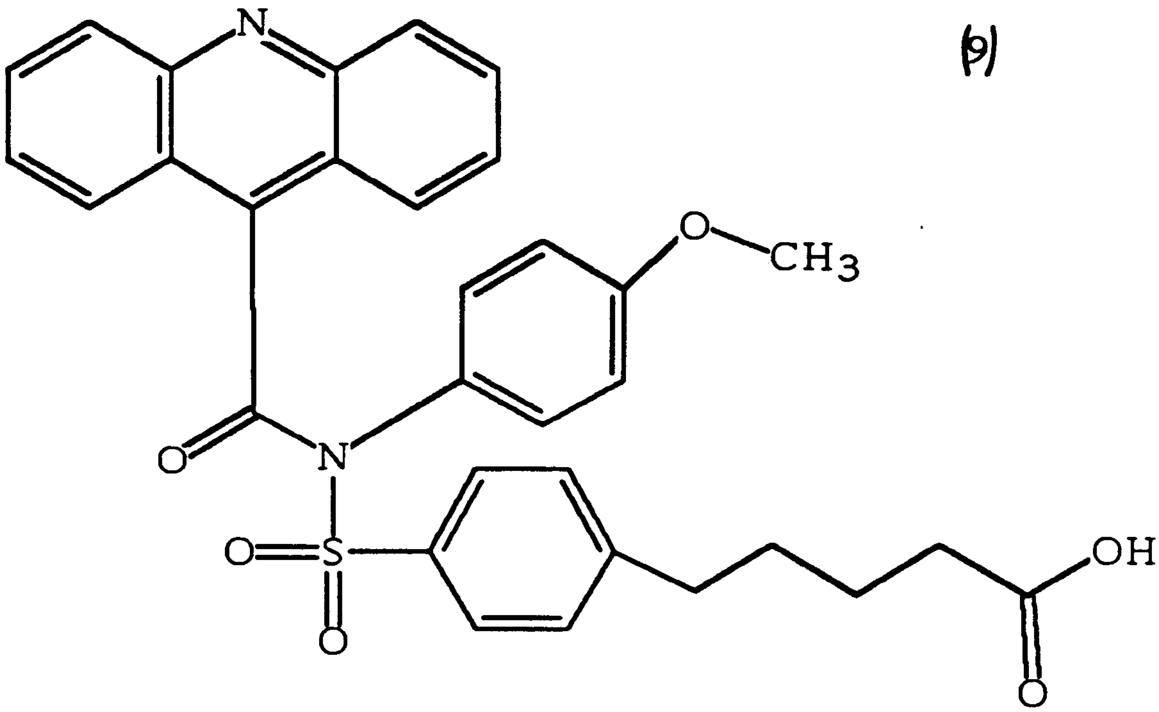

(9)

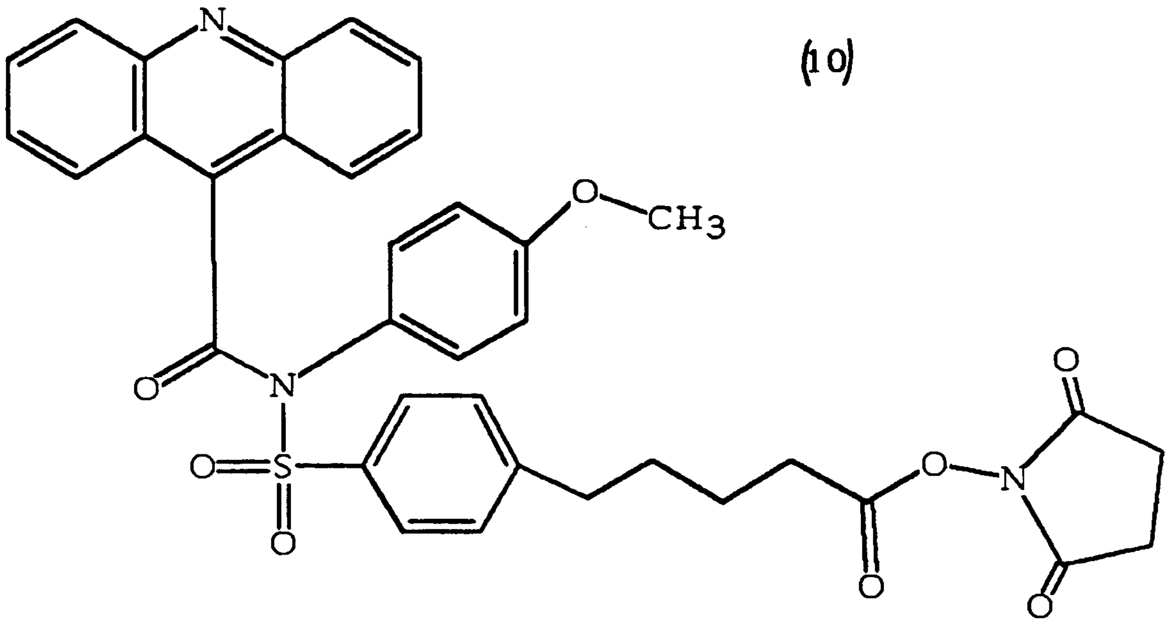

(10)

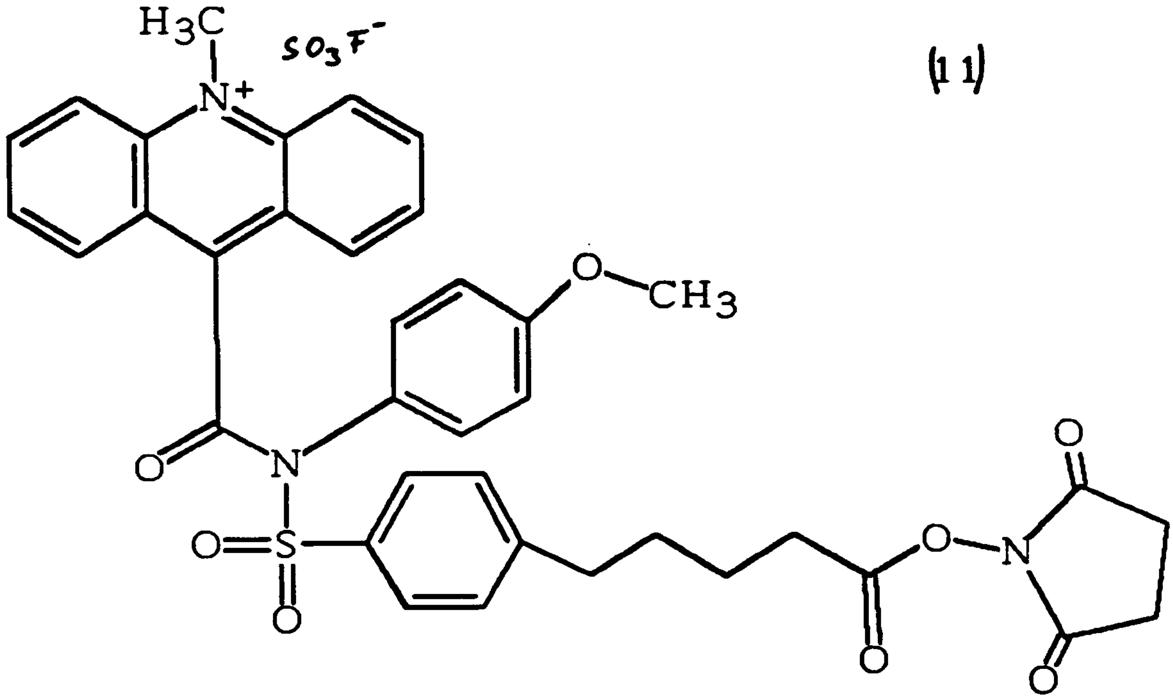

(11)

**Beispiel 3:**

Die Darstellung von N-(2,4-Dimethoxyphenyl)-N-[4-(2-succinimidyloxycarbonylethyl-benzolsulfonyl]-10-methylacridinium-9-carbonsäureamid-fluorosulfonat (16a) ausgehend von 4′-|N-(2,4-Dimethoxyphenyl)sulfamido]-3-phenylpropionsäurebenzylester (12a) und Acridin-9-carbonsäurechloridhydrochlorid (2) erfolgt analog zur Synthese von (6). Die Ausbeuten der einzelnen Syntheseschritte sowie die spektroskopische Charakterisierung sind nachfolgend angegeben.

N-(2,4-Dimethoxyphenyl)-N-[4-(2-benzyloxycarbonylethyl)benzolsulfonyl]acridin-9-carbonsäureamid (13a)

Ausbeute: 50 % Schmelzpunkt: 74°C
NMR (DMSO, 100 MHz): δ= 2,9 ppm (d,br,2H), δ= 3,1 ppm (d,br,2H), δ= 3,3 ppm (s,3H), δ= 3,4 ppm (s,3H), δ= 5,1 ppm (s,2H), δ= 5,9-6,2 ppm (m,2H), δ= 7,0 ppm (d,1H), δ= 7,35 ppm (s,5H), δ= 7,5-8,2 ppm (m,12H)

N-(2,4-Dimethoxyphenyl)-N-[4-(2-carboxyethyl)benzolsulfonyl]acridin-9-carbonsäureamid-hydrobromid (14a)

Ausbeute: 95 %
NMR (DMSO, 100 MHz): δ= 2,75 ppm (d,br,2H), δ= 3,05 ppm (d,br,2H), δ= 3,3 ppm (s,3H), δ= 3,5 ppm (s,3H), δ= 5,95-6,3 ppm (m,2H), δ= 7,05 ppm (d,1H), δ= 7,6-8,6 ppm (m,12H), δ= 9,2 ppm (s,br,2H).

N-(2,4-Dimethoxyphenyl)-N-[4-(2-succinimidyloxycarbonylethyl)benzolsulfonyl]acridin-9-carbonsäureamid (15a)

Ausbeute: 45 % Schmelzpunkt: ~ 105°C Zersetzung
NMR (DMSO, 100 MHz): δ= 2,9 ppm (s,4H), δ= 3 ppm (br,2H), δ= 3,2 ppm (s,3H), δ= 3,4 ppm (s,3H), δ= 5,9-6,3

ppm (m,2H), δ= 7,0 ppm (d,1H), δ= 7,5-8,4 ppm (m,12H) IR (KBr-Pressling): 3440 $cm^{-1}$ (br), 3060 (w), 2930(w),

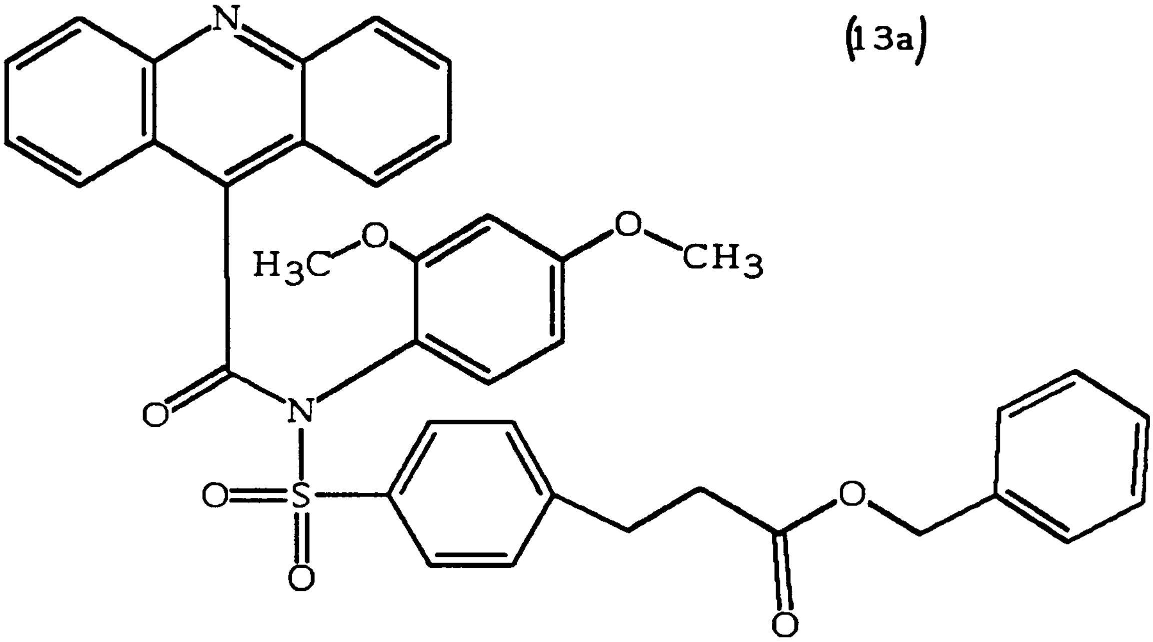

(13a)

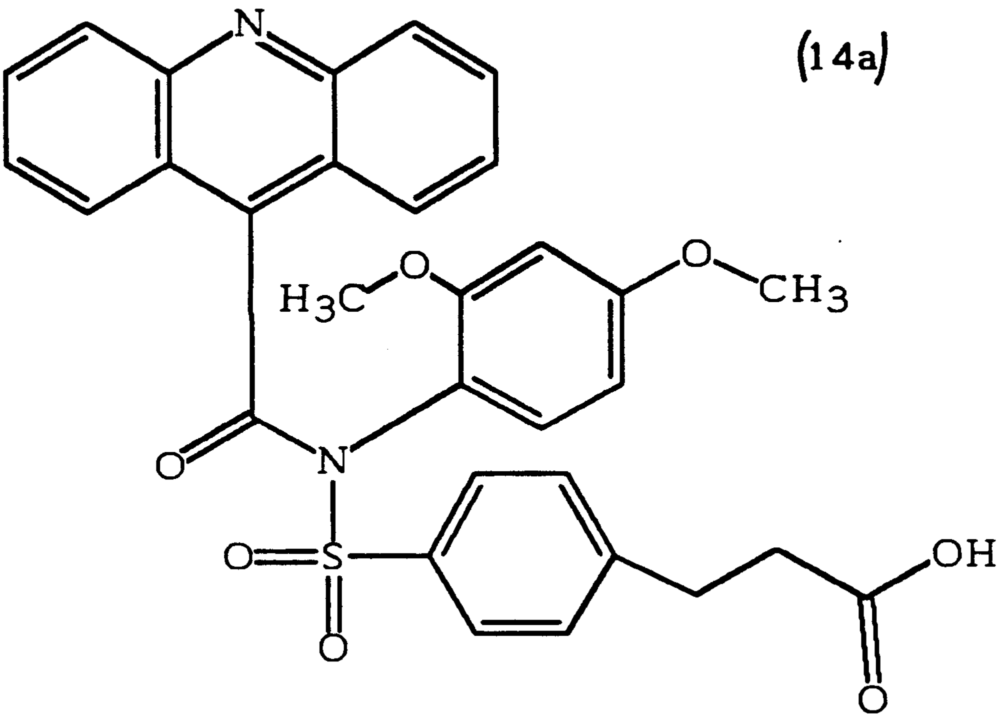

(14a)

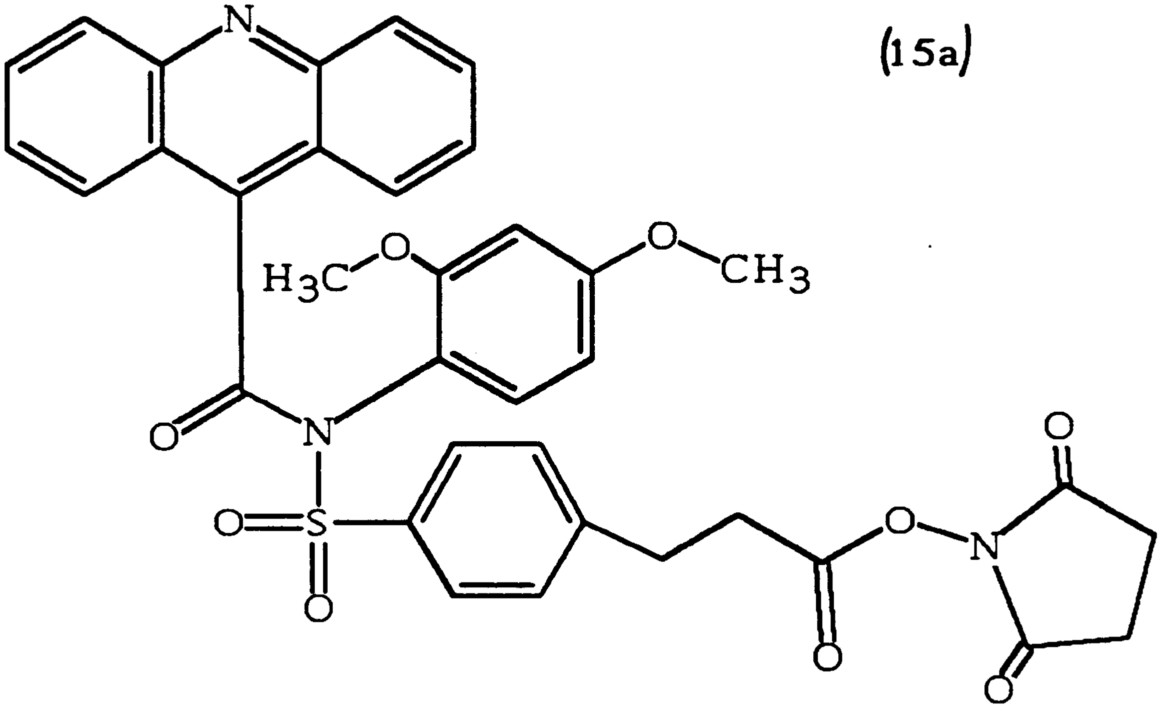

(15a)

2850(w), 1815(w), 1785(w), 1740(s), 1695(m), 1600(w), 1510(m), 1460(w), 1440(w), 1365(m), 1320(w), 1290(w), 1240(m), 1210(s), 1165(m), 1085(m)

N-(2,4-Dimethoxyphenyl)-N-[4-(2-succinimidyloxycarbonylethyl)benzolsulfonyl]-10-methylacridinium-9-carbonsäureamid-fluorosulfonat (16a)

Ausbeute: 80 % Schmelzpunkt: ~ 135°C Zersetzung
NMR (DMSO, 100 MHz): δ= 2,9 ppm (s, 4H), δ= 2,95-4,2 ppm (m 10H), δ= 4,8-5,0 ppm (s,s,3H), δ= 6,05-6,25 ppm (m,1H), δ= 7,6-9,0 ppm (m,14H)
IR (KBr-Pressling): 3430 $cm^{-1}$(m), 2950(w), 2870(w), 2825(w), 1810(w), 1780(m), 1750(s), 1695(m), 1610(m), 1555(w), 1510(m), 1465(m), 1380(m), 1285(m), 1250(m), 1210(s), 1170(m)

**Beispiel 4:**

Die Synthese von N-(3,4-Ethylendioxyphenyl)-N-[4-(2-succinimidyloxycarbonylethyl)benzolsulfonyl]-10-methylacridinium-9-carbonsäureamid-fluorosulfonat (16b) ausgehend von 4′-[N-(3,4-Ethylendioxyphenyl)sulfamido]-3-phenylpropionsäurebenzylester (12b) und Acridin-9-carbonsäurechloridhydrochlorid (2) erfolgt analog Beispiel 1. Die Ausbeuten der einzelnen Schritte sowie die spektroskopische Charakterisierung sind nachfolgend angegeben.

N-(3,4-Ethylendioxyphenyl)-N-[4-(2-benzyloxycarbonylethyl)benzolsulfonyl]acridin-9-carbonsäureamid (13b)

Ausbeute: 50 % Schmelzpunkt: 91,5°C
NMR (DMSO, 100 MHz): δ= 2,9 ppm (d,br,2H), δ= 3,1 ppm (d, br,2H), δ= 4,0 ppm (s,Br,4H), δ= 5,1 ppm (s,2H), δ= 6,3-6,8 ppm (m,3H), δ= 7,3 ppm (s,5H), δ= 7,6-8,3 ppm (m,12H).

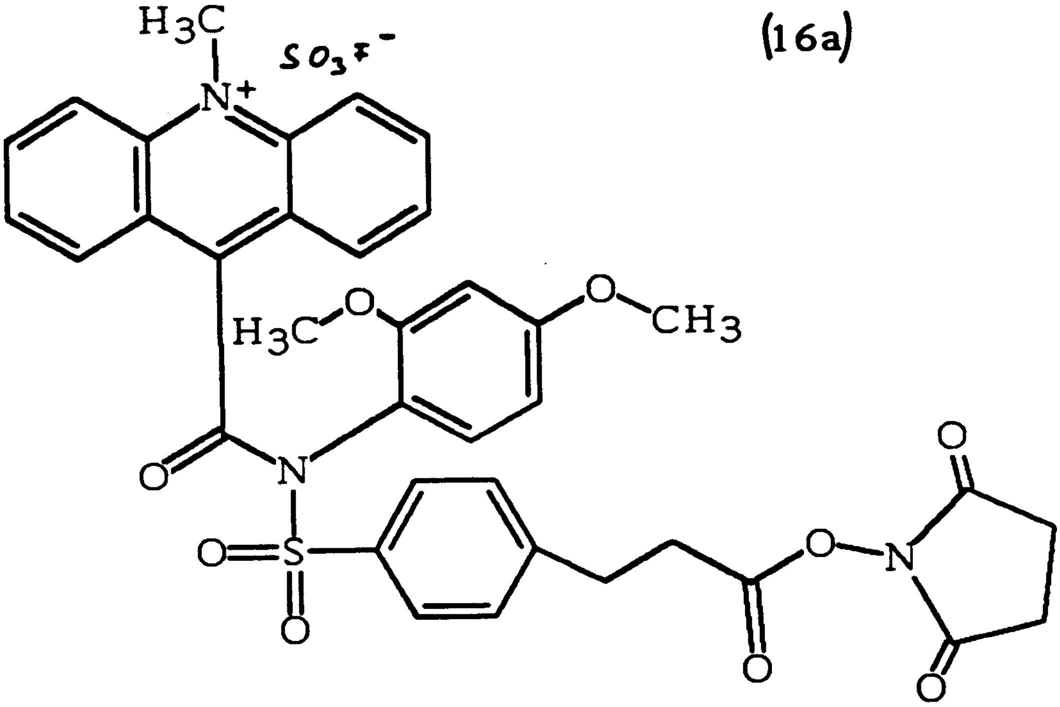

(16a)

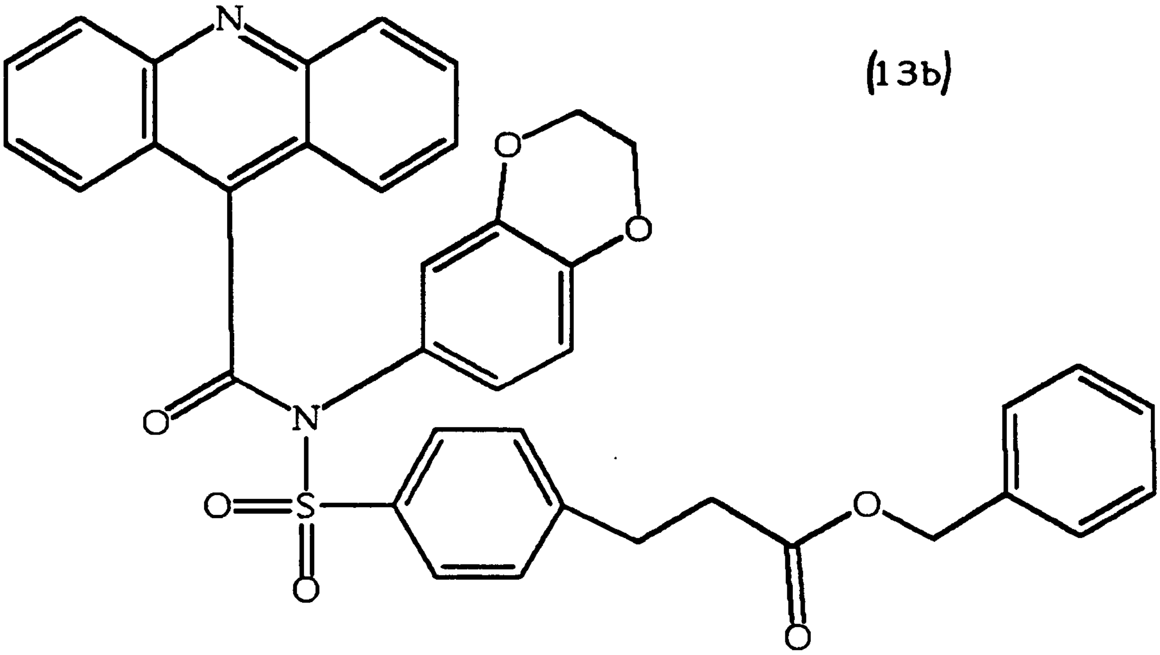

(13b)

N-(3,4-Ethylendioxyphenyl)-N-[4-(2-carboxyethyl)benzolsulfonyl]acridin-9-carbonsäureamid-hydrobromid (14b)

Ausbeute: 95 % Schmelzpunkt: > 200°C
NMR (DMSO, 100 MHz): δ= 2,7 ppm (m,2H), δ= 3,05 ppm (m,2H), δ= 4,0 ppm (s,br,4H), δ= 6,3 - 6,8 ppm (m,3H), δ= 7,5-8,6 ppm (m,12H), δ= 9,6 ppm (s,br,2H).

N-(3,4-Ethylendioxyphenyl)-N-[4-(2-succinimidyloxycarbonylethyl)benzolsulfonyl]acridin-9-carbonsäureamid (15b)

Ausbeute: 45 % Schmelzpunkt: 140°C Zersetzung
NMR (DMSO, 100 MHz): δ= 2,7-2,9 ppm (d,s, überlagert, 6H), δ= 3,0 ppm (d,2H), δ= 4,0 ppm (s,br,4H), δ= 6,3-6,8 ppm (m,3H), δ= 7,5-8,4 ppm (m,12H)
IR(KBr-Pressling): 3420 $cm^{-1}$ (br), 3060(m), 2980(m), 2930(m), 1810(w), 1790(w), 1740(m), 1695(s), 1590(m). 1460(w), 1430(w), 1410(w), 1370(m), 1300(m), 1225(s), 1175(s).

N-(3,4-Ethylendioxyphenyl)-N-[4-(2-succinimidyloxycarbonylethyl)benzolsulfonyl]-10-methylacridinium-9-carbonsäureamid-fluorosulfonat (16b)

Ausbeute: 80 % Schmelzpunkt: ~ 110°C, Zersetzung
NMR (DMSO, 100 MHz): δ= 2,85 ppm (s,4H), δ= 3,0-3,3 ppm (s,s,br,4H), δ= 3,8-4,5 ppm (m,br,4H), δ= 4,75-5,1 ppm (s, br mit Schulter, 3H), δ= 6,3-9,0 ppm (m,15H).

**Beispiel 5:**

N-(4-Carboxyphenyl)-4-toluolsulfonsäureamid (5-1)

Zu einer Mischung von 252 g (3 Mol) Natriumhydrogencarbonat und 139,1 g (1 Mol) 4-Aminobenzoesäure in 2,5 l Wasser tropft man bei 20 - 30°C eine solche von 190,5 g (1 Mol) 4-Toluolsulfochlorid in 300 ml i-Propylether. Man rührt intensiv 2-4 Stunden, bis das Sulfochlorid verbraucht ist. Die abgetrennte wäßrige Lösung stellt man mit konz.

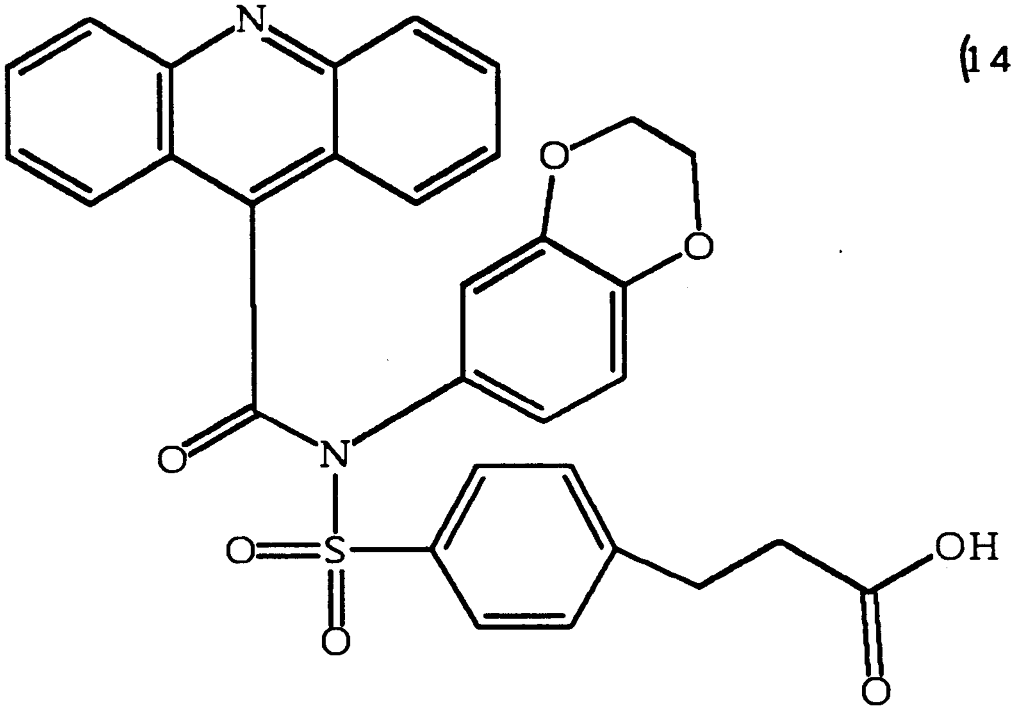

(14b)

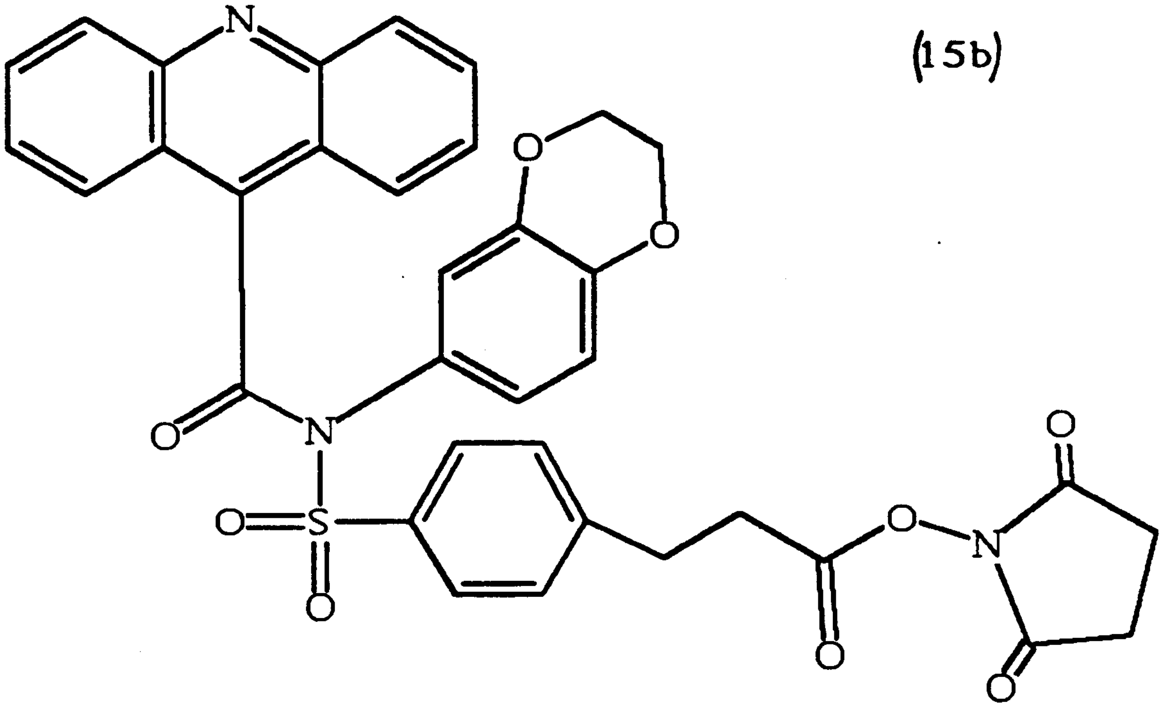

(15b)

(16b)

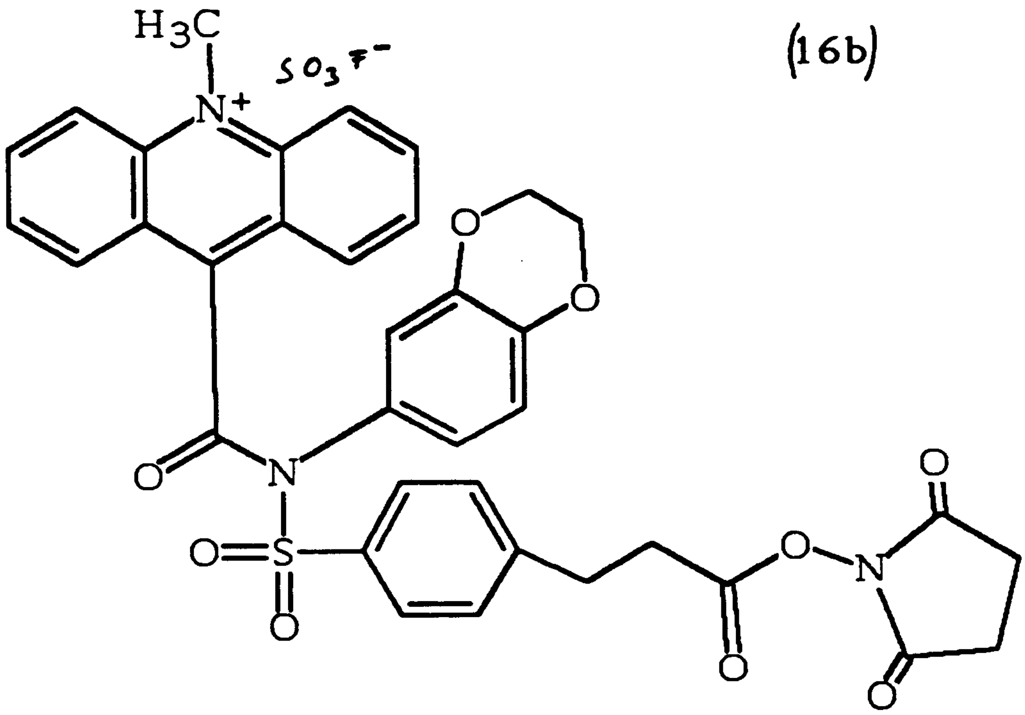

(5-1)

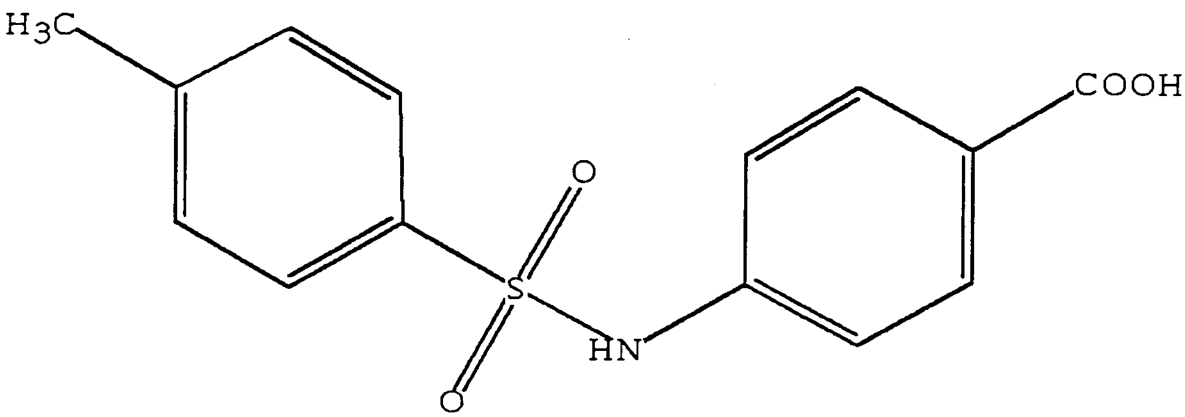

Salzsäure auf pH 1 und nimmt die Fällung in Propylacetat auf. Den Extract wäscht man 2x mit 2n-Salzsäure, 1x mit Wasser aus, trocknet über Natriumsulfat und dampft ein. Man erhält 240 g, 82,5 % d.Th.)

N-(4-Carboxyphenyl)-4-toluolsulfonsäureamid

$^{1}$H-NMR (DMSO $d_6$): δ= 2,3 (s; $CH_3$); 7,1 (d, Aromat, 2H) 7,3 (d, Aromat, 2H); 7,6-7,9 (m, Aromat, 4H); 10,75 (breit); 12,7 (breit).

N-(4-Benzyloxycarbonylphenyl)-4-toluolsulfonsäureamid (5-2)

Eine Lösung von 11,64 g (40 mMol) N-(4-Carboxyphenyl)-4-toluolsulfonsäureamid, 5,06 g (40 mMol) Benzylchlorid, 5,20 g (44 mMol) Di-i-propyl-ethylamin in 100 ml Dimethylformamid erhitzt man 4 Stunden auf 140°C. Nach beendeter Reaktion dampft man im Vakuum ein, nimmt auf in Propylacetat, wäscht 2x mit 2n Salzsäure, 2x mit gesättiger $NaHCO_3$-Lösung, trocknet über Natriumsulfat und dampft ein. Man erhält 11,8 g (78 % d.Th.) N-(4-Benzyloxycarbonylphenyl)-4-toluolsulfonsäureamid, der aus Methanol umkristallisiert wird.

$^1$H-NMR (DMSO $d_6$): δ= 2,3 (s; $CH_3$); 5,3 (s, $CH_2$); 7,1-7,3 (dd, 4 aromat. H); 7,4 (s, $C_6H_5$); 7,7-7,9 (dd, 4 aromat. H); 10,8 (breit, NH).

N-(4-Benzyloxycarbonylphenyl)-N-(4-toluolsulfonyl)-acridin-9-carbonsäureamid (5-3)

Zu der Lösung von 1,5 g (4 mMol) N-(4-Benzyloxycarbonyl phenyl)-4-toluolsulfonsäureamid, 1,23 g (4,4 mMol) Acridincarbonsäurechlorid-hydrochlorid und 0,02 g Dimethylaminopyridin in 20 ml wasserfreiem Tetrahydrofuran tropft man bei 25°C 2,1 ml (15 mMol) Triethylamin in 10 ml Tetrahydrofuran. Man steigert die Temperatur auf 60°C.

(5-2)

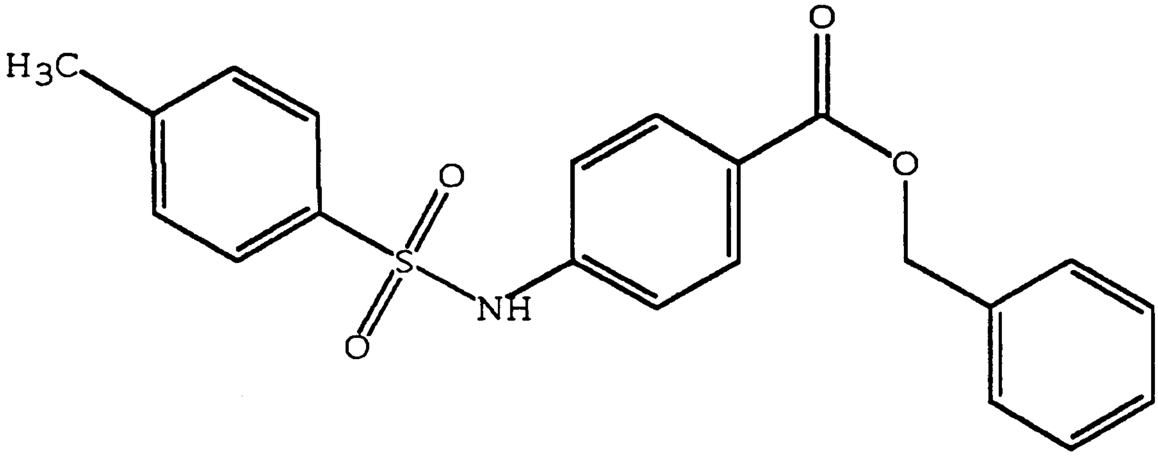

(5-3)

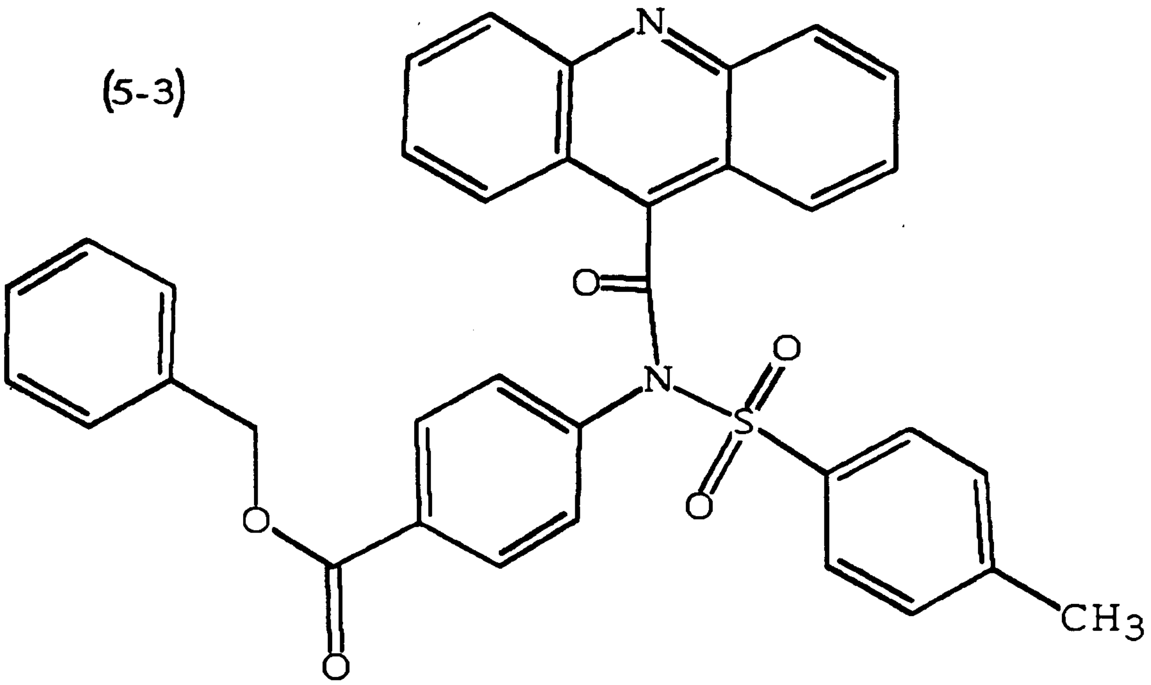

Das auskristallisierende Produkt wird nach beendeter Reaktion mit Methanol verrührt abgesaugt und aus Ethylacetat umkristallisiert.

Ausbeute: 1,57 (67,0 % d.Th.)
$^1$H-NMR ($CDCl_3$): δ= 2,5 (s, $CH_3$); 5,2 (s, $CH_2$); 7,3 (s, $C_6H_5$), 7,0-8,2 (m, 16 aromat. H).

N-(4-Carboxyphenyl)-N-(4-toluolsulfonyl)-acridin-9 carbonsäureamid-hydrobromid (5-4)

1,17 g (2 mMol) N-(4-Benzyloxycarbonylphenyl)-N-(4-toluolsulfonyl)-acridin-9-carbonsäureamid werden mit 10 ml 33 %iger Bromwasserstoff-Eisessiglösung unter Rühren 4 Stunden auf 60°C erwärmt. Nach Abkühlung saugt man die Fällung ab und trocknet i.V. Ausbeute 1,00 g (87 % d.Th.)

$^1$H-NMR (TFA): δ= 2,6 (s, $CH_3$); 7,3-8,6 (m, 16 aromat. H); 11,65 (s, NH); MS: 496 ($M^+$).

N-(4-succinimidyloxycarbonylphenyl)-N-(4-toluolsulfonyl)acridin-9-carbonsäureamid (5-5)

Zu einer Lösung von 0,57 g (1 mMol) N-(4-Carboxyphenyl)-N-(4-toluolsulfonyl)-acridin-9-carbonsäureamid-hydrobromid und 0,21 g (2 mMol) Triethylamin in 25 ml wasserfreiem Tetrahydrofuran gibt man unter Rühren bei -15°C 0,11 g (1 mMol) Chlorameisensäureethylester. Man rührt 1 Stunde bei gleicher Temperatur und setzt dann 0,12 g (1 mMol) N-Hydroxysuccinimid zu. Nach einer weiteren Stunde läßt man den Ansatz bei Raumtemperatur 15 Stunden stehen. Man dampft i.V. ein, nimmt in Ethylacetat auf, wäscht mit Wasser, Natriumhydrogencarbonatlösung und Wasser aus und trocknet über Natriumsulfat. Nach dem Eindampfen erhält man 0,42 g (70,8 % d.Th.) des Zielproduktes.

$^1$H-NMR (TFA): δ= 2,6 (s, $CH_3$), 3,1 (s, $CH_2$-$CH_2$), 7,0-8,6 (m, 16 aromat. H).

(5-4)

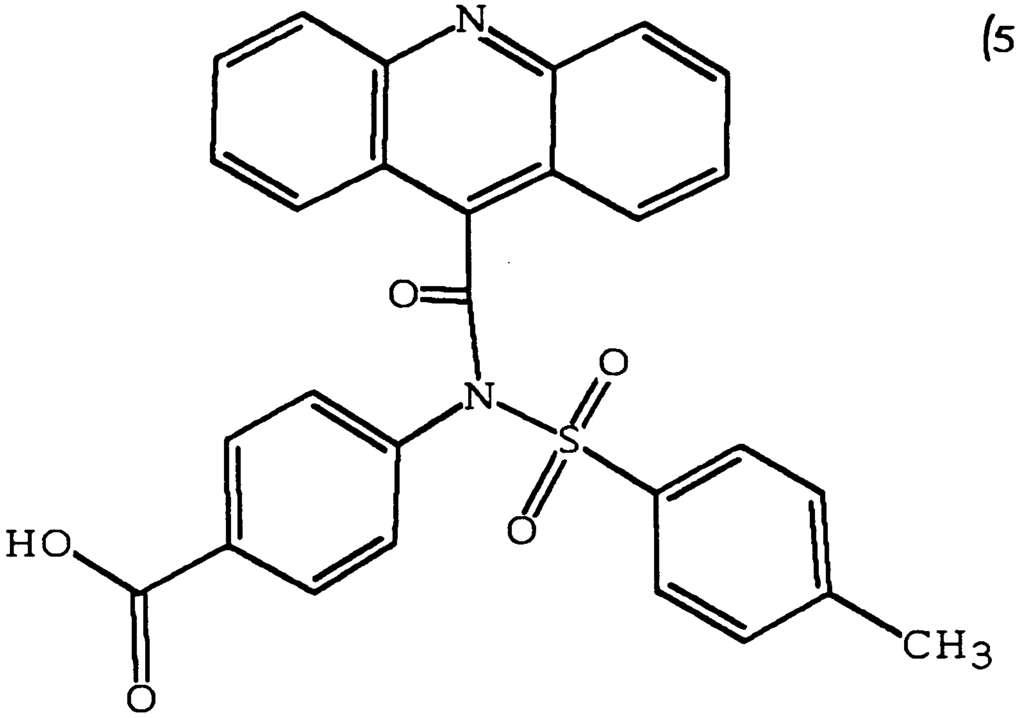

(5-5)

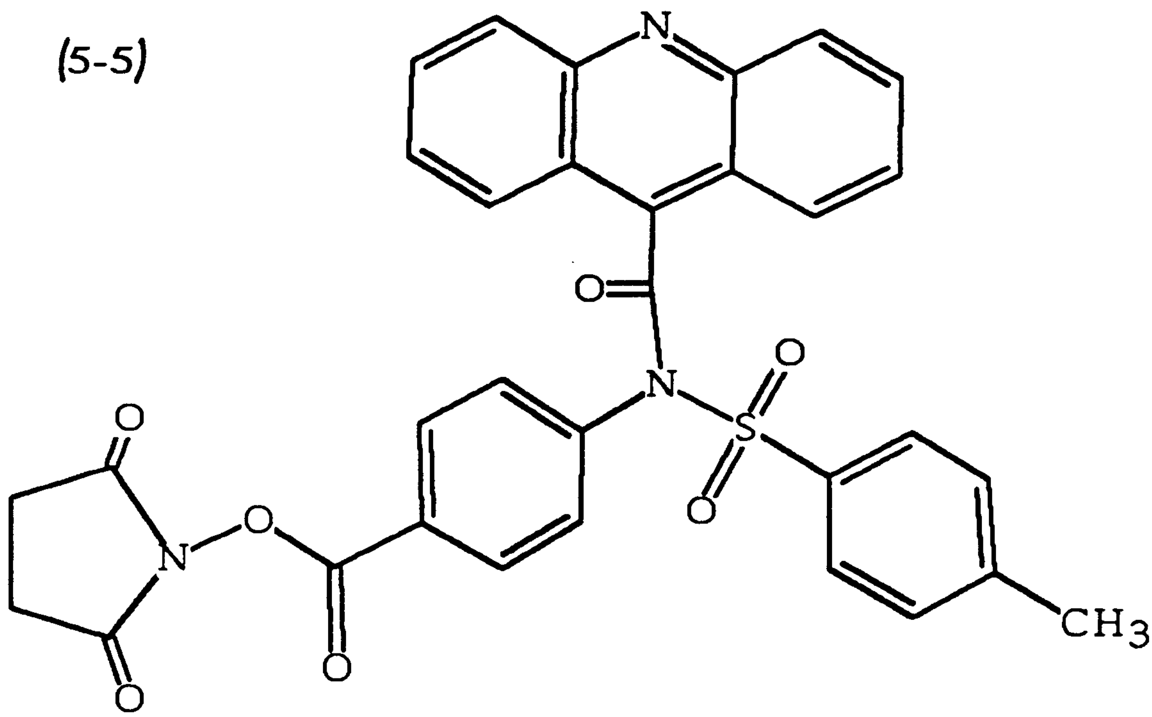

N-(4-Succinimidyloxycarbonylphenyl)-N-(4-toluolsulfonyl)-10-methylacridinium-9-carbonsäureamid-fluorosulfonat (5-6)

Zu einer Lösung von 0,59 g (1 mMol) N-(4-succinimidyloxycarbonylphenyl)-N-(4-toluolsulfonyl)-acridin-9-carbonsäureamid in 30 ml 1,2-Dichlorethan gibt man unter Rühren bei 25°C 0,85 g (7,5 mMol) Fluorsulfonsäuremethylester. Das Reaktionsprodukt fällt innerhalb von 4 Stunden aus. Nach Absaugen und Trocknen erhält man 0,43 g (60,8 % d.Th.) des Zielproduktes.

$^1$H-NMR (TFA): δ= 2,6 (s, Ar-$CH_3$); 3,1 (s, $CH_2$-$CH_2$); 4,9 (s, N-$CH_3$); 7,3-8,8 (m, 16 arom. H).

**Beispiel 6**

In gleicher Weise wie in Beispiel 5 erhält man aus N-(4-carboxymethylphenyl)-4-toluol-sulfonsäureamid N-(4-Succinimidyloxycarbonylmethylphenyl)-N-(4-toluolsulfonyl)-10-methylacridinium-9-carbonsäureamid-fluorosulfonat

N-(4-Carboxymethylphenyl)-4-toluolsulfonsäureamid (6-1)

$^1$H-NMR (DMSO): δ= 2,3 (s, $CH_3$); 3,5 (s, $CH_2$); 7,0 (AB, $C_6H_4$); 7,3-7,6 (AB; $C_6H_4$); 10.2 (s, NH).

N-(4-Benzyloxycarbonylmethylphenyl)-4-toluolsulfonsäureamid (6-2)

Ausbeute: 35 % d.Th.
$^1$H-NMR (DMSO): δ= 2,3 (s, $CH_3$); 3,6 (s, $COCH_2$); 5,1 (s, $OCH_2$); 6,9-7,7 (m, 13 aromat. H); 10,2 (s, NH).

N-(4-Benzyloxycarbonylmethylphenyl)-N-(4-toluolsulfonyl)-acridin-9-carbonsäureamid (6-3)

Ausbeute 35 % d.Th.
$^1$H-NMR ($CDCl_3$); δ= 2,55 (s, $CH_3$; 3,3 (s, $COCH_2$); 5,0 (s, O-$CH_2$), 6,7-8,2 (m, 21 aromat. H).

(5-6)

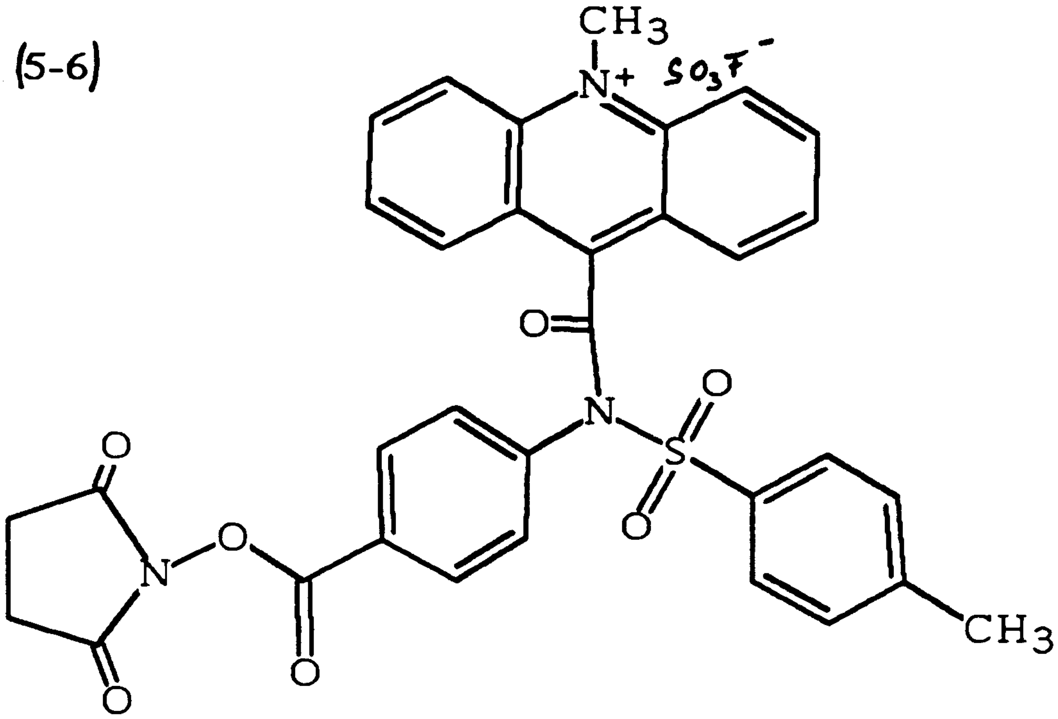

(6-1)

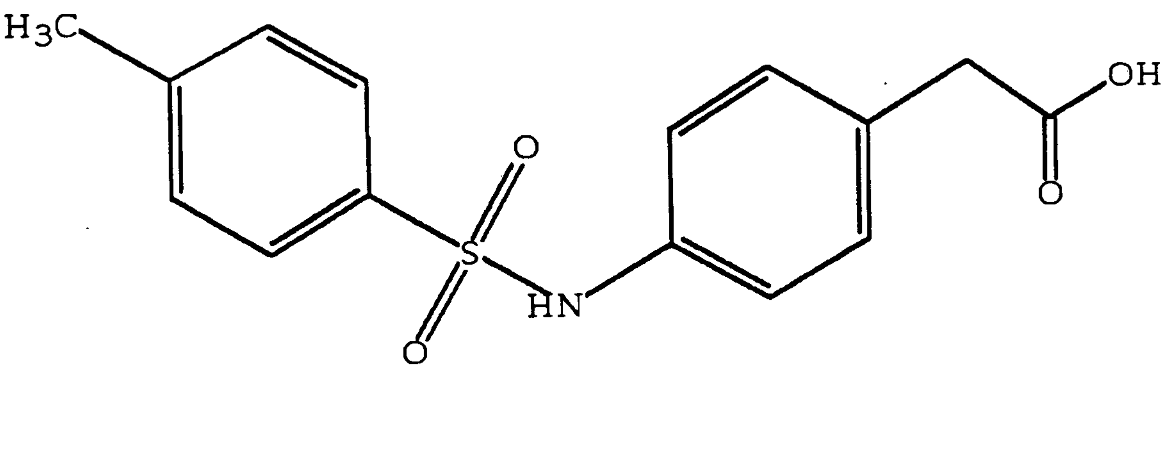

(6-2)

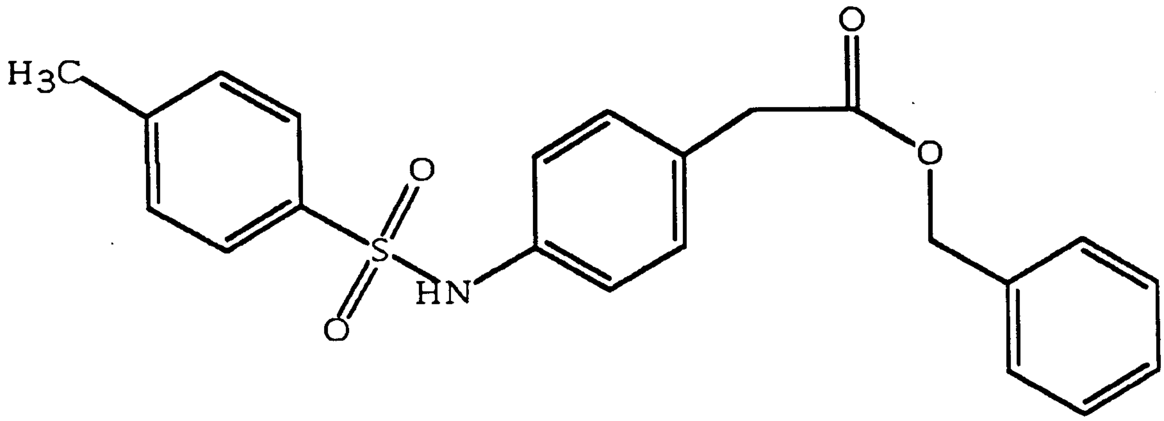

(6-3)

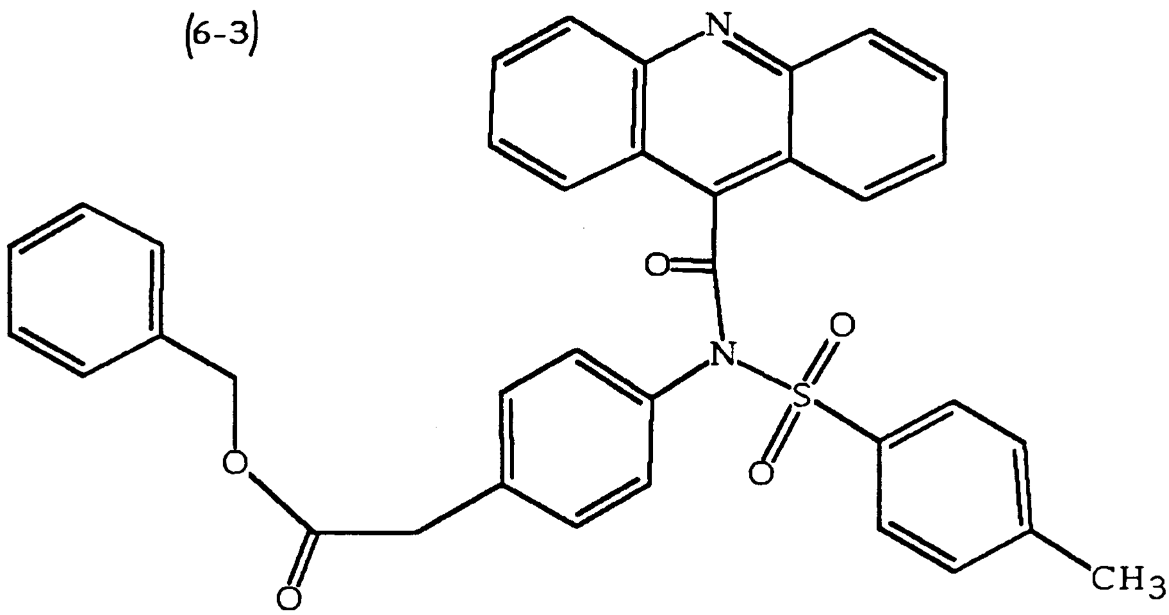

N-4-Carboxymethylphenyl)-N-(4-toluolsulfonyl)-acridin-9-carbonsäureamid-hydrobromid (6-4)

Ausbeute: 95 % d.Th.
$^1$H-NMR (TFA); δ= 2,65 (s, $CH_3$); 3,55 (s,$CH_2$); 6,8-8,6 (m, 16 aromat. H)

N-(4-Succinimidyloxycarbonylmethylphenyl)-N-(4-toluolsulfonyl)-acridin-9-carbonsäureamid (6-5)

Ausbeute: 71 % d.Th.
$^1$H-NMR (TFA): δ= 2,65 (s, Toluol-$CH_3$); 3,0 (s, $CH_2$-$CH_2$); 3,6 (breit, $COCH_2$); 6,9-8,5 (m, Aromat)

N-(4-Succinimidyloxycarbonylmethylphenyl)-N-(4-toluolsulfonyl)-10-methylacridinium-9-carbonsäureamidfluorosulfonat (6-6)

Ausbeute: 80 % d.Th.
$^1$H-NMR (TFA): δ= 2,6 (s, Toluol-$CH_3$); 2,9 (s, $CH_2$-$CH_2$); 3,6 (breit, $COCH_2$); 4,9 (s, N-$CH_3$); 6,8-8,9 (m, Aromat)

**Beispiel 7**

In gleicher Weise wie in Beispiel 5 erhält man aus N-[4-(2-Carboxyethyl)-phenyl]-4-toluolsulfonsäureamid N-[4-(2-Succinimidyloxycarbonylethyl)-phenyl]-N-(4-toluolsulfonyl)-10-methyl-acridinium-9-carbonsäureamidfluorosulfonat.

N-[4-(2-Carboxyethyl)-phenyl]-4-toluolsulfonsäureamid (7-1)

Ausbeute: 44 % d.Th.
$^1$H-NMR (DMSO): δ= 2,3 (s, $CH_3$); 2,4-2,8 (m, $CH_2$-$CH_2$); 7,0 (AB, 4H); 7,2-7,8 (AB, 4H); 10,1 (s, NH).

N-[4-(2-Benzyloxycarbonylethyl)-phenyl]-4-toluolsulfonsäureamid (7-2)

Ausbeute: 78 % d.Th.
$^1$H-NMR ($CDCl_3$): δ= 2,35 (s, $CH_3$); 2,4-3,0 (m, $CH_2$-$CH_2$); 5,1 (s, O-$CH_2$); 7,0-7,8 (13 aromat. H, NH)

(6-4)

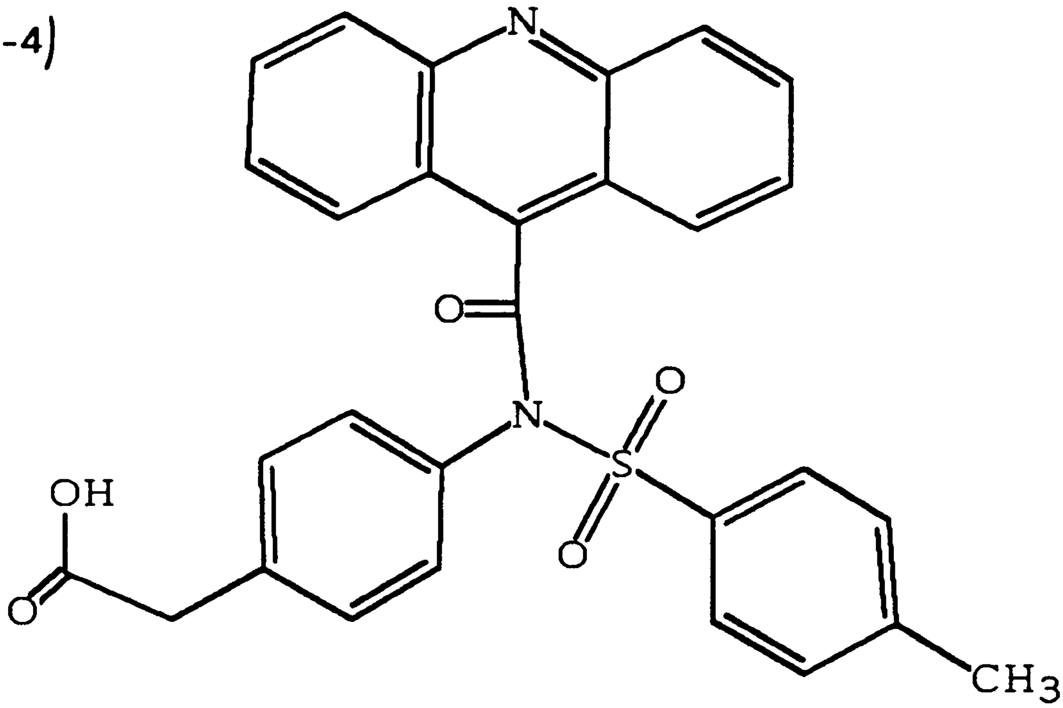

(6-5)

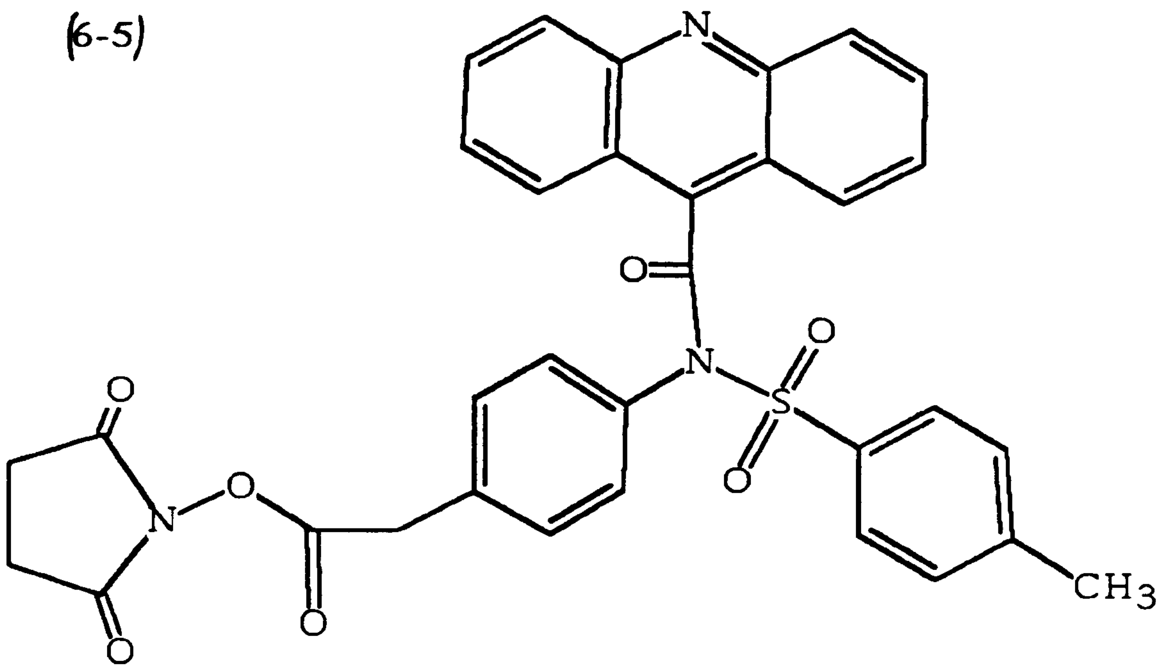

(6-6)

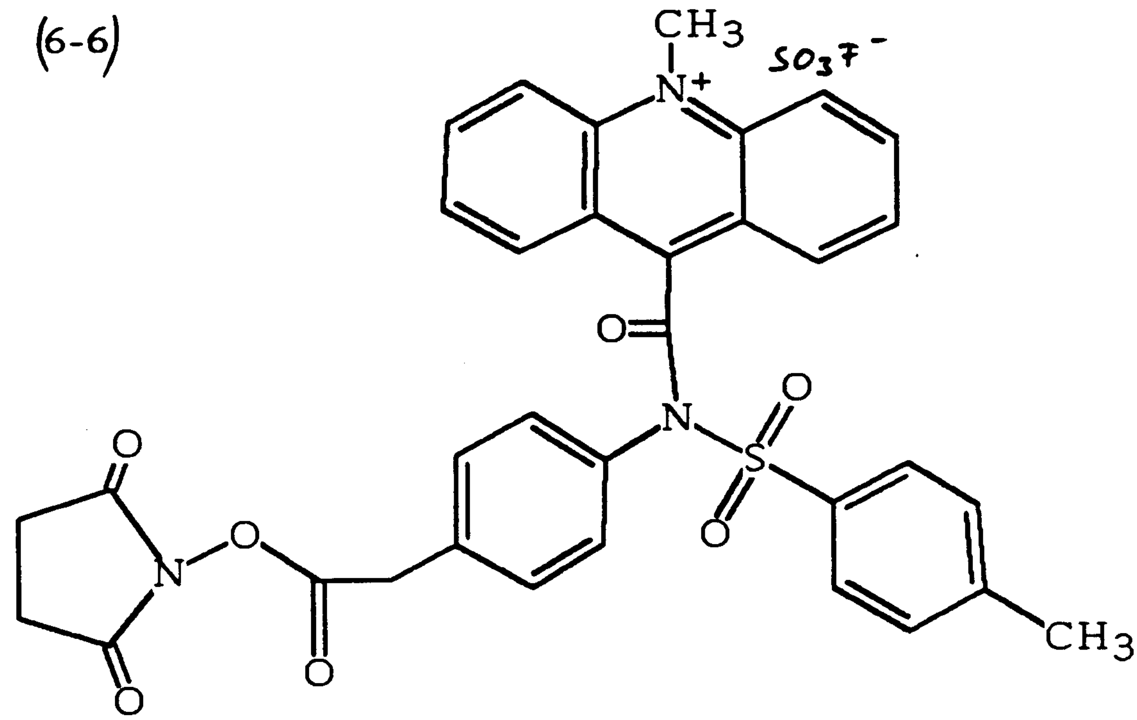

(7-1)

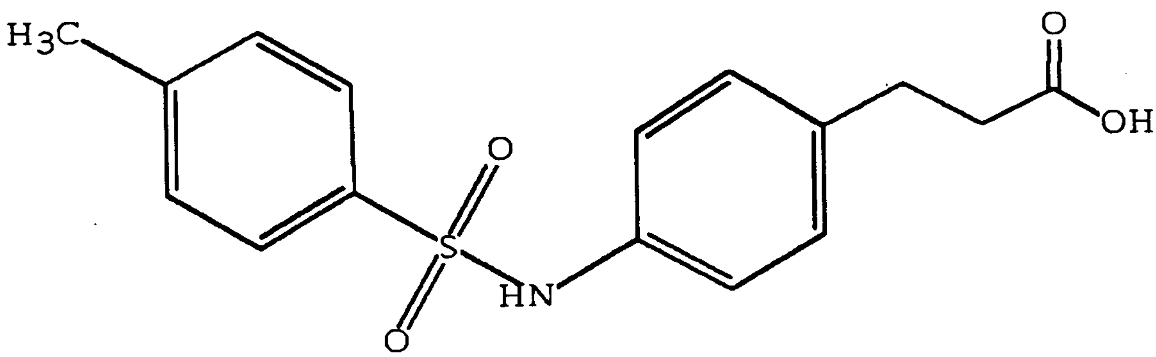

(7-2)

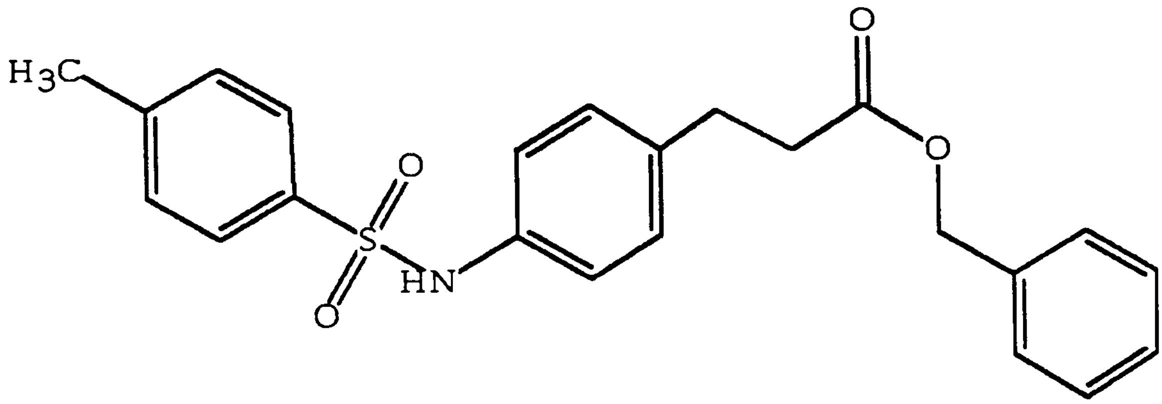

N-(4-(2-Benzyloxycarbonylethyl)-phenyl]-N-(4-toluolsulfonyl)-acridin-9-carbonsäureamid (7-3)

Ausbeute: 77 % d.Th.
$^1$H-NMR ($CDCl_3$): δ= 2,2-2,8 (m, 7H); 5,0 (s, $CH_2$); 6,65-7,0 (AB, 4 aromat. H); 7,3-8,3 (m, 17 aromat H),

N-[4-(2-Carboxyethyl)-phenyl]-N-(4-toluolsulfonyl)-acridin-9-carbonsäureamid-hydrobromid (7-4)

Ausbeute: 65 % d.Th.
$^1$H-NMR ($CD_3OD$): δ= 2,1-2,8 (m, $CH_2$-$CH_2$); 2,5 (s, $CH_3$); 6,7-8,5 (16 aromat. H)

N-(4-(2-Succinimidyloxycarbonylethyl)-phenyl)-N-(4-toluolsulfonyl)-acridin-9-carbonsäureamid (7-5)

Ausbeute: 90 % d.Th.
$^1$H-NMR ($CDCl_3$): δ= 2,6 (s, $CH_3$); 2,7 (breit, $CH_2$-$CH_2$); 2,8 (s, $CH_2$-$CH_2$); 6,6-8,3 (16 aromat. H)

N-[4-(2-Succinimidyloxycarbonylethyl)-phenyl]-N-(4-toluolsulfonyl)-10-methyl-acridinium-9-carbonsäureamidfluorosulfonat (7-6)

Ausbeute: 84 % d.Th.
$^1$H-NMR (TFA): δ= 2,6 (s, $CH_3$); 2,3-3,3 (breiter Untergrund, 11H; s,3,1); 4,9 (s, N-$CH_3$); 6,7-8,8 (16 aromat. H)

**Beispiel 7a**

In analoger Weise wie in den vorangegangenen Beispielen erhält man N-[4-(N-Methylmorpholino-N-2-ethoxy)-phenyl]-N-[4-(2-succinimidyl-oxycarbonylethyl)-phenylsulfonyl]-10-methylacridin-9-carbonsäureamid-diium-difluorosulfonat. Abweichende Verfahrensschritte sind in den folgenden Reaktionsstufen beschrieben.

(7-3)

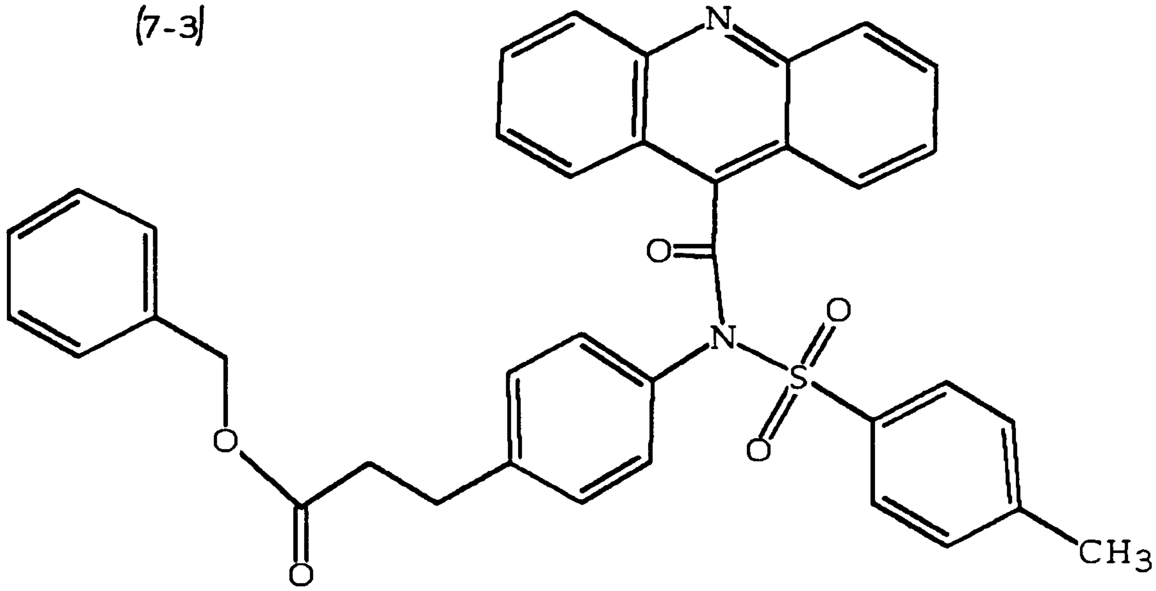

(7-4)

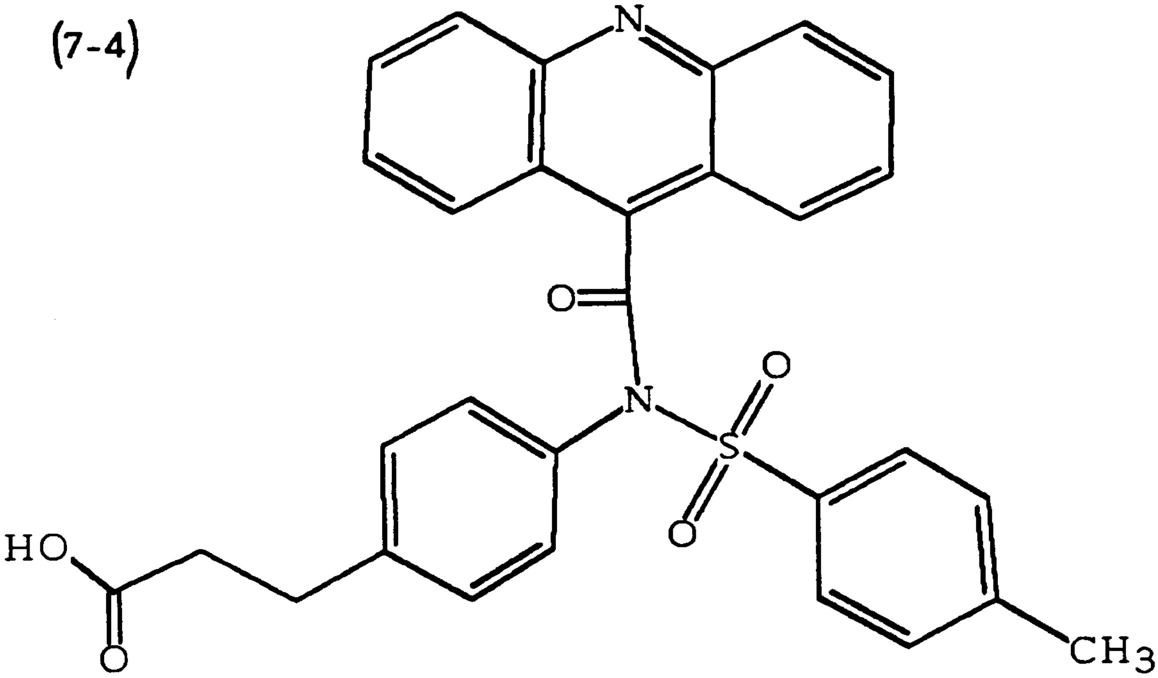

(7-5)

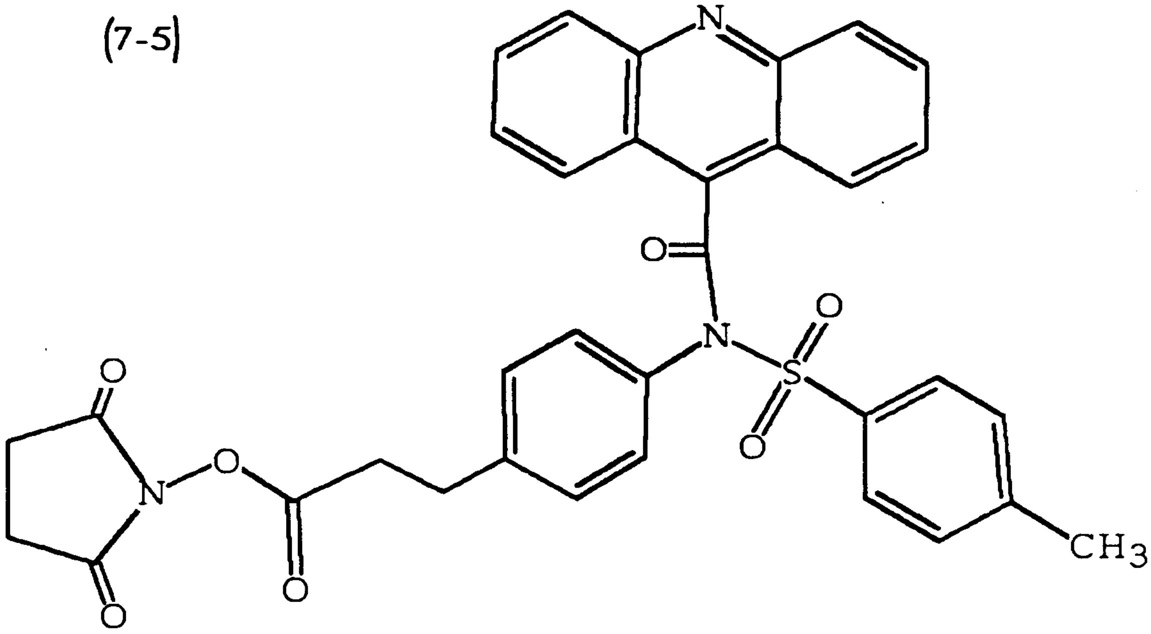

(7-6)

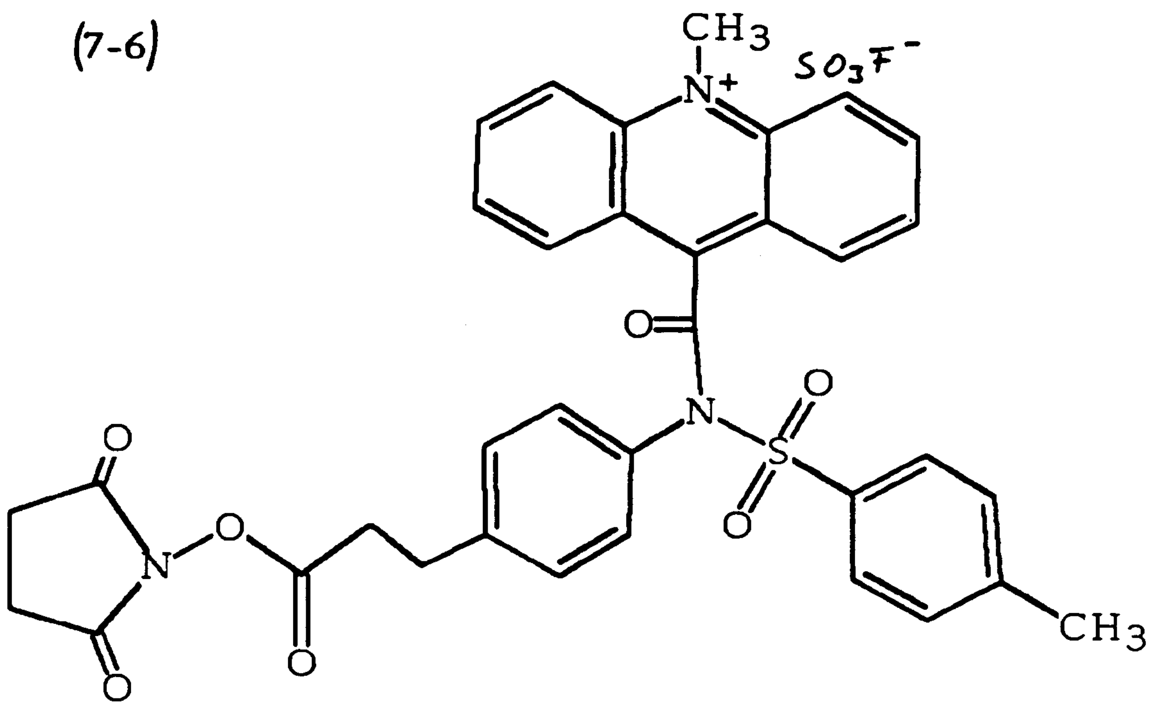

3-(4-Chlorsulfonyl-phenyl)-propionsäure-tert.butylester (7-a1)

25 g (0,1 Mol) 3-(4-Chlorsulfonyl-phenyl)-propionsäure, 12 ml tert.Butanol, 60 ml i-Buten und 3 ml konz. Schwefelsäure werden bei -15 °C, vereinigt und in einem Autoklaven bei Raumtemperatur 24 Stunden intensiv gerührt.

Die erneut auf -15 °C abgekühlte Reaktionsmischung wird in überschüssigen Natriumhydrogencarbonatlösung eingerührt, mit Methylenchlorid extrahiert und zuletzt unter Vakuum eingedampft.

Ausbeute: 20,4 g (67 % d. Th.). Das Produkt wird aus Hexan umkristallisiert.
$^1$H-NMR ($CDCl_3$): δ= 1,4 (s); 2,6 (m); 3,0 (t); 7,4 (m); 7,95 (m).
MS: $\frac{m}{z}$ = 305 ($M^+H$)

3-(4-Chlorsulfonyl-phenyl)-propionsäure wurde in bekannter Weise aus 3-Phenylpropionsäure und Chlorsulfonsäure hergestellt.

4-(Morpholino-N-2-ethoxy)anilin (7-a2)

25 g (0,1 M) 4-(Morpholino-N-2-ethoxy)-nitrobenzol werden mit 75 g Zinngranulat in 400 ml halb-konzentrierter Salzsäure 4 Stunden unter Rückfluß gekocht. Nach Abkühlen wird in 400 ml 33 %ige Salzsäure eingegossen, mit i-Propylether extrahiert und die organische Phase nach Trocknen eingedampft. Man erhält 20 g (90 % d. th.) Zielprodukt.

$^1$H-NMR ($CDCl_3$): δ = 2,5 (t); 2,7 (t); 3,5 (breit); 3,7 (t); 4,0 (t); 6,6 (m)
MS: $\frac{m}{z}$ = 222 ($M^+$)

Das gleiche Produkt erhält man durch Hydrierung der Nitroverbindung in Methanol über Palladium-Tierkohle.

(7-a1)

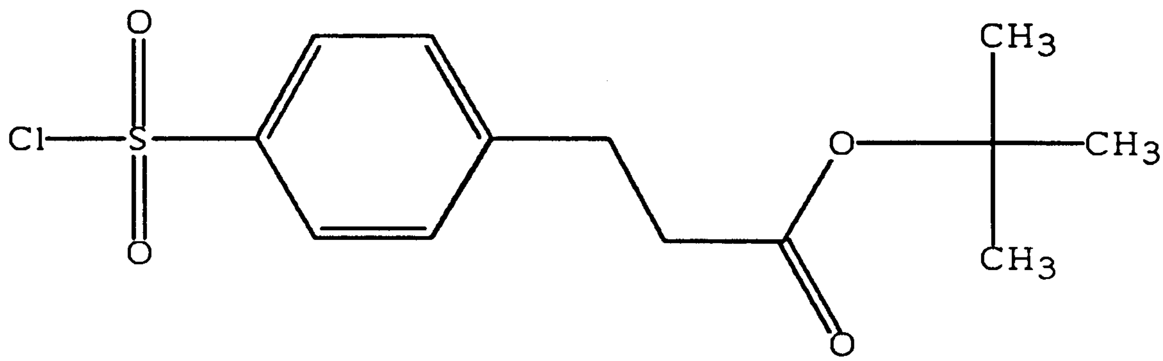

(7-a2)

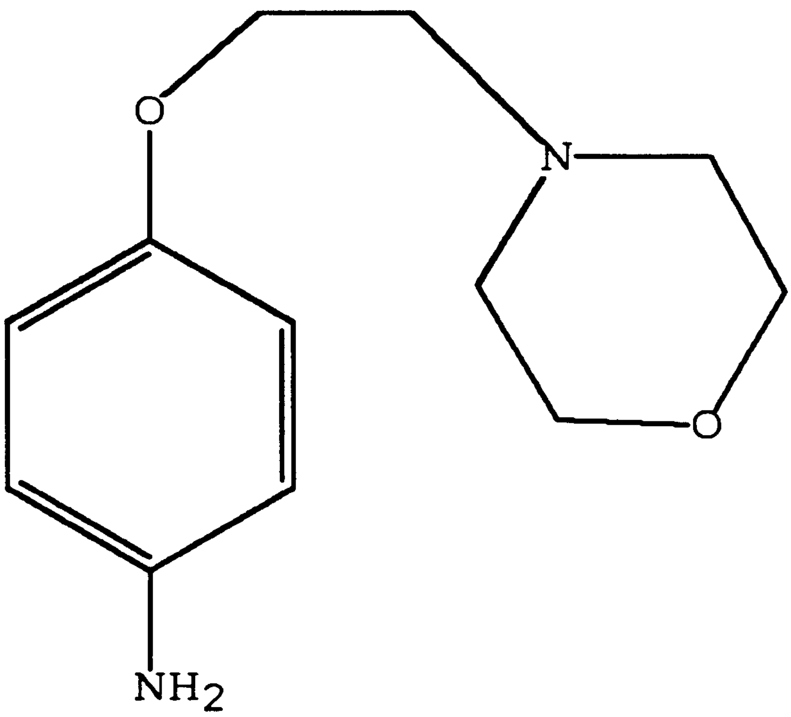

Die Nitroverbindung wurde gemäß Bull. Soc. chim. France 1955, 1353-62 hergestellt.

N-[4-(Morpholino-N-2-ethoxy)-phenyl]N-[4-(2-t-butoxycarbonylethyl)-phenylsulfonamid] (7-a3)

Die klare Lösung von 9,3 g (30 mMol) 4-chlorsulfonyl-phenylpropionsäure-t-butylester, 6,9 g 4-(Morpholino-N-2-ethoxy)-anilin und 0,3 g Dimethylaminopyridin in 150 ml Methylenchlorid wird nach 10 stündigem stehen bei Rautemperatur mit gesättigter Natriumhydrogencarbonatlösung ausgewaschen, auf 1/3 eingeengt und über eine Kieselgursäule mit einer Mischung von 90 % Methylenchlorid und 10 % Methanol chromatographiert. Die Hauptfraktion des Eluats wird eingedampft.

Ausbeute: 9 g (61,2 % d. Th.).
$^1$H-NMR ($CDCl_3$): δ = 1,35 (s); 2,5 (m); 2,7-3,0 (m); 3,7 (m); 4,0 (t); 6,7-7,0 (m); 7,2-7,7 (m)
MS: $\frac{m}{z}$ = 491 ($M^+$H).

N-[4-(Morpholino-N-2-ethoxy)-phenyl]-N-[4-(2-t-butoxycarbonylethyl)-phenylsulfonyl]-9-acridincarbonsäureamid (7-a4)

Eine Lösung von 2,0 g (4,1 mMol) N-[4-(Morpholino-N-2-ethoxy)-phenyl]-N-[4-(2-t-butoxycarbonylethyl)-phenylsulfonamid] in 50 ml Methylenchlorid versetzt man unter kräftigem Rühren nacheinander mit 50 ml 33 %iger Natronlauge, 30 mg Dimethylaminopyridin, 1,2 g Tetrabutylammoniumchlorid und 1,55 g (5,6 mMol) 9-Acridincarbonsäurechloridhydrochlorid. Nach 6 Stunden trennt man die organische Phase ab, wäscht mit Wasser aus, trocknet über Natriumsulfat und dampft ein.

Ausbeute: 2,8 g (98 % d. Th.)
$^1$H-NMR ($CDCl_3$): δ = 1,4 (s); 2,3-2,5 (m); 2,5-2,8 (m); 3,0-3,3 (t); 3,6-3,9 (m); 6,3 (d); 6,9 (d); 7,4-8,3 (m).
MS: $\frac{m}{z}$ = 695 ($M^+$)

(7-a3)

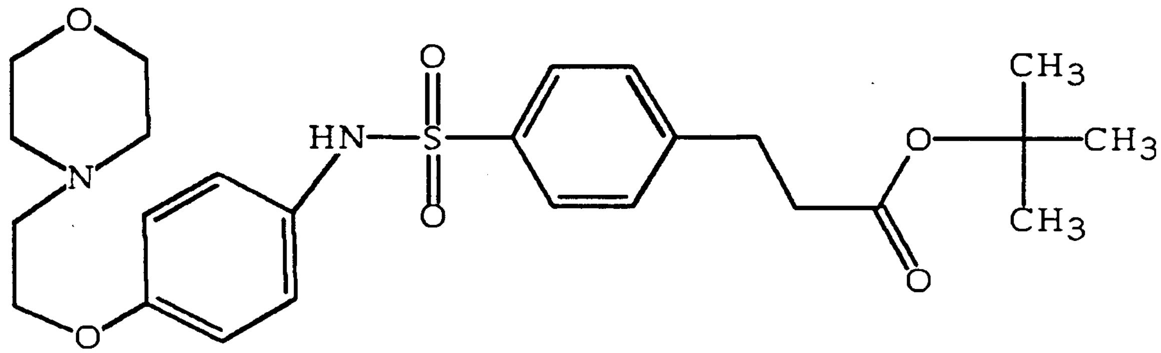

(7-a4)

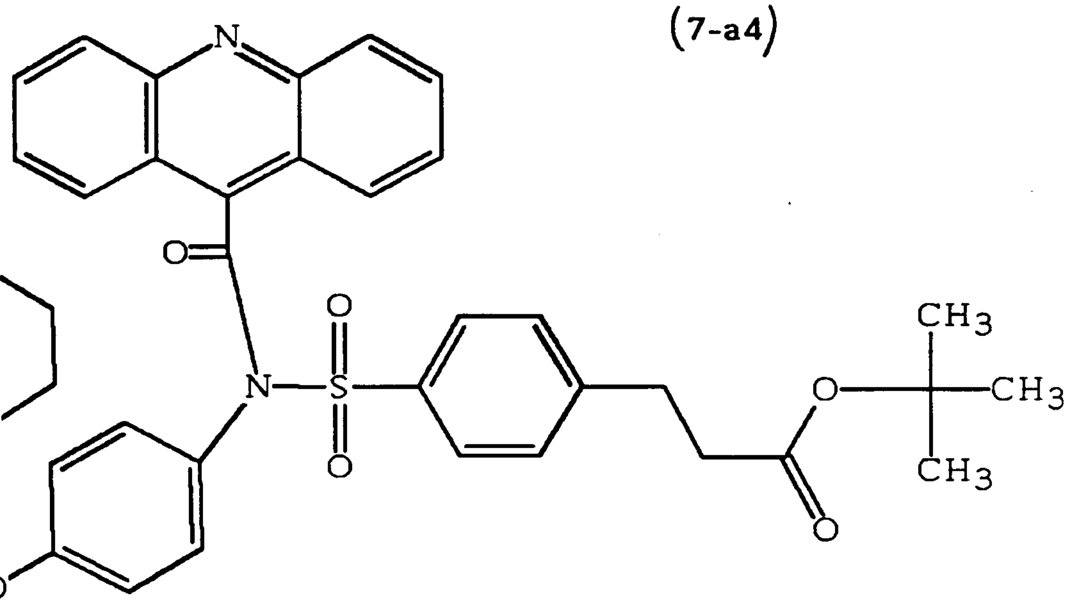

N-[4-(Morpholino-N-2-ethoxy)-phenyl]N-[4-(2-carboxyethyl)phenylsulfonyl]-9-acridincarbonsäureamid (7-a5)

0,7 g (1 mMol) N-[4-(Morpholino-2-ethoxy)-phenyl]-N-[4-(2-t-butoxycarbonylethyl)-phenylsulfonyl]-9-acridincarbonsäureamid werden in 5 ml Trifluoressigsäure gelöst und über Nacht bei Raumtemperatur stehen gelassen. Man dampft an der Wasserstrahlpumpe ein, löst in Wasser und stumpft die filtrierte Lösung mit Natriumacetat auf pH 4 ab. Das angefallene Produkt extrahiert man mit Methylenchlorid, trocknet über Natriumsulfat und dampft ein.

Ausbeute: 0,6 g (94 % d. Th.)
$^1$H-NMR (DMSO): δ = 2,6-3,0 (m); 3,0-3,3 (m); 3,6-4,0 (m); 4,0-4,4 (m); 5,8-6,6 (m); 6,8-7,0,(m); 7,3-8,4 (m).
MS: $\frac{m}{z}$ = 639 ($M^+$)

N-[4-(Morpholino-N-2-ethoxy)-phenyl]-N-[4-(2-succinimidyloxycarbonylethyl)-phenylsulfonyl]-9-acridincarbonsäureamid (7-a6)

Ausbeute: 95 % d. Th.
$^1$H-NMR ($CDCL_3$): δ= 2,3-2,7 (m); 2,8 (s); 2,9-3,4 (m); 3,5-3,9 (m); 3,9-4,3 (m); 6,2-7,0 (m); 7,3-8,3 (m).
MS: 736 ($M^+$)

N-[4-(N-Methylmorpholino-N-2-ethoxy)-phenyl]-N-[4-(2-succinimidyloxycarbonylethyl)-phenyl-sulfonyl]-10-methylacridin-9-carbonsäureamiddiium-di-fluorsulfonat (7-a7)

Ausbeute: 84 % d. Th.
Die Verbindung ist mit gelber Farbe klar wasserslöslich.
$^1$H-NMR (DMSO): δ = 2,85 (s); 3,1 (S) 3,2-4,4 (m); 4,75 (s); 6,4-8,3 (m)

**Beispiel 8**

Tracer-Herstellung für den TSH-Chemilumineszenzimmunoassay

91 µl Antikörper (100 µg), 20 µl des nach Beispiel 1 hergestellten Acridiniumderivats (Verbindung (6)) (1 mg/ml in Acetonitril und 600 µl Konjugationspuffer (0.01 M

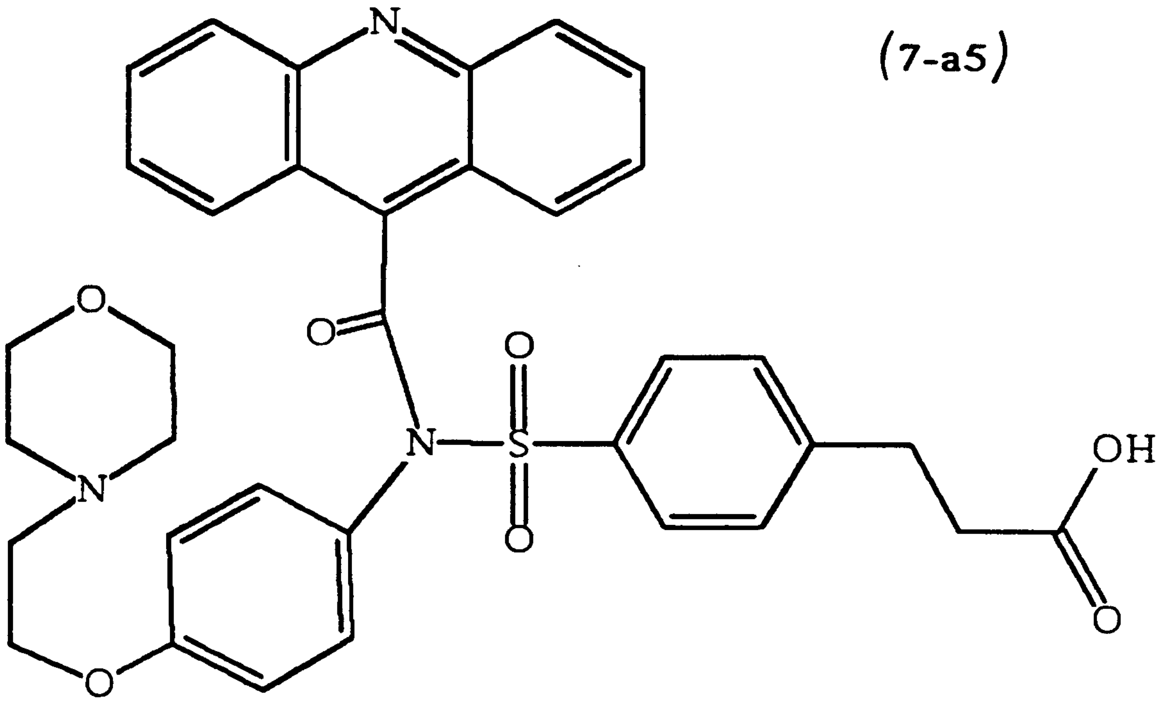

(7-a5)

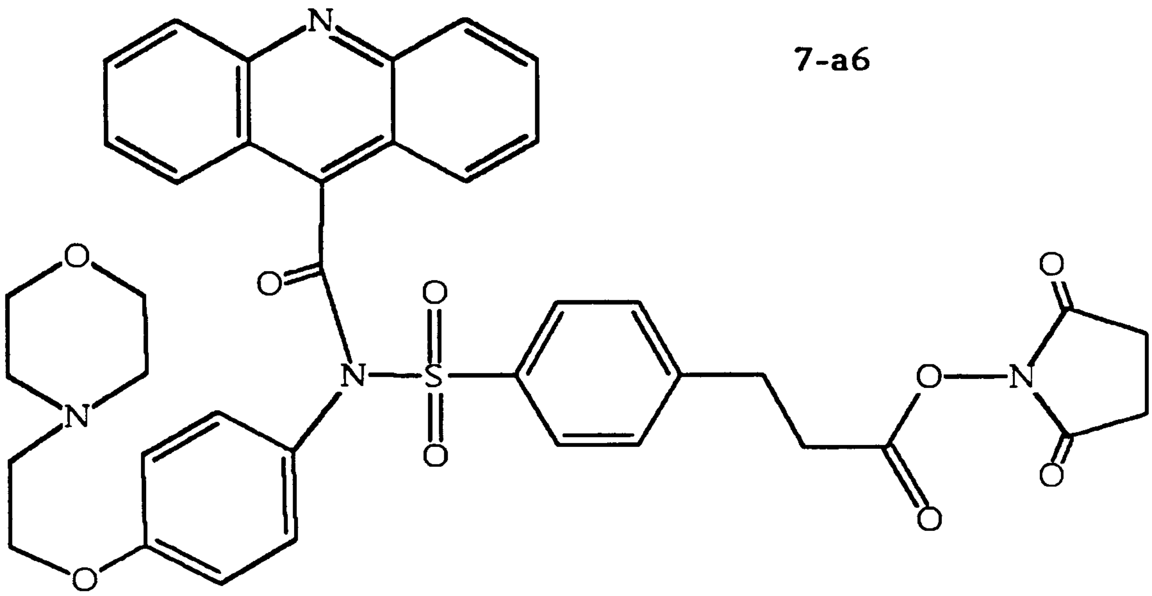

7-a6

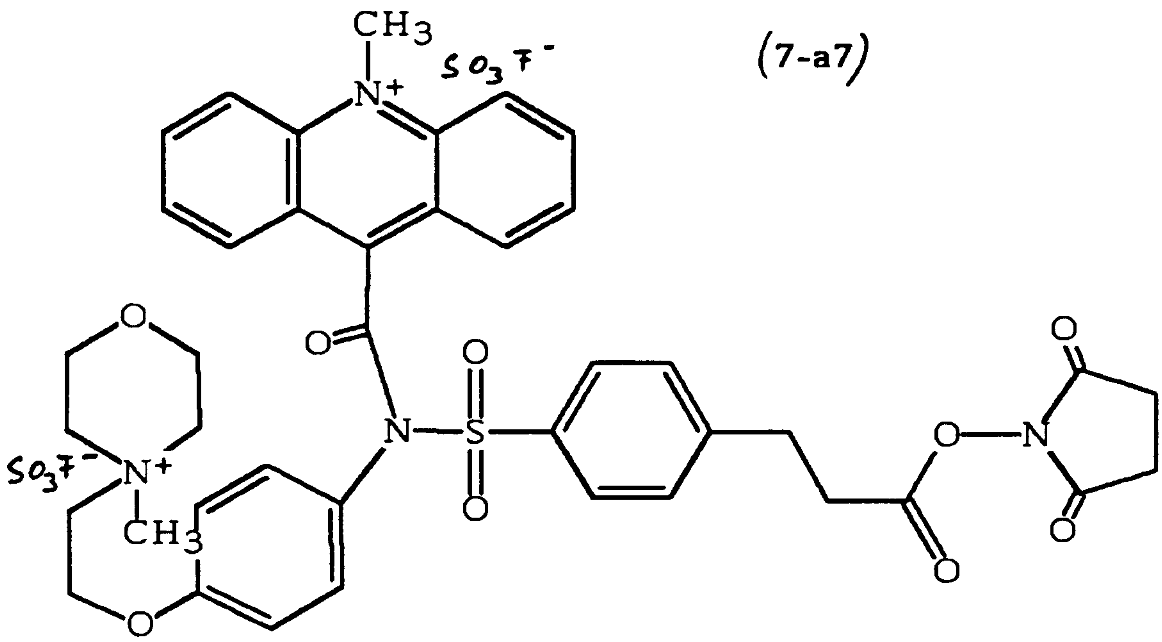

(7-a7)

Phosphat, pH 8,0) werden 15 Minuten inkubiert. Danach werden 200 µl-Lysin (10 mg/ml in Konjugationspuffer) zugefügt und weitere 15 Minuten inkubiert. Dieser Ansatz wird auf eine PD 10-Säule (Sephadex(R) G 25 Medium, (kugelförmiges quervernetzten Dextrangel, Hersteller Fa. Pharmacia, Schweden)), gegeben und mit 0.1 M Phosphat, pH 6.3 als Fließmittel eluiert. 10 Tropfen/Fraktion werden gesammelt. Die einzelnen Fraktionen werden nach geeigneter Verdünnung auf ihre Chemilumineszenaktivität getestet (350 µl Oxidationsmittel: 0.1 % $H_2O_2$ in 0.1 N NaOH). Die Tracerfraktionen (1. Aktivitätspeak) werden "gepoolt" und bei 4°C gelagert. Der gebrauchsfertige Tracer für den h-TSH-Chemilumineszenzimmunoassay wird durch geeignete Verdünnung mit einem Phosphatpuffer (0.1 M Phosphat, pH 6,3, 1 % Tween(R) 20, (Polyethylensorbitanmonolaureat, Hersteller z.B. ICI American Inc., USA), 0.1 % Rinderserumalbumin, 0.1 M NaCl, 0.01 % $NaN_3$) hergestellt.

**Beispiel 9**

Durchführung des h TSH-Chemilumineszenimmunoassays

50 µl Standard/Probe und 200 µl Tracer wurden 2 Stunden bei Raumtemperatur in mit monoklonalen Anti-TSH-Antikörper beschichteten Röhrchen geschüttelt. Danach wird 3x mit 1 ml Puffer bzw. dest. Wasser gewaschen. Die Lichtemission erfolgt durch Zugabe von jeweils 300 µl Aktivierungsreagenz (pH 1-Puffer, 0.5 % $H_2O_2$ und 300 µl Startreagenz (0.2 N NaOH) über 2 Dispensoren im Luminometer in die Röhrchen. Die Meßzeit beträgt 1 sec.

Figur 3 zeigt den typischen Verlauf einer Standardkurve eines Immuno-chemiluminometrischen Assays (ICMA) für das humane Thyreoidea-stimulierende Hormon (h-TSH).

**Patentansprüche**

**Patentansprüche für folgende Vertragsstaaten : AT, BE, CH, DE, DK, FR, GB, IE, IT, LI, LU, MC, NL, PT, SE**

**1.** Chemilumineszierende Acridiniumderivate der Formel I

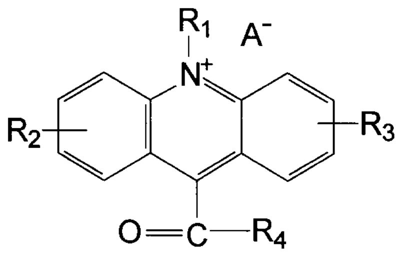

(I)

in der bedeuten:

$R_1$: Wasserstoff
ein Alkyl-, Alkenyl- oder Alkinylrest mit bis zu 10 Kohlenstoffatomen
eine Benzyl- oder Arylgruppe;

$R_2$ und $R_3$: Wasserstoff
eine Alkylgruppe mit 1 bis 4 Kohlenstoffatomen
eine substituierte oder unsubstituierte Aminogruppe
eine Carboxy-, Alkoxy-, Cyano- oder Nitrogruppe oder Halogen;

$R_4$ einen Rest der Formel II oder III:

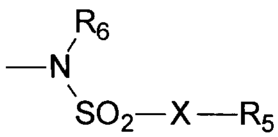

(II)

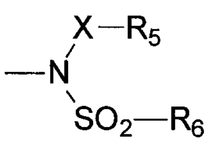

(III)

$R_5$: eine reaktive Gruppe,
die unter schonenden Bedingungen selektiv mit Amino-, Carboxy-, Thiol- oder anderen funktionellen Gruppen in Substanzen von biologischem Interesse eine Bindung eingehen kann;

$R_6$: eine Alkylgruppe mit 1 bis 4 Kohlenstoffatomen oder
eine Phenylgruppe, die mit bis zu 3 Alkyl- oder Alkoxygruppen mit jeweils 1 bis 4 Kohlenstoffatomen mit einer $-(-O-CH_2-CH_2-)_n-OR$ Gruppe, wobei
n die Bedeutung 0 bis 8 hat und
R eine Morpholinoethyl- oder eine N,N-Dimethylaminoethyl- oder eine Alkygruppe mit 1 bis 4 Kohlenstoffatomen ist, oder mit einer Ethylendioxygruppe substituiert sein kann; und

X: eine Arylengruppe, die
direkt oder über eine Alkylen- oder eine Oxyalkylengruppe an das Stickstoff- oder Schwefelatom gebunden ist,
und
über eine Alkylen- oder Oxyalkylengruppe an den Rest $R_5$ gebunden ist, und
die auch mit
Alkyl-, Alkenyl-, Hydroxy-, Amino-, Alkoxy- oder Aryloxygruppen und/oder
Heteroatomen einfach oder mehrfach substituiert sein kann oder
den Rest einer aromatischen natürlichen Aminosäure bedeutet oder
eine Phenylengruppe ist - wenn $R_6$ eine Phenylgruppe ist - die einfach oder mehrfach mit Alkygruppen mit 1 bis 6 Kohlenstoffatomen substituiert ist und

$A^-$: ein die Chemolumineszenz nicht beeinträchtigendes Anion ist.

**2.** Acridiniumderivate nach Anspruch 1, dadurch gekennzeichnet, daß X eine Gruppe der Formel IV ist

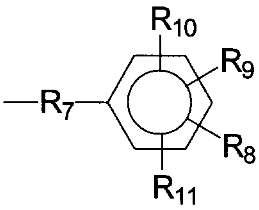

(IV)

in der

$R_7$ ein Substituent der Formel $-(CH_2)n-$ oder $-((CH_2)m-O-)n-$ ist, mit n=0 bis 4 und m=1 bis 6;

$R_8$ ein Substituent der Formel $-(CH_2)p-$, $-(O-(CH_2)m-)p$ oder $-((CH_2)m-O)p$ mit p= 1 bis 6 und m= 1 bis 6, in ortho-, meta- oder para-Stellung zu $R_7$ oder
ein verzweigter oder unverzweigter Kohlenwasserstoffrest mit 1 bis 4 Kohlenstoffatomen ist; und

$R_9$ bis $R_{11}$ Wasserstoff, geradkettige oder verzweigte Kohlenwasserstoffreste mit bis zu 30 Kohlenstoffatomen sind,
wobei eine oder mehrere $-CH_2-$ Einheiten durch O, S, SO, NH oder N-Alkyl ersetzt sein können und zwei dieser Substituenten ringförförmig miteinander verknüpft sein können.

**3.** Acridiniumderivate nach Anspruch 1 oder 2, dadurch gekennzeichnet, daß $R_5$ eine Gruppe der Formel V ist:

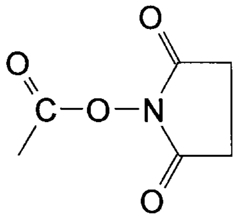

(V)

4. Acridiniumderivate nach mindestens einem der Ansprüche 1 bis 3, gekennzeichnet durch die Formel VI

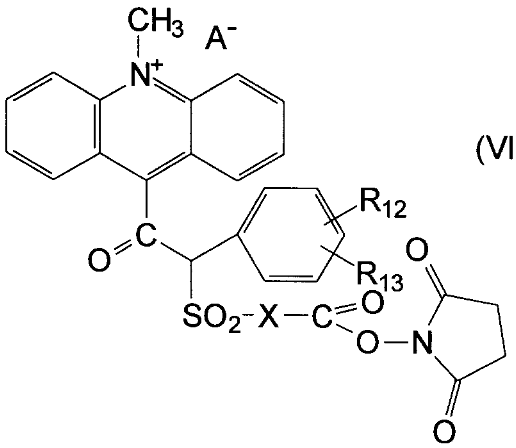

(VI)

in der X eine Gruppe der Formel VII ist

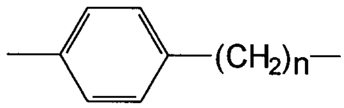

(VII)

mit n= 2 oder 4 und
$R_{12}$ und $R_{13}$ unabhängig voneinander
Wasserstoff, eine Alkylgruppe, eine Alkoxygruppe mit 1 bis 4 Kohlenstoffatomen,
eine $-(O-CH_2-CH_2-)_n-OR$ Gruppe, wobei
n die Bedeutung 0 bis 8 hat und
R eine Morpholinoethyl- oder eine N,N-Dimethylaminoethyl- oder eine Alkylgruppe mit 1 bis 4 Kohlenstoffatomen ist,
oder zusammen eine Ethylendioxygruppe sind; und

$A^-$ die in Anspruch 1 genannte Bedeutung hat.

5. Acridiniumderivat nach Anspruch 4, dadurch gekennzeichnet, daß

R eine Morpholinoethyl- oder eine N,N-Dimethylaminoethylgruppe ist,
sowie die quartären Ammoniumverbindungen.

**6.** Acridiniumderivat nach Anspruch 4, dadurch gekennzeichnet, daß

X eine p-Ethylenphenylgruppe und
$R_{12}$ ein Wasserstoffatom und $R_{13}$ eine p-Methoxygruppe, oder
$R_{12}$ eine o-Methoxygruppe und $R_{13}$ eine p-Methoxygruppe, oder
$R_{12}$ und $R_{13}$ zusammen eine 3,4-Ethylendioxygruppe sind.

**7.** Acridiniumderivat nach mindestens einem der Ansprüche 1 bis 2, gekennzeichnet durch die Formel VIII

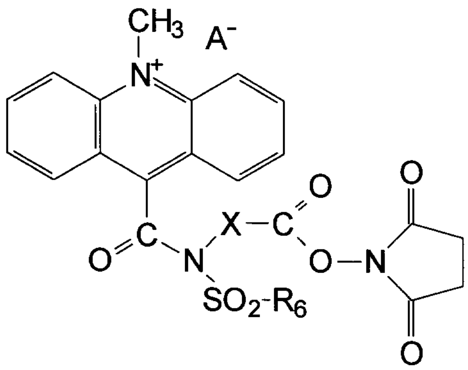

(VIII)

in der X die in Anspruch 1 oder 2 genannte Bedeutung hat und

$R_6$ eine Alkylgruppe mit 1 bis 4 Kohlenstoffatomen oder eine Phenylgruppe, die
mit bis zu 3 Alkyl- oder Alkoxygruppen mit jeweils 1 bis 4 Kohlenstoffatomen,
mit einer $-(-O-CH_2-CH_2-)_n-OR$ Gruppe, wobei
n die Bedeutung 0 bis 8 hat und
R eine Morpholinoethyl- oder eine N,N-Dimethylaminoethyl- oder eine Alkygruppe mit 1 bis 4 Kohlenstoffatomen ist, oder
mit einer Ethylendioxygruppe substituiert sein kann; oder
in der X eine o-,m- oder p-Phenylengruppe ist und
$R_6$ eine Phenylgruppe, die mit bis zu drei Alkylgruppen mit jeweils 1 bis 4 Kohlenstoffatomen substituiert sein kann.

**8.** Acridiniumderivat nach Anspruch 7, dadurch gekennzeichnet, daß

R eine Morpholinoethyl- oder eine N,N-Dimethylaminoethylgruppe ist,
sowie die quartären Ammoniumverbindungen.

**9.** Verfahren zur Herstellung von Verbindungen gemäß einem der Ansprüche 1-8 dadurch gekennzeichnet, daß man eine
Verbindung der Formel IX

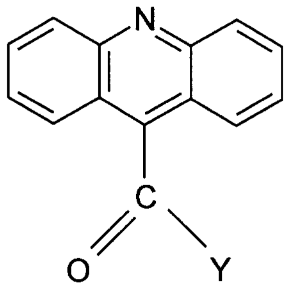

(IX)

mit einer geschützten Sulfonamidcarbonsäure der Formel X

$$R_6 - SO_2\ \overset{H}{\overset{|}{N}} - X - \overset{O}{\overset{\|}{C}} - \quad OZ \qquad (X)$$

oder der Formel XI

$$R_6 - \overset{H}{\overset{|}{N}} - SO_2 - X - COOZ \qquad (XI)$$

umsetzt, wobei Y= Halogen, eine Oxycarbonylalkyl-, Oxycarbonylaryl- oder Imidazolidgruppe ist, X und $R_6$ die in Anspruch 1 genannten Bedeutungen haben und Z ein die Carboxygruppe schützender Rest ist, der anschließend abgespalten wird, und die dabei entstehende Säure in das den Rest $R_5$ enthaltende gewünschte Acridiniumderivat umgesetzt wird, das dann noch am Stickstoff des Acridingerüstes quaternisiert wird.

**10.** Lumineszente Verbindung, dadurch gekennzeichnet, daß ein Acridiniumderivat gemäß einem der Ansprüche 1-9 direkt oder über ein Brückenmolekül an ein Protein, ein Polypeptid oder an eine andere Substanz von biologischem Interesse unter Ausbildung eines stabilen, immunologisch aktiven Konjugates gebunden ist.

**11.** Lumineszenzimmunoassay zur Bestimmung einer antigenen Substanz in einer Flüssigkeitsprobe, bei dem in einem kompetitiven- oder einem Sandwich-Verfahren mindestens eine immunologisch aktive Komponente auf einer festen Phase immobilisiert ist und mindestens eine weitere Komponente, der Tracer, eine lumineszente Verbindung entsprechend Anspruch 10 darstellt.

**12.** Lumineszenzimmunoassay nach Anspruch 11, dadurch gekennzeichnet, daß man

a) einen mit einem Antigen spezifisch reagierenden, immobilisierten Antikörper mit einer Probe der zu untersuchenden Flüssigkeit und einem Konjugat aus dem Antigen und einem chemilumineszierenden Acridiniumderivat nach Anspruch 1 inkubiert;

b) die Probe und das nichtgebundene, markierte Konjugat abtrennt;

c) das gebundene, markierte Konjugat sukzessive oder gleichzeitig mit einem oder mehreren Reagenzien zusammenbringt, um eine Lichtemission hervorzurufen
und

d) aus der gemessenen Stärke der Lichtemission die Menge des vorhandenen Antigens bestimmt.

**13.** Lumineszenzimmunoassay nach Anspruch 12, dadurch gekennzeichnet, daß vor der Zugabe des markierten Konjugates die zu untersuchende Flüssigkeit von immobilisiertem Antikörper abgetrennt wird.

**14.** Lumineszenzimmunoassay nach Anspruch 11, dadurch gekennzeichnet, daß man

a) einen mit dem Antigen spezifisch reagierenden, immobilisierten Antikörper mit einer Probe der zu untersuchenden Flüssigkeit und einem Konjugat aus einem zweiten, spezifisch reagierenden Antikörper und einem chemilumineszierenden Acridiniumderivat nach Anspruch 1 inkubiert;
b) die Probe und das nichtgebundene, markierte Konjugat abtrennt;
c) das gebundene, markierte Konjugat sukzessive oder gleichzeitig mit einem oder mehreren Reagenzien zusammenbringt, um eine Lichtemission hervorzurufen und
d) aus der gemessenen Stärke der Lichtemission die Menge des vorhandenen Antigens bestimmt.

**15.** Lumineszenzimmunoassay nach Anspruch 14, dadurch gekennzeichnet, daß vor der Zugabe des markierten Konjugates die zu untersuchende Flüssigkeit vom immobilisierten Antikörper abgetrennt wird.

**16.** Lumineszenzimmunoassay nach Anspruch 11, dadurch gekennzeichnet, daß man

a) ein mit dem Antikörper spezifisch reagierendes, immobilisiertes Antigen mit einer Probe der zu untersuchenden Flüssigkeit und einer Lösung eines Konjugats aus dem Antikörper und einem chemilumineszierenden Acridiniumderivat nach Anspruch 1 inkubiert;
b) die Probe und das nichtgebundene, markierte Konjugat abtrennt;
c) das gebundene, markierte Konjugat sukzessive oder gleichzeitig mit einem oder mehreren Reagenzien zusammenbringt, um eine Lichtemission hervorzurufen
und
d) aus der gemessenen Stärke der Lichtemission die Menge des vorhandenen Antigens bestimmt.

**17.** Lumineszenzimmunoassay nach Anspruch 11, dadurch gekennzeichnet, daß man

a) ein mit dem Antikörper spezifisch reagierendes, immobilisiertes Antigen mit einer Lösung eines Konjugats aus dem Antikörper und einem chemilumineszierenden Acridiniumderivat nach Anspruch 1 inkubiert;
b) das nicht umgesetzte, markierte Konjugat abtrennt,
c) eine Probe der zu untersuchenden Flüssigkeit zufügt,
d) die Probe anschließend wieder abtrennt,
e) das gebundene, markierte Konjugat sukzessive oder gleichzeitig mit einem oder mehreren Reagenzien zusammenbringt, um eine Lichtemission hervorzurufen
und
f) aus der gemessenen Stärke der Lichtemission die Menge des vorhandenen Antigens bestimmt.

**18.** Lumineszenzimmunoassay nach Anspruch 11, dadurch gekennzeichnet, daß man

a) ein mit dem Antikörper spezifisch reagierendes, immobilisiertes Antigen mit einer Lösung eines Konjugats aus dem Antikörper und einem chemilumineszierenden Acridiniumderivat nach Anspruch 1 inkubiert
b) eine Probe der zu untersuchenden Flüssigkeit zufügt,
c) die Probe und das nichtgebundene Konjugat abtrennt;
d) das gebundene, markierte Konjugat sukzessive oder gleichzeitig mit einem oder mehreren Reagenzien zusammenbringt, um eine Lichtemission hervorzurufen
und
e) aus der gemessenen Stärke der Lichtemission die Menge des vorhandenen Antigens bestimmt.

**Patentansprüche für folgende Vertragsstaaten : ES, GR**

**1.** Verfahren zur Herstellung chemilumineszierender Acridiniumderivate der Formel I

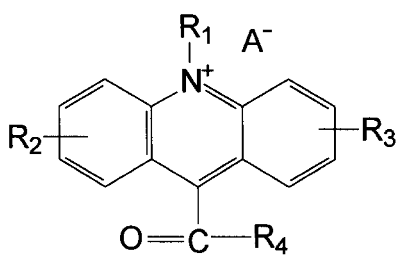

(I)

in der bedeuten:

$R_1$: Wasserstoff
ein Alkyl-, Alkenyl- oder Alkinylrest mit bis zu 10 Kohlenstoffatomen
eine Benzyl- oder Arylgruppe;

$R_2$ und $R_3$: Wasserstoff
eine Alkylgruppe mit 1 bis 4 Kohlenstoffatomen
eine substituierte oder unsubstituierte Aminogruppe

eine Carboxy-, Alkoxy-, Cyano- oder Nitrogruppe oder Halogen;

$R_4$ einen Rest der Formel II oder III:

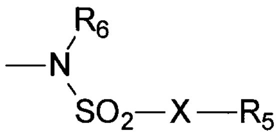

(II)

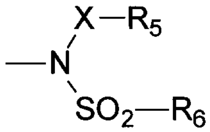

(III)

$R_5$: eine reaktive Gruppe, die unter schonenden Bedingungen selektiv mit Amino-, Carboxy-, Thiol- oder anderen funktionellen Gruppen in Substanzen von biologischem Interesse eine Bindung eingehen kann

$R_6$: eine Alkylgruppe mit 1 bis 4 Kohlenstoffatomen oder eine Phenylgruppe, die mit bis zu 3 Alkyl- oder Alkoxygruppen mit jeweils 1 bis 4 Kohlenstoffatomen mit einer $-(-O-CH_2-CH_2-)_n-OR$ Gruppe, wobei

n die Bedeutung 0 bis 8 hat und

R eine Morpholinoethyl- oder eine N,N-Dimethylaminoethyl- oder eine Alkygruppe mit 1 bis 4 Kohlenstoffatomen ist, oder mit einer Ethylendioxygruppe substituiert sein kann; und

X: eine Arylengruppe, die

direkt oder über eine Alkylen- oder eine Oxyalkylengruppe an das Stickstoff- oder Schwefelatom gebunden ist,

und

über eine Alkylen- oder Oxyalkylengruppe an den Rest $R_5$ gebunden ist, und

die auch mit

Alkyl-, Alkenyl-, Hydroxy-, Amino-, Alkoxy- oder Aryloxygruppen und/oder

Heteroatomen einfach oder mehrfach substituiert sein kann oder

den Rest einer aromatischen natürlichen Aminosäure bedeutet oder

eine Phenylengruppe ist - wenn $R_6$ eine Phenylgruppe ist - die einfach oder mehrfach mit Alkygruppen mit 1 bis 6 Kohlenstoffatomen substituiert ist und

$A^-$: ein die Chemolumineszenz nicht beeinträchtigendes Anion ist,

dadurch gekennzeichnet, daß man eine Verbindung der Formel IX

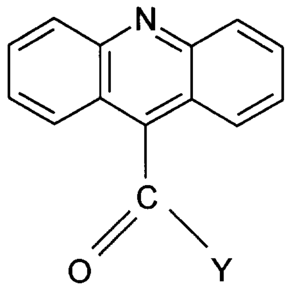

(IX)

mit einer geschützten Sulfonamidcarbonsäure der Formel X

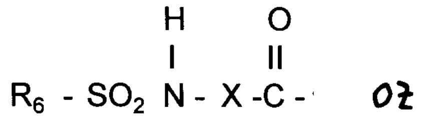

(X)

oder der Formel XI

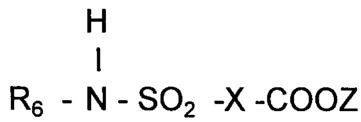

(XI)

umsetzt, wobei Y= Halogen, eine Oxycarbonylalkyl-, Oxycarbonylaryl- oder Imidazolidgruppe ist, X und $R_6$ die oben genannten Bedeutungen haben und Z ein die Carboxygruppe schützender Rest ist, der anschließend abgespalten wird, und die dabei entstehende Säure in das den Rest $R_5$ enthaltende gewünschte Acridiniumderivat umgesetzt wird, das dann noch am Stickstoff des Acridingerüstes quaternisiert wird.

**2.** Verfahren zur Herstellung einer lumineszenten Verbindung, dadurch gekennzeichnet, daß ein nach Anspruch 1 hergestelltes Acridiniumderivat direkt oder über ein Brückenmolekül an ein Protein, ein Polypeptid oder an eine andere Substanz von biologischem Interesse unter Ausbildung eines stabilen, immunologisch aktiven Konjugates gebunden wird.

**3.** Lumineszenzimmunoassay zur Bestimmung einer antigenen Substanz in einer Flüssigkeitsprobe, bei dem in einem kompetitiven- oder einem Sandwich-Verfahren mindestens eine immunologisch aktive Komponente auf einer festen Phase immobilisiert ist und mindestens eine weitere Komponente, der Tracer, eine lumineszente Verbindung, die nach dem Verfahren gemäß Anspruch 2 hergestellt worden ist.

**4.** Lumineszenzimmunoassay nach Anspruch 3, dadurch gekennzeichnet, daß man

a) einen mit einem Antigen spezifisch reagierenden, immobilisierten Antikörper mit einer Probe der zu untersuchenden Flüssigkeit und einem Konjugat aus dem Antigen und einem nach Anspruch 1 hergestellten chemilumineszierenden Acridiniumderivat inkubiert;

b) die Probe und das nichtgebundene, markierte Konjugat abtrennt;

c) das gebundene, markierte Konjugat sukzessive oder gleichzeitig mit einem oder mehreren Reagenzien zusammenbringt, um eine Lichtemission hervorzurufen
und

d) aus der gemessenen Stärke der Lichtemission die Menge des vorhandenen Antigens bestimmt.

**5.** Lumineszenzimmunoassay nach Anspruch 4, dadurch gekennzeichnet, daß vor der Zugabe des markierten Konjugates die zu untersuchende Flüssigkeit von immobilisiertem Antikörper abgetrennt wird.

**6.** Lumineszenzimmunoassay nach Anspruch 3, dadurch gekennzeichnet, daß man

a) einen mit dem Antigen spezifisch reagierenden, immobilisierten Antikörper mit einer Probe der zu untersuchenden Flüssigkeit und einem Konjugat aus einem zweiten, spezifisch reagierenden Antikörper und einem nach Anspruch 1 hergestellten chemilumineszierenden Acridiniumderivat inkubiert;
b) die Probe und das nichtgebundene, markierte Konjugat abtrennt;
c) das gebundene, markierte Konjugat sukzessive oder gleichzeitig mit einem oder mehreren Reagenzienzusammenbringt, um eine Lichtemission hervorzurufen
und
d) aus der gemessenen Stärke der Lichtemission die Menge des vorhandenen Antigens bestimmt.

**7.** Lumineszenzimmunoassay nach Anspruch 6, dadurch gekennzeichnet, daß vor der Zugabe des markierten Konjugates die zu untersuchende Flüssigkeit vom immobilisierten Antikörper abgetrennt wird.

**8.** Lumineszenzimmunoassay nach Anspruch 4, dadurch gekennzeichnet, daß man

a) ein mit dem Antikörper spezifisch reagierendes, immobilisiertes Antigen mit einer Probe der zu untersuchenden Flüssigkeit und einer Lösung eines Konjugats aus dem Antikörper und einem nach Anspruch 1 hergestellten chemilumineszierenden Acridiniumderivat inkubiert;
b) die Probe und das nichtgebundene, markierte Konjugat abtrennt;
c) das gebundene, markierte Konjugat sukzessive oder gleichzeitig mit einem oder mehreren Reagenzien zusammenbringt, um eine Lichtemission hervorzurufen
und
d) aus der gemessenen Stärke der Lichtemission die Menge des vorhandenen Antigens bestimmt.

**9.** Lumineszenzimmunoassay nach Anspruch 3, dadurch gekennzeichnet, daß man

a) ein mit dem Antikörper spezifisch reagierendes, immobilisiertes Antigen mit einer Lösung eines Konjugats aus dem Antikörper und einem nach Anspruch 1 hergestellten chemilumineszierenden Acridiniumderivat inkubiert;
b) das nicht umgesetzte, markierte Konjugat abtrennt,
c) eine Probe der zu untersuchenden Flüssigkeit zufügt,
d) die Probe anschließend wieder abtrennt
e) das gebundene, markierte Konjugat sukzessive oder gleichzeitig mit einem oder mehreren Reagenzien zusammenbringt, um eine Lichtemission hervorzurufen
und
f) aus der gemessenen Stärke der Lichtemission die Menge des vorhandenen Antigens bestimmt.

10. Lumineszenzimmunoassay nach Anspruch 3, dadurch gekennzeichnet, daß man

a) ein mit dem Antikörper spezifisch reagierendes, immobilisiertes Antigen mit einer Lösung eines Konjugats aus dem Antikörper und einem nach Anspruch 1 hergestellten chemilumineszierenden Acridiniumderivat inkubiert;
b) eine Probe der zu untersuchenden Flüssigkeit zufügt,
c) die Probe und das nichtgebundene Konjugat abtrennt;
d) das gebundene, markierte Konjugat sukzessive oder gleichzeitig mit einem oder mehreren Reagenzien zusammenbringt, um eine Lichtemission hervorzurufen
und
e) aus der gemessenen Stärke der Lichtemission die Menge des vorhandenen Antigens bestimmt.

**Claims**

**Claims for the following Contracting States : AT, BE, CH, DE, DK, FR, GB, IE, IT, LI, LU, MC, NL, PT, SE**

**1.** A chemiluminescent acridinium derivative of the formula I

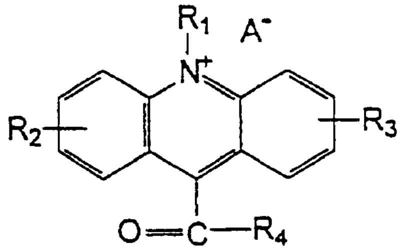

(I)

in which:

$R_1$ is hydrogen
an alkyl, alkenyl or alkynyl radical having up to 10 carbon atoms
a benzyl or aryl group;

$R_2$ and $R_3$ are hydrogen
an alkyl group having 1 to 4 carbon atoms
a substituted or unsubstituted amino group
a carboxyl, alkoxy, cyano or nitro group or halogen;

$R_4$ is a radical of the formula II or III:

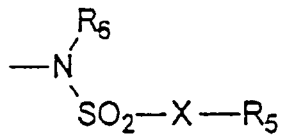

(II)

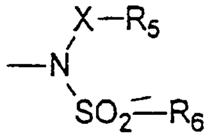

(III)

$R_5$ is a reactive group which is able to undergo bonding under mild conditions selectively with amino, carboxyl, thiol or other functional groups in substances of biological interest;

$R_6$ is an alkyl group having 1 to 4 carbon atoms or a phenyl group which can be substituted by up to 3 alkyl or alkoxy groups each having 1 to 4 carbon atoms,
by an $-(-O-CH_2-CH_2-)_n-OR$ group, where
n has the meaning 0 to 8 and
R is a morpholinoethyl or an N,N-dimethylaminoethyl or an alkyl group having 1 to 4 carbon atoms, or
by an ethylenedioxy group; and

X is an arylene group which is bonded to the nitrogen or sulfur atom directly or via an alkylene or an oxyalkylene group and is bonded to the radical $R_5$ via an alkylene or oxyalkylene group, and
which can also be substituted one or more times by alkyl, alkenyl, hydroxyl, amino, alkoxy or aryloxy groups and/or hetero atoms or
is the radical of an aromatic natural amino acid or
is a phenylene group - when $R_6$ is a phenyl group - which is substituted one or more times by alkyl groups having 1 to 6 carbon atoms and

A⁻ is an anion which does not adversely affect the chemiluminescence,

or of the formula

**2.** An acridinium derivative as claimed in claim 1, wherein X is a group of the formula IV

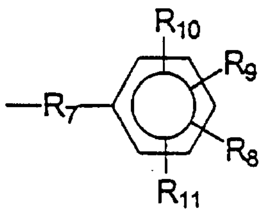

(IV)

in which

$R_7$ is a substituent of the formula $-(CH_2)_n-$ or $-((CH_2)_m-O-)_n$, with n = 0 to 4 and m = 1 to 6;

$R_8$ is a substituent of the formula $-(CH_2)_p-$, $-(O-(CH_2)_m-)_p$ or $-((CH_2)_m-O)_p$, with p = 1 to 6 and m = 1 to 6, in ortho, meta or para position to $R_7$ or
a branched or unbranched hydrocarbon radical having 1 to 4 carbon atoms; and

$R_9$ to $R_{11}$ are hydrogen, straight-chain or branched hydrocarbon radicals having up to 30 carbon atoms, it being possible for one or more $-CH_2-$units to be replaced by O, S, SO, NH or N-alkyl and for two of these substituents to be linked to form a ring.

**3.** An acridinium derivative as claimed in claim 1 or 2, wherein $R_5$ is a group of the formula V:

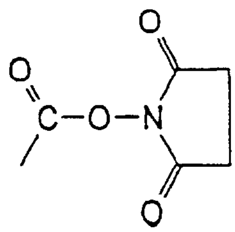

(V)

**4.** An acridinium derivative as claimed in at least one of claims 1 to 3, which has the formula VI

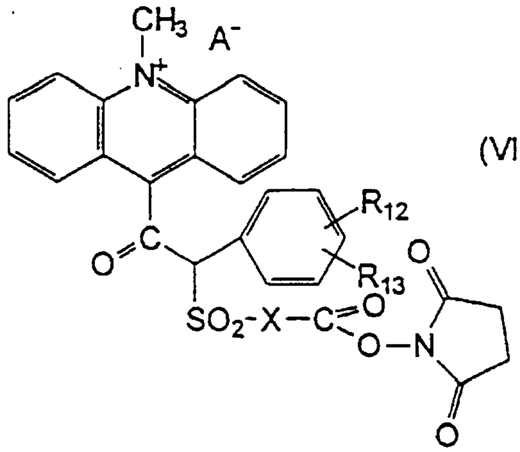

(VI)

in which X is a group of the formula VII

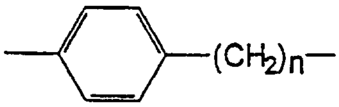

(VII)

with n = 2 or 4 and
$R_{12}$ and $R_{13}$ are, independently of one another, hydrogen, an alkyl group, an alkoxy group having 1 to 4 carbon atoms,
an $-(-O-CH_2-CH_2)_n-OR$ group, where
n has the meaning 0 to 8, and
R is a morpholinoethyl or an N,N-dimethylaminoethyl or an alkyl group having 1 to 4 carbon atoms,
or are together an ethylenedioxy group; and

$A^-$ has the meaning mentioned in claim 1.

**5.** An acridinium derivative as claimed in claim 4, wherein

R is a morpholinoethyl or an N,N-dimethylaminoethyl group, and the quaternary ammonium compounds.

**6.** An acridinium derivative as claimed in claim 4, wherein

X is a p-ethylenephenyl group and
$R_{12}$ is a hydrogen atom and $R_{13}$ is a p-methoxy group, or
$R_{12}$ is an o-methoxy group and $R_{13}$ is a p-methoxy group, or
$R_{12}$ and $R_{13}$ are together a 3,4-ethylenedioxy group.

**7.** An acridinium derivative as claimed in at least one of claims 1 to 2, which has the formula VIII

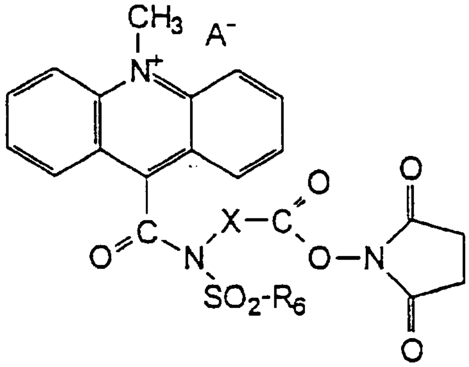

(VIII)

in which X has the meaning mentioned in claim 1 or 2, and

$R_6$ is an alkyl group having 1 to 4 carbon atoms or a phenyl group which can be substituted by up to 3 alkyl or alkoxy groups each having 1 to 4 carbon atoms,
by an $-(-O-CH_2-CH_2-)_n-OR$ group, where
n has the meaning 0 to 8 and
R is a morpholinoethyl or an N,N-dimethylaminoethyl or an alkyl group having 1 to 4 carbon atoms, or
by an ethylenedioxy group; or
in which X is an o-, m- or p-phenylene group, and

$R_6$ is a phenyl group which can be substituted by up to three alkyl groups each having 1 to 4 carbon atoms.

**8.** An acridinium derivative as claimed in claim 7, wherein

R is a morpholinoethyl or an N,N-dimethylaminoethyl group,
and the quaternary ammonium compounds.

**9.** A process for the preparation of compounds as claimed in one of claims 1-8, which comprises reacting a compound of the formula IX

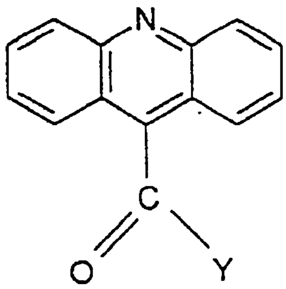

(IX)

with a protected sulfonamide carboxylic acid of the formula X

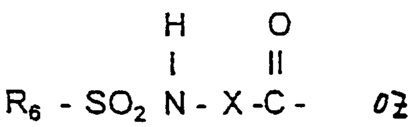

(X)

or of the formula XI

$$R_6 - \overset{\displaystyle H}{\overset{|}{N}} - SO_2 - X - COOZ \qquad (XI)$$

where Y is halogen, an oxycarbonylalkyl, oxycarbonylaryl or imidazolide group, X and $R_6$ have the meanings mentioned in claim 1, and Z is a radical which protects the carboxyl group and is subsequently eliminated, and converting the acid resulting from this into the desired acridinium derivative which contains the radical $R_5$ and is then additionally quaternized on the nitrogen of the acridine skeleton.

**10.** A luminescent compound wherein an acridinium derivative as claimed in one of claims 1-9 is bonded directly or via a bridging molecule to a protein, a polypeptide or to another substance of biological interest with the formation of a stable immunologically active conjugate.

**11.** A luminescence immunoassay for the determination of an antigenic substance in a liquid sample, in which, in a competitive or sandwich method, at least one immunologically active component is immobilized on a solid phase, and at least one other component, the tracer, represents a luminescent compound as claimed in claim 10.

**12.** A luminescence immunoassay as claimed in claim 11, wherein

a) an immobilized antibody which reacts specifically with an antigen is incubated with a sample of the liquid which is to be investigated and with a conjugate composed of the antigen and of a chemiluminescent acridinium derivative as claimed in claim 1;

b) the sample and the unbound conjugate with marker are separated off;

c) the bound conjugate with marker is contacted successively or simultaneously with one or more reagents in order to bring about light emission and

d) the amount of antigen present is determined from the measured intensity of light emission.

**13.** A luminescence immunoassay as claimed in claim 12, wherein the liquid which is to be investigated is separated from the immobilized antibody before the addition of the conjugate with marker.

**14.** A luminescence immunoassay as claimed in claim 11, wherein

a) an immobilized antibody which reacts specifically with the antigen is incubated with a sample of the liquid which is to be investigated and with a conjugate composed of a second specifically reacting antibody and of a chemiluminescent acridinium derivative as claimed in claim 1;
b) the sample and the unbound conjugate with marker are separated off;
c) the bound conjugate with marker is contacted successively or simultaneously with one or more reagents in order to bring about light emission and
d) the amount of antigen present is determined from the measured intensity of light emission.

**15.** A luminescence immunoassay as claimed in claim 14, wherein the liquid which is to be investigated is separated from the immobilized antibody before the addition of the conjugate with marker.

**16.** A luminescence immunoassay as claimed in claim 11, wherein

a) an immobilized antigen which reacts specifically with the antibody is incubated with a sample of the liquid which is to be investigated and with a solution of a conjugate composed of the antibody and of a chemiluminescent acridinium derivative as claimed in claim 1;
b) the sample and the unbound conjugate with marker are separated off;
c) the bound conjugate with marker is contacted successively or simultaneously with one or more reagents in order to bring about light emission and

d) the amount of antigen present is determined from the measured intensity of light emission.

**17.** A luminescence immunoassay as claimed in claim 11, wherein

a) an immobilized antigen which reacts specifically with the antibody is incubated with a solution of a conjugate composed of the antibody and of a chemiluminescent acridinium derivative as claimed in claim 1;
b) the unreacted conjugate with marker is separated off;
c) a sample of the liquid which is to be investigated is added,
d) the sample is subsequently separated off again,
e) the bound conjugate with marker is contacted successively or simultaneously with one or more reagents in order to bring about light emission and
f) the amount of antigen present is determined from the measured intensity of light emission.

**18.** A luminescence immunoassay as claimed in claim 11, wherein

a) an immobilized antigen which reacts specifically with the antibody is incubated with a solution of a conjugate composed of the antibody and of a chemiluminescent acridinium derivative as claimed in claim 1;
b) a sample of the liquid is to be investigated is added;
c) the sample and the unbound conjugate are separated off;
d) the bound conjugate with marker is contacted successively or simultaneously with one or more reagents in order to bring about light emission and
e) the amount of antigen present is determined from the measured intensity of light emission.

**Claims for the following Contracting States : ES, GR**

**1.** A process for the preparation of chemiluminescent acridinium derivatives of the formula I

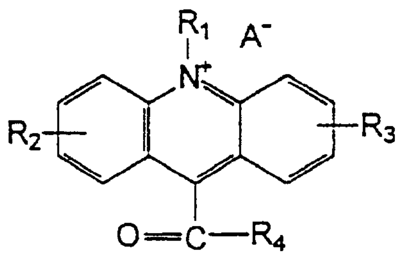

(I)

in which:

| | |
|---|---|
| $R_1$ | is hydrogen<br>an alkyl, alkenyl or alkynyl radical having up to 10 carbon atoms<br>a benzyl or aryl group; |
| $R_2$ and $R_3$ | are hydrogen<br>an alkyl group having 1 to 4 carbon atoms<br>a substituted or unsubstituted amino group<br>a carboxyl, alkoxy, cyano or nitro group or halogen; |
| $R_4$ | is a radical of the formula II or III: |

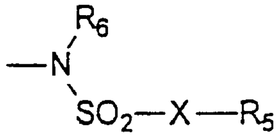

(II)

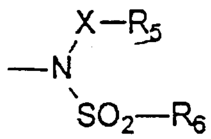

(III)

$R_5$ is a reactive group which is able to undergo bonding under mild conditions selectively with amino, carboxyl, thiol or other functional groups in substances of biological interest;

$R_6$ is an alkyl group having 1 to 4 carbon atoms or a phenyl group which can be substituted by up to 3 alkyl or alkoxy groups each having 1 to 4 carbon atoms,
by an $-(-O-CH_2-CH_2-)_n-OR$ group, where
n has the meaning 0 to 8 and
R is a morpholinoethyl or an N,N-dimethylaminoethyl or an alkyl group having 1 to 4 carbon atoms, or
by an ethylenedioxy group; and

X is an arylene group which is bonded to the nitrogen or sulfur atom directly or via an alkylene or an oxyalkylene group and is bonded to the radical $R_5$ via an alkylene or oxyalkylene group, and
which can also be substituted one or more times by alkyl, alkenyl, hydroxyl, amino, alkoxy or aryloxy groups and/or hetero atoms or
is the radical of an aromatic natural amino acid or
is a phenylene group - when $R_6$ is a phenyl group - which is substituted one or more times by alkyl groups having 1 to 6 carbon atoms and

$A^-$ is an anion which does not adversely affect the chemiluminescence.

which comprises reacting a compound of the formula IX

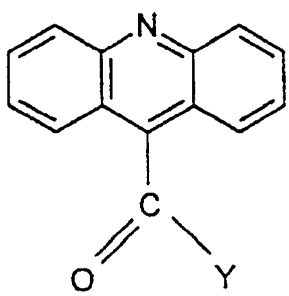

(IX)

with a protected sulfonamide carboxylic acid of the formula X

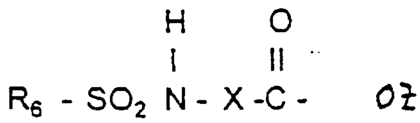 (X)

or of the formula XI

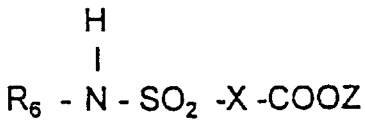 (XI)

where Y is halogen, an oxycarbonylalkyl, oxycarbonylaryl or imidazolide group, X and $R_6$ have the above-mentioned meanings, and Z is a radical which protects the carboxyl group and is subsequently eliminated, and converting the acid resulting from this into the desired acridinium derivative which contains the radical $R_5$ and is then additionally quaternized on the nitrogen of the acridine skeleton.

**2.** A process for the preparation of a luminescent compound, which comprises bonding an acridinium derivative prepared as claimed in claim 1 directly or via a bridging molecule to a protein, a polypeptide or to another substance of biological interest with the formation of a stable immunologically active conjugate.

**3.** A luminescence immunoassay for the determination of an antigenic substance in a liquid sample, in which, in a competitive or sandwich method, at least one immunologically active component is immobilized on a solid phase, and at least one other component, the tracer, represents a luminescent compound, which has been prepared by the process as claimed in claim 2.

**4.** A luminescence immunoassay as claimed in claim 3, wherein

a) an immobilized antibody which reacts specifically with an antigen is incubated with a sample of the liquid which is to be investigated and with a conjugate composed of the antigen and of a chemiluminescent acridinium derivative prepared as claimed in claim 1;

b) the sample and the unbound conjugate with marker are separated off;

c) the bound conjugate with marker is contacted successively or simultaneously with one or more reagents in order to bring about light emission and

d) the amount of antigen present is determined from the measured intensity of light emission.

**5.** A luminescence immunoassay as claimed in claim 4, wherein the liquid which is to be investigated is separated from the immobilized antibody before the addition of the conjugate with marker.

**6.** A luminescence immunoassay as claimed in claim 3, wherein

a) an immobilized antibody which reacts specifically with the antigen is incubated with a sample of the liquid which is to be investigated and with a conjugate composed of a second specifically reacting antibody and of a chemiluminescent acridinium derivative prepared as claimed in claim 1;
b) the sample and the unbound conjugate with marker are separated off;
c) the bound conjugate with marker is contacted successively or simultaneously with one or more reagents in order to bring about light emission and
d) the amount of antigen present is determined from the measured intensity of light emission.

**7.** A luminescence immunoassay as claimed in claim 6, wherein the liquid which is to be investigated is separated from the immobilized antibody before the addition of the conjugate with marker.

**8.** A luminescence immunoassay as claimed in claim 4, wherein

a) an immobilized antigen which reacts specifically with the antibody is incubated with a sample of the liquid which is to be investigated and with a solution of a conjugate composed of the antibody and of a chemiluminescent acridinium derivative prepared as claimed in claim 1;
b) the sample and the unbound conjugate with marker are separated off;
c) the bound conjugate with marker is contacted successively or simultaneously with one or more reagents in order to bring about light emission and
d) the amount of antigen present is determined from the measured intensity of light emission.

**9.** A luminescence immunoassay as claimed in claim 3, wherein

a) an immobilized antigen which reacts specifically with the antibody is incubated with a solution of a conjugate composed of the antibody and of a chemiluminescent acridinium derivative prepared as claimed in claim 1;
b) the unreacted conjugate with marker is separated off,
c) a sample of the liquid which is to be investigated is added,
d) the sample is subsequently separated off again,
e) the bound conjugate with marker is contacted successively or simultaneously with one or more reagents in order to bring about light emission and
f) the amount of antigen present is determined from the measured intensity of light emission.

**10.** A luminescence immunoassay as claimed in claim 3, wherein

a) an immobilized antigen which reacts specifically with the antibody is incubated with a solution of a conjugate composed of the antibody and of a chemiluminescent acridinium derivative prepared as claimed in claim 1;
b) a sample of the liquid which is to be investigated is added,
c) the sample and the unbound conjugate are separated off;
d) the bound conjugate with marker is contacted successively or simultaneously with one or more reagents in order to bring about light emission and
e) the amount of antigen present is determined from the measured intensity of light emission.

**Revendications**

**Revendications pour les Etats contractants suivants : AT, BE, CH, DE, DK, FR, GB, IE, IT, LI, LU, MC, NL, PT, SE**

**1.** Dérivés d'acridinium chimiluminescents de formule I

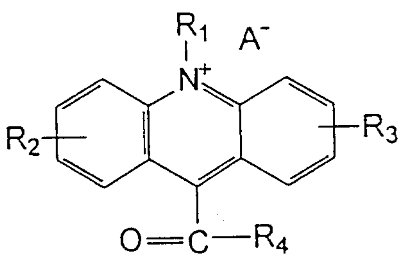

(I)

dans laquelle

$R_1$ représente
un atome d'hydrogène,
un radical alkyle, alcényle ou alcynyle ayant jusqu'à 10 atomes de carbone,
un groupe benzyle ou aryle;
$R_2$ et $R_3$ représentent
un atome d'hydrogène,
un groupe alkyle ayant de 1 à 4 atomes de carbone,
un groupe amino substitué ou non substitué,

un groupe carboxyle, alcoxy, cyano ou nitro, ou
un atome d'halogène;

$R_4$ représente
un radical de formule II ou III:

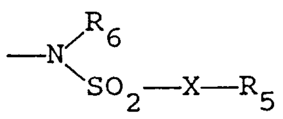

(II)

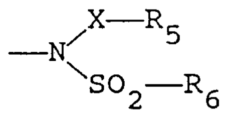

(III)

$R_5$ représente un groupe réactif
qui peut, dans des conditions ménagées, engendrer une liaison sélectivement avec des groupes amino, carboxyle, thiol ou d'autres groupes fonctionnels dans des substances d'intérêt biologique;

$R_6$ représente
un groupe alkyle ayant de 1 à 4 atones de carbone ou
un groupe phényle qui peut être substitué
par jusqu'à 3 groupes alkyle ou alcoxy ayant chacun de 1 à 4 atones de carbone,
par un groupe $-(-O-CH_2-CH_2-)_n-OR$,
n représentant 0 à 8 et
R étant le groupe morpholinoéthyle ou N,N-diméthylaminoéthyle ou un groupe alkyle ayant de 1 à 4 atomes de carbone, ou
par un groupe éthylènedioxy; et

X représente un groupe arylène qui
est relié à l'atone d'azote ou de soufre directement ou par l'intermédiaire d'un groupe alkylène ou oxyalkylène,
et
est relié au radical $R_5$ par un groupe alkylène ou oxyalkylène, et
qui peut également être une ou plusieurs fois substitué par
des groupes alkyle, alcényle, hydroxyle, amino, alcoxy ou aryloxy et/ou
des hétéroatomes, ou
le reste d'un aminoacide aromatique naturel, ou
un groupe phénylène - lorsque $R_6$ est un groupe phényle -
qui est une ou plusieurs fois substitué par des groupes alkyle ayant de 1 à 6 atones de carbone,
et

$A^-$ est un anion ne nuisant pas à la chimiluminescence.

**2.** Dérivés d'acridinium selon la revendication 1, caractérisés en ce que X est un groupe de formule IV

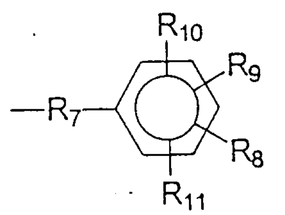

(IV)

dans laquelle

$R_7$ représente un substituant de formule $-(CH_2)_n-$ ou $-((CH_2)_m-O-)_n-$, où n = 0 à 4 et m = 1 à 6;

$R_8$ représente un substituant de formule $-(CH_2)_p-$, $-(O-(CH_2)_m-)_p$ ou $((CH_2)_m-O)_p-$ où p = 1 à 6 et m = 1 à 6, en position ortho, méta ou para par rapport à $R_7$, ou un radical hydrocarboné ramifié ou non ramifié

ayant de 1 à 4 atomes de carbone, et

$R_9$ à $R_{11}$ représentent des atomes d'hydrogène ou des radicaux hydrocarbonés à chaîne droite ou ramifiés ayant jusqu'à 30 atomes de carbone,
un ou plusieurs groupes $-CH_2-$ pouvant être remplacés par O, S, SO, NH ou par des groupes N-alkyle, et deux de ces substituants pouvant être liés l'un à l'autre pour former un cycle.

**3.** Dérivés d'acridinium selon la revendication 1 ou 2, caractérisés en ce que $R_5$ est un groupe de formule V

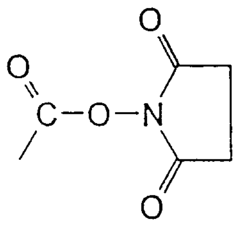

(V)

**4.** Dérivés d'acridinium selon au moins l'une des revendications 1 à 3, caractérisés par la formule VI

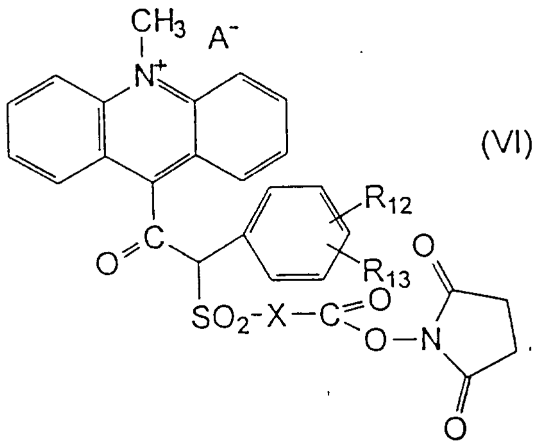

(VI)

dans laquelle X est un groupe de formule VII

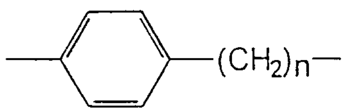

(VII)

où n = 2 ou 4 et
$R_{12}$ et $R_{13}$ représentent, indépendamment l'un de l'autre,

un atome d'hydrogène,
un groupe alkyle, un groupe alcoxy ayant de 1 à 4 atomes de carbone,
un groupe $-(-O-CH_2-CH_2-)_n-OR$,

n représentant 0 à 8 et
R étant le groupe morpholinoéthyle ou N,N-diméthylaminoéthyle ou un groupe alkyle ayant de 1 à 4 atomes de carbone,

ou représentent ensemble un groupe éthylènedioxy, et

$A^-$ a la signification donnée dans la revendication 1.

**5.** Dérivé d'acridinium selon la revendication 4, caractérisé en ce que

R représente le groupe morpholinoéthyle ou N,N-diméthylaminoéthyle,

ainsi que les composés d'ammonium quaternaire.

**6.** Dérivé d'acridinium selon la revendication 4, caractérisé en ce que

X est le groupe p-éthylènephényle et
$R_{12}$ est un atome d'hydrogène et $R_{13}$ est le groupe p-méthoxy, ou
$R_{12}$ est le groupe o-méthoxy et $R_{13}$ est le groupe p-méthoxy, ou
$R_{12}$ et $R_{13}$ forment ensemble le groupe 3,4-éthylènedioxy.

**7.** Dérivé d'acridinium selon la revendication 1 ou 2, caractérisé par la formule VIII

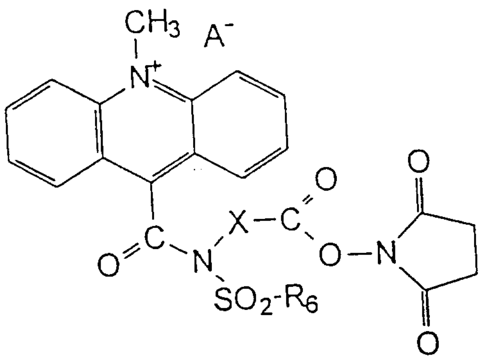

(VIII)

dans laquelle X a la signification donnée dans la revendication 1 ou 2, et

$R_6$ représente
un groupe alkyle ayant de 1 à 4 atomes de carbone ou
un groupe phényle qui peut être substitué
par jusqu'à 3 groupes alkyle ou alcoxy ayant chacun de 1 à 4 atomes de carbone,
par un groupe $-(-O-CH_2-CH_2)_n-OR$,
n représentant 0 à 8 et
R étant le groupe morpholinoéthyle ou N,N-diméthylaminoéthyle ou un groupe alkyle ayant de 1 à 4 atomes de carbone, ou
par un groupe éthylènedioxy; ou
dans laquelle X est le groupe o-, m- ou p-phénylène, et
$R_6$ est un groupe phényle qui peut être substitué par jusqu'à trois groupes alkyle ayant chacun de 1 à 4 atomes de carbone.

**8.** Dérivé d'acridinium selon la revendication 7, caractérisé en ce que

R représente le groupe morpholinoéthyle ou N,N-diméthylaminoéthyle,

ainsi que les composés d'ammonium quaternaire.

**9.** Procédé pour la préparation des composés selon l'une des revendications 1 à 8, caractérisé en ce que l'on fait réagir un composé de formule IX

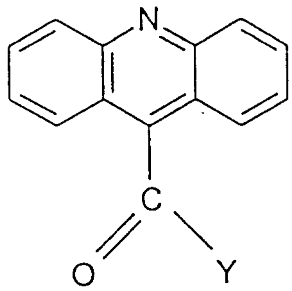

(IX)

avec un acide sulfonamidocarboxylique protégé de formule X

$$R_6-SO_2\overset{H}{\overset{|}{N}}-X-\overset{O}{\overset{\|}{C}}-OZ \qquad (X)$$

ou de formule XI

$$R_6-\overset{H}{\overset{|}{N}}-SO_2-X-COOZ \qquad (XI)$$

Y étant un atome d'halogène ou un groupe oxycarbonylalkyle, oxycarbonylaryle ou imidazolide, X et $R_6$ ayant les significations données dans la revendication 1 et Z étant un radical protecteur de groupe carboxyle, qui est ensuite éliminé, et l'acide ainsi formé est converti en le dérivé d'acridinium recherché, contenant le radical $R_5$, qui est ensuite rendu quaternaire au niveau de l'atome d'azote du squelette acridine.

**10.** Composé luminescent, caractérisé en ce qu'un dérivé d'acridinium selon l'une des revendications 1 à 9 est lié, directement ou par l'intermédiaire d'une molécule formant un pont, à une protéine, un polypeptide ou une autre substance d'intérêt biologique, avec formation d'un conjugué stable, immunologiquement actif.

**11.** Essai immunologique en luminescence pour la détermination d'une substance antigénique dans un échantillon de liquide, dans lequel, dans un procédé compétitif ou en sandwich, au moins un composant immunologiquement actif est immobilisé sur une phase solide et au moins un autre composant, le marqueur, représente un composé luminescent selon la revendication 10.

**12.** Essai immunologique en luminescence selon la revendication 11, caractérisé en ce que

a) on met à incuber un anticorps immobilisé, réagissant spécifiquement avec un antigène, avec un échantillon du liquide à analyser et un conjugué de l'antigène et d'un dérivé d'acridinium chimiluminescent selon la revendication 1,
b) on sépare l'échantillon et le conjugué marqué non fixé,
c) on met en contact le conjugué marqué fixé, simultanément ou successivement avec un ou plusieurs réactifs, afin de provoquer une émission de lumière,
et
d) on détermine la quantité de l'antigène présent à partir de l'intensité mesurée de l'émission de lumière.

**13.** Essai immunologique en luminescence selon la revendication 12, caractérisé en ce que le liquide à analyser est séparé de l'anticorps immobilisé, avant l'addition du conjugué marqué.

**14.** Essai immunologique en luminescence selon la revendication 11, caractérisé en ce que

a) on met à incuber un anticorps immobilisé, réagissant spécifiquement avec l'antigène, avec un échantillon du liquide à analyser et un conjugué d'un second anticorps réagissant spécifiquement et d'un dérivé d'acridinium

chimiluminescent selon la revendication 1,
b) on sépare l'échantillon et le conjugué marqué non fixé;
c) on met en contact le conjugué marqué fixé, simultanément ou successivement avec un ou plusieurs réactifs, afin de provoquer une émission de lumière,
et
d) on détermine la quantité de l'antigène présent à partir de l'intensité mesurée de l'émission de lumière.

**15.** Essai immunologique en luminescence selon la revendication 14, caractérisé en ce que le liquide à analyser est séparé de l'anticorps immobilisé, avant l'addition du conjugué marque.

**16.** Essai immunologique en luminescence selon la revendication 11, caractérisé en ce que

a) on met à incuber un antigène immobilisé, réagissant spécifiquement avec l'anticorps, avec un échantillon du liquide à analyser et une solution d'un conjugué de l'anticorps et d'un dérivé d'acridinium chimiluminescent selon la revendication 1,
b) on sépare l'échantillon et le conjugué marqué non fixé;
c) on met en contact le conjugué marqué fixé, simultanément ou successivement avec un ou plusieurs réactifs, afin de provoquer une émission de lumière,
et
d) on détermine la quantité de l'antigène présent à partir de l'intensité mesurée de l'émission de lumière.

**17.** Essai immunologique en luminescence selon la revendication 11, caractérisé en ce que

a) on met à incuber un antigène immobilisé, réagissant spécifiquement avec l'anticorps, avec une solution d'un conjugué de l'anticorps et d'un dérivé d'acridinium chimiluminescent selon la revendication 1,
b) on sépare le conjugué marqué n'ayant pas réagi,
c) on ajoute un échantillon du liquide à analyser,
d) on sépare ensuite à nouveau l'échantillon,
e) on met en contact le conjugué marqué fixé, simultanément ou successivement avec un ou plusieurs réactifs, afin de provoquer une émission de lumière,
et
f) on détermine la quantité de l'antigène présent à partir de l'intensité mesurée de l'émission de lumière.

**18.** Essai immunologique en luminescence selon la revendication 11, caractérisé en ce que

a) on met à incuber un antigène immobilisé, réagissant spécifiquement avec l'anticorps, avec une solution d'un conjugué de l'anticorps et d'un dérivé d'acridinium chimiluminescent selon la revendication 1,
b) on ajoute un échantillon du liquide à analyser,
c) on sépare l'échantillon et le conjugué non fixé,
d) on met en contact le conjugué marqué fixé, simultanément ou successivement avec un ou plusieurs réactifs, afin de provoquer une émission de lumière
et
e) on détermine la quantité de l'antigène présent à partir de l'intensité mesurée de l'émission de lumière.

**Revendications pour les Etats contractants suivants : ES, GR**

**1.** Procédé pour la préparation de dérivés d'acridinium chimiluminescents de formule I

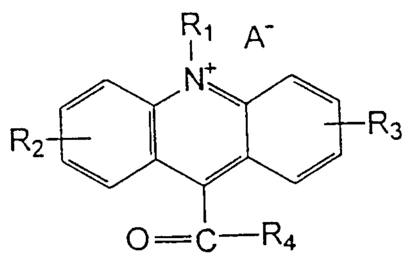

(I)

dans laquelle

$R_1$ représente
un atome d'hydrogène,
un radical alkyle, alcényle ou alcynyle ayant jusqu'à 10 atomes de carbone,
un groupe benzyle ou aryle;

$R_2$ et $R_3$ représentent
un atome d'hydrogène,
un groupe alkyle ayant de 1 à 4 atomes de carbone,
un groupe amino substitué ou non substitué,
un groupe carboxyle, alcoxy, cyano ou nitro, ou
un atome d'halogène;

$R_4$ représente
un radical de formule II ou III:

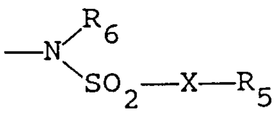

(II)

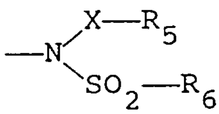

(III)

$R_5$ représente un groupe réactif
qui peut, dans des conditions ménagées, engendrer une liaison sélectivement avec des groupes amino, carboxyle, thiol ou d'autres groupes fonctionnels dans des substances d'intérêt biologique;

$R_6$ représente
un groupe alkyle ayant de 1 à 4 atomes de carbone ou
un groupe phényle qui peut être substitué
par jusqu'à 3 groupes alkyle ou alcoxy ayant chacun de 1 à 4 atomes de carbone,
par un groupe $-(-O-CH_2-CH_2-)_n-OR$,
n représentant 0 à 8 et
R étant le groupe morpholinoéthyle ou N,N-diméthylaminoéthyle ou un groupe alkyle ayant de 1 à 4 atomes de carbone, ou
par un groupe éthylènedioxy; et

X représente un groupe arylène qui
est relié à l'atome d'azote ou de soufre directement ou par l'intermédiaire d'un groupe alkylène ou oxyalkylène,
et
est relié au radical $R_5$ par un groupe alkylène ou oxyalkylène, et
qui peut également être une ou plusieurs fois substitué par
des groupes alkyle, alcényle, hydroxyle, amino, alcoxy ou aryloxy et/ou
des hétéroatomes, ou
le reste d'un aminoacide aromatique naturel, ou
un groupe phénylène - lorsque $R_6$ est un groupe phényle -
qui est une ou plusieurs fois substitué par des groupes alkyle ayant de 1 à 6 atomes de carbone, et

$A^-$ est un anion ne nuisant pas à la chimiluminescence,

caractérisé en ce que l'on fait réagir un composé de formule IX

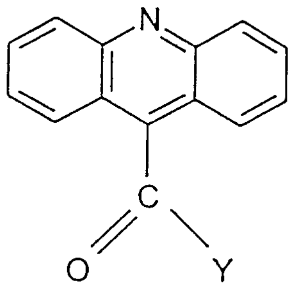

(IX)

avec un acide sulfonamidocarboxylique protégé de formule X

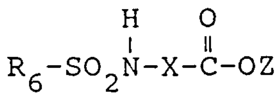

(X)

ou de formule XI

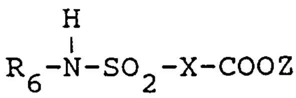

(XI)

Y étant un atone d'halogène ou un groupe oxycarbonylalkyle, oxycarbonylaryle ou imidazolide, X et $R_6$ ayant les significations données plus haut et Z étant un radical protecteur de groupe carboxyle, qui est ensuite éliminé, et l'acide ainsi formé est converti en le dérivé d'acridinium recherché, contenant le radical $R_5$, qui est ensuite rendu quaternaire au niveau de l'atome d'azote du squelette acridine.

**2.** Procédé pour la préparation d'un composé luminescent, caractérisé en ce qu'un dérivé d'acridinium préparé selon la revendications 1 est lié, directement ou par l'intermédiaire d'une molécule formant un pont, à une protéine, un polypeptide ou une autre substance d'intérêt biologique, avec formation d'un conjugué stable, immunologiquement actif.

**3.** Essai immunologique en luminescence pour la détermination d'une substance antigénique dans un échantillon de liquide, dans lequel, dans un procédé compétitif ou en sandwich, au moins un composant immunologiquement actif est immobilisé sur une phase solide et au moins un autre composant, le marqueur, représente un composé luminescent, qui a été préparé par le procédé selon la revendication 2.

**4.** Essai immunologique en luminescence selon la revendication 3, caractérisé en ce que

a) on met à incuber un anticorps immobilisé, réagissant spécifiquement avec un antigène, avec un échantillon du liquide à analyser et un conjugué de l'antigène et d'un dérivé d'acridinium chimiluminescent préparé selon la revendication 1,
b) on sépare l'échantillon et le conjugué marqué non fixé,
c) on met an contact le conjugué nargué fixé, simultanément ou successivement avec un ou plusieurs réactifs, afin de provoquer une émission de lumière,
et
d) on détermine la quantité de l'antigène présent à partir de l'intensité mesurée de l'émission de lumière.

**5.** Essai immunologique en luminescence selon la revendication 4, caractérisé en ce que le liquide à analyser est séparé de l'anticorps immobilisé, avant l'addition du conjugué marqué.

**6.** Essai immunologique en luminescence selon la revendication 3, caractérisé en ce que

a) on met à incuber un anticorps immobilisé, réagissant spécifiquement avec l'antigène, avec un échantillon du liquide à analyser et un conjugué d'un second anticorps réagissant spécifiquement et d'un dérivé d'acridinium chimiluminescent préparé selon la revendication 1,
b) on sépare l'échantillon et le conjugué marqué non fixé;
c) on met an contact le conjugué marqué fixé, simultanément ou successivement avec un ou plusieurs réactifs, afin de provoquer une émission de lumière,
et
d) on détermine la quantité de l'antigène présent à partir de l'intensité mesurée de l'émission de lumière.

**7.** Essai immunologique en luminescence selon la revendication 6, caractérisé an ce que le liquide à analyser est séparé de l'anticorps immobilisé, avant l'addition du conjugué marqué.

**8.** Essai immunologique an luminescence selon la revendication 4, caractérisé an ce que

a) on met à incuber un antigène immobilisé, réagissant spécifiquement avec l'anticorps, avec un échantillon du liquide à analyser et une solution d'un conjugué de l'anticorps et d'un dérivé d'acridinium chimiluminescent préparé selon la revendication 1,
b) on sépare l'échantillon et le conjugué marqué non fixé;
c) on met en contact le conjugué marqué fixé, simultanément ou successivement avec un ou plusieurs réactifs, afin de provoquer une émission de lumière,
et
d) on détermine la quantité de l'antigène présent à partir de l'intensité mesurée de l'émission de lumière.

**9.** Essai immunologique'en luminescence selon la revendication 3, caractérisé en ce que

a) on met à incuber un antigène immobilisé, réagissant spécifiquement avec l'anticorps, avec une solution d'un conjugué de l'anticorps et d'un dérivé d'acridinium chimiluminescent préparé selon la revendication 1,
b) on sépare le conjugué marqué n'ayant pas réagi,
c) on ajoute un échantillon du liquide à analyser,
d) on sépare ensuite à nouveau l'échantillon,
e) on met en contact le conjugué marqué fixé, simultanément ou successivement avec un ou plusieurs réactifs, afin de provoquer une émission de lumière,
et
f) on détermine la quantité de l'antigène présent à partir de l'intensité mesurée de l'émission de lumière.

**10.** Essai immunologique en luminescence selon la revendication 3, caractérisé en ce que

a) on met à incuber un antigène immobilisé, réagissant spécifiquement avec l'anticorps, avec une solution d'un conjugué de l'anticorps et d'un dérivé d'acridinium chimiluminescent préparé selon la revendication 1,
b) on ajoute un échantillon du liquide à analyser,
c) on sépare l'échantillon et le conjugué non fixé,
d) on met en contact le conjugué marqué fixé, simultanément ou successivement avec un ou plusieurs réactifs, afin de provoquer une émission de lumière
et
e) on détermine la quantité de l'antigène présent à partir de l'intensité mesurée de l'émission de lumière.

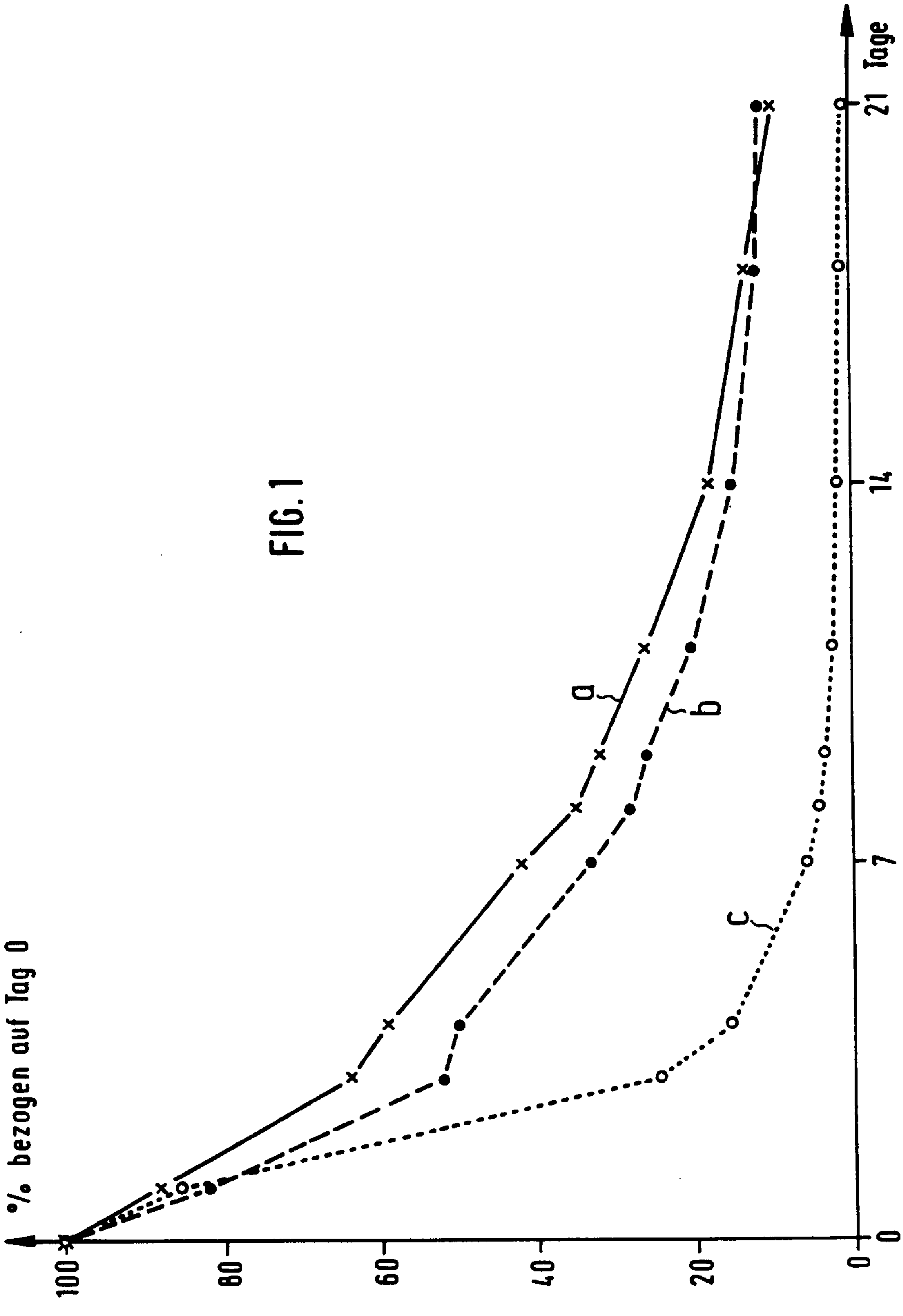


FIG. 1

FIG. 2

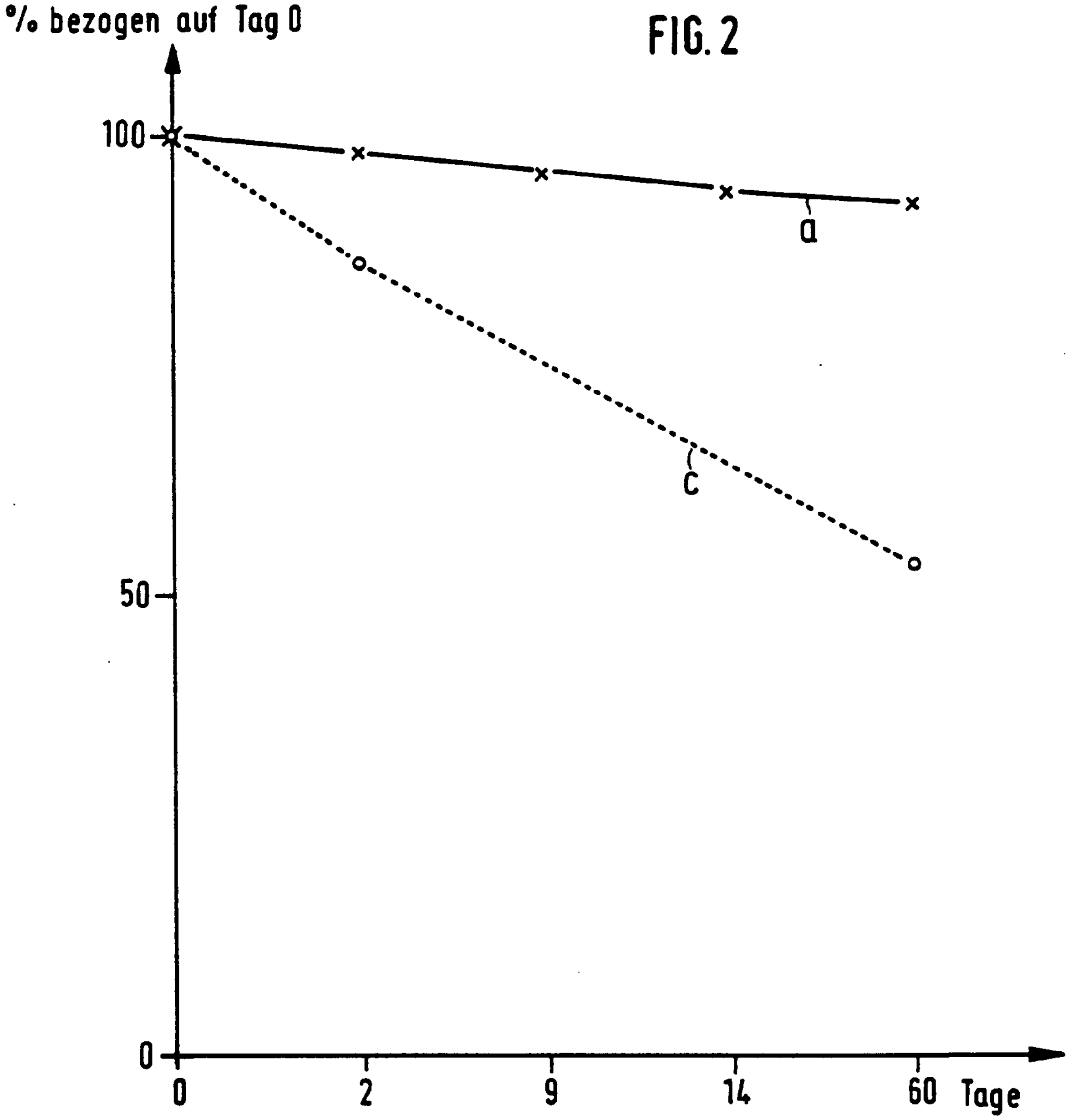

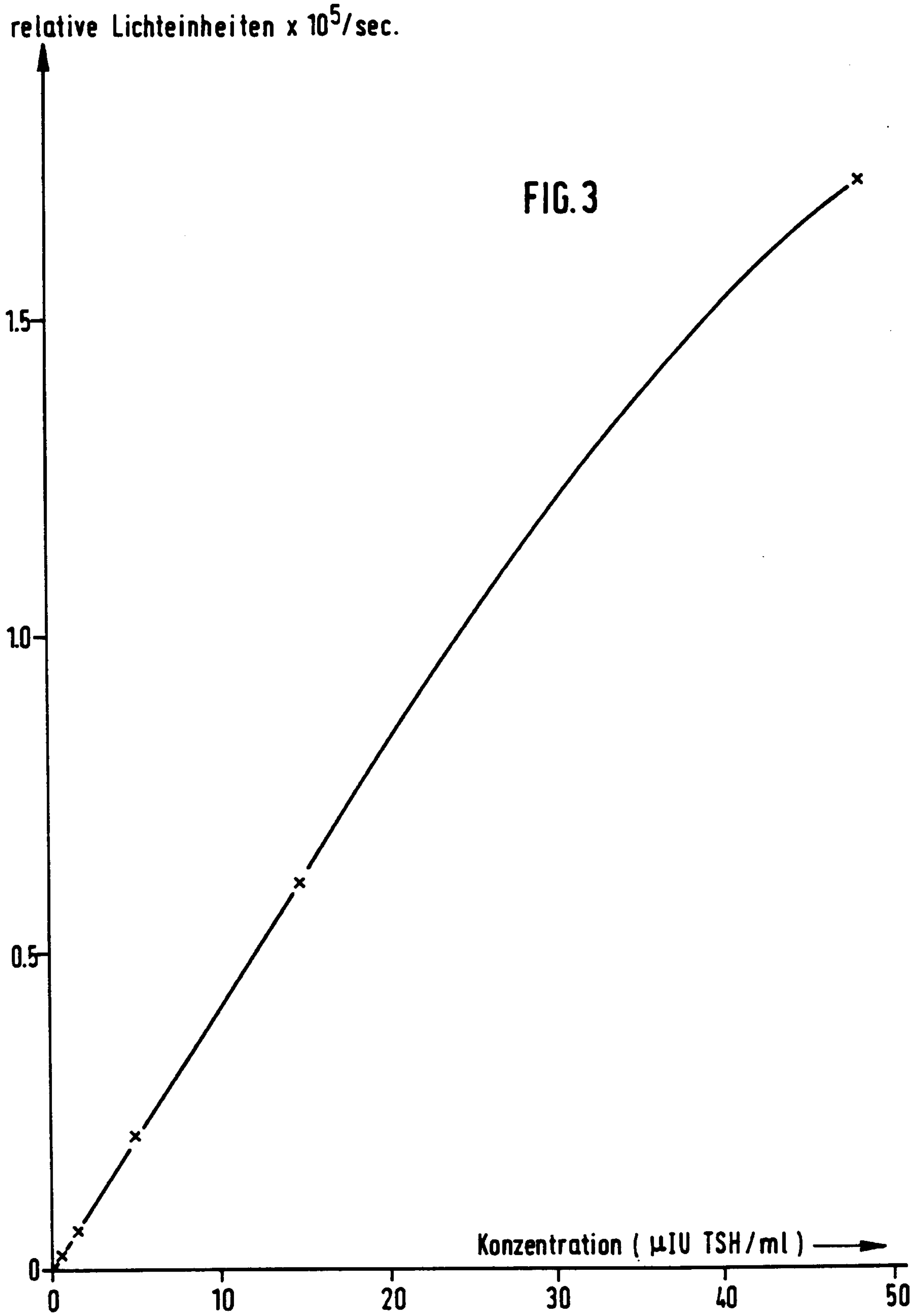
relative Lichteinheiten x $10^5$/sec.
1.5
1.0
0.5
0
Konzentration ( µIU TSH/ml )
0
10
20
30
40
50

FIG. 3